# EUROPEAN PATENT SPECIFICATION

(11) **EP 2 922 971 B1**
(45) Date of publication and mention of the grant of the patent: **17.10.2018**
(21) Application number: 13814254.2
(22) Date of filing: 20.11.2013
(51) Int. Cl.: C12Q 1/6886

(54) **GENE EXPRESSION PROFILE IN DIAGNOSTICS**
GENEXPRESSIONSPROFIL IN EINER DIAGNOSTIK
PROFIL D'EXPRESSION GÉNIQUE DANS DES DIAGNOSTICS

(30) Priority: 20.11.2012 NO 20121378
(43) Date of publication of application: 30.09.2015
(73) Proprietor: Lund, Tore Eiliv, 9013 Tromsø (NO)
(72) Inventor: DUMEAUX, Vanessa, St-Lambert, Quebec J4P 1G9 (CA); LUND, Eiliv, 9013 Tromsø (NO)
(74) Representative: Zacco Norway AS
(86) International application number: PCT/NO2013/050203
(87) International publication number: WO 2014/081313

(56) References cited:
- WO-A1-2005/118851
- WO-A1-2010/127399
- WO-A2-01/51664
- WO-A2-2011/086174
- WEIGELT B ET AL: "Breast cancer molecular profiling with single sample predictors: a retrospective analysis", LANCET ONCOLOGY, LANCET PUBLISHING GROUP, LONDON, GB, vol. 11, no. 4, 1 April 2010 (2010-04-01), pages 339-349, XP026987303, ISSN: 1470-2045 [retrieved on 2010-03-29]
- DUMEAUX V ET AL: "Gene expression analyses in breast cancer epidemiology - The Norwegian Women and Cancer postgenome cohort study", EUROPEAN JOURNAL OF CANCER. SUPPLEMENT, PERGAMON, OXFORD, GB, vol. 6, no. 9, 1 July 2008 (2008-07-01), pages 194-195, XP022834213, ISSN: 1359-6349, DOI: 10.1016/S1359-6349(08)71867-6 [retrieved on 2008-07-01]
- AAROE JORGEN ET AL: "Gene expression profiling of peripheral blood cells for early detection of breast cancer", BREAST CANCER RESEARCH, CURRENT SCIENCE, LONDON, GB, vol. 12, no. 1, 15 January 2010 (2010-01-15), page R7, XP021070771, ISSN: 1465-5411 cited in the application
- SHARMA PRAVEEN ET AL: "Early detection of breast cancer based on gene-expression patterns in peripheral blood cells", BREAST CANCER RESEARCH, CURRENT SCIENCE, LONDON, GB, vol. 7, no. 5, 14 June 2005 (2005-06-14), pages R634-R644, XP021011859, ISSN: 1465-5411, DOI: 10.1186/BCR1203
- "Affymetrix GeneChip Human Genome U133 Array Set HG-U133A", GEO,, 11 March 2002 (2002-03-11), XP002355386, [retrieved on 2002-03-11]

### SEQUENCE LISTING

<110> University of Tromsoe
   Dumeaux, Vanessa (US only)
   Lund, Eiliv (US only)
<120> Gene Expression Profile In Diagnostics
<130> P612001578NO01
<160> 545
<170> PatentIn version 3.5
<210> 1
   <211> 50
   <212> DNA
   <213> Artificial Sequence
<220>
   <223> Oligonucleotide
<400> 1
   gtgctctttg ttcatcattg gccctcattc caagcacttt acgctgtctg 50
<210> 2
   <211> 50
   <212> DNA
   <213> Artificial Sequence
<220>
   <223> Oligonucleotide
<400> 2
   ccaggcagag actacctttg tgaccagctc ccagtaaaaa ccccaggcac 50
<210> 3
   <211> 50
   <212> DNA
   <213> Artificial Sequence
<220>
   <223> Oligonucleotide
<400> 3
   tgtcctgtga ctgccccaca gagataaggg gccaggaggg attgaaaggc 50
<210> 4
   <211> 50
   <212> DNA
   <213> Artificial Sequence
<220>
   <223> Oligonucleotide
<400> 4
   aaacgccagg tctgcctgtt cttgctgggc aatggctgat ggctgccagt 50
<210> 5
   <211> 50
   <212> DNA
   <213> Artificial Sequence
<220>
   <223> Oligonucleotide
<400> 5
   taaggagaat ggcccagtcc tctcccaagt ccacacaggg gaggtgatag 50
<210> 6
   <211> 50
   <212> DNA
   <213> Artificial Sequence
<220>
   <223> Oligonucleotide
<400> 6
   cggctacagc ttcaccacca cggccgagcg ggaaatcgtg cgtgacatta 50
<210> 7
   <211> 50
   <212> DNA
   <213> Artificial Sequence
<220>
   <223> Oligonucleotide
<400> 7
   acaggaagtc ccttgccatc ctaaaagcca ccccacttct ctctaaggag 50
<210> 8
   <211> 50
   <212> DNA
   <213> Artificial Sequence
<220>
   <223> Oligonucleotide
<400> 8
   gaggctggca agaaccagtt gttttgtctt gcgggtctgt cagggttgga 50
<210> 9
   <211> 50
   <212> DNA
   <213> Artificial Sequence
<220>
   <223> Oligonucleotide
<400> 9
   cgtttctctg ccggtcgcaa tggaagaaga gatcgccgcg ctggtcattg 50
<210> 10
   <211> 50
   <212> DNA
   <213> Artificial Sequence
<220>
   <223> Oligonucleotide
<400> 10
   agctcctttg tcgctctcat ggctgtcaga tcctggtccc tccacactgg 50
<210> 11
   <211> 50
   <212> DNA
   <213> Artificial Sequence
<220>
   <223> Oligonucleotide
<400> 11
   gtggaattgg caaacccact gcagcccctg agaggaggtc gaatgggtaa 50
<210> 12
   <211> 50
   <212> DNA
   <213> Artificial Sequence
<220>
   <223> Oligonucleotide
<400> 12
   agttccacag gctcccgtcc agggggagga ctacggcaaa ggtgtcatct 50
<210> 13
   <211> 50
   <212> DNA
   <213> Artificial Sequence
<220>
   <223> Oligonucleotide
<400> 13
   cagtgtgaca gtgccagcca atgtgcagag gtggatgagg tcttgtgaaa 50
<210> 14
   <211> 50
   <212> DNA
   <213> Artificial Sequence
<220>
   <223> Oligonucleotide
<400> 14
   gcaggcagga acagtgtggg tgaattgcta tggcgtggta agtgcccagt 50
<210> 15
   <211> 50
   <212> DNA
   <213> Artificial Sequence
<220>
   <223> Oligonucleotide
<400> 15
   caattctccc aatgagcctt ttgtctgtgg gaaaagcagg agacgcttcg 50
<210> 16
   <211> 50
   <212> DNA
   <213> Artificial Sequence
<220>
   <223> Oligonucleotide
<400> 16
   ctacctgctt ggcctggggc ctctggaagt ctgctgtgaa tttgtattcc 50
<210> 17
   <211> 50
   <212> DNA
   <213> Artificial Sequence
<220>
   <223> Oligonucleotide
<400> 17
   ctctcagggg ccagaactcc tttgccagcg tggatttctc aagtcgggac 50
<210> 18
   <211> 50
   <212> DNA
   <213> Artificial Sequence
<220>
   <223> Oligonucleotide
<400> 18
   ggtgtgaacc tacctgcctt ggagagggcc caggtcccaa atctcttcaa 50
<210> 19
   <211> 50
   <212> DNA
   <213> Artificial Sequence
<220>
   <223> Oligonucleotide
<400> 19
   ggttcctgta gcagcagccg aagggagacc tcgcttaaac caacacaatc 50
<210> 20
   <211> 50
   <212> DNA
   <213> Artificial Sequence
<220>
   <223> Oligonucleotide
<400> 20
   cctggtggct ctttgtggag gaaactaaac attcccttga tggtctcaag 50
<210> 21
   <211> 50
   <212> DNA
   <213> Artificial Sequence
<220>
   <223> Oligonucleotide
<400> 21
   cgtgcttggg gttcagctgg tgaggctgtc cctgtaggaa gaaagctctg 50
<210> 22
   <211> 50
   <212> DNA
   <213> Artificial Sequence
<220>
   <223> Oligonucleotide
<400> 22
   tgtggtggag atgactgaag cccgacacgg cctgagcgtc cagaaatggt 50
<210> 23
   <211> 50
   <212> DNA
   <213> Artificial Sequence
<220>
   <223> Oligonucleotide
<400> 23
   gtgcctgcac caccccactg ccttccttca gcacctttag ctgcatttgt 50
<210> 24
   <211> 50
   <212> DNA
   <213> Artificial Sequence
<220>
   <223> Oligonucleotide
<400> 24
   gccttctctg cctgtccctc cgatccttgt ccaccgtcta tttattgccc 50
<210> 25
   <211> 50
   <212> DNA
   <213> Artificial Sequence
<220>
   <223> Oligonucleotide
<400> 25
   ttatacggat gactgggagg cactgcacca caacgtagga ccctggctcc 50
<210> 26
   <211> 50
   <212> DNA
   <213> Artificial Sequence
<220>
   <223> Oligonucleotide
<400> 26
   cctagctcct tgttggtgag cttcttgtgc cttaatcctg tgacccagcc 50
<210> 27
   <211> 50
   <212> DNA
   <213> Artificial Sequence
<220>
   <223> Oligonucleotide
<400> 27
   acccccacag accccgttcc tccagcctgc gtgcccctaa cctggctttt 50
<210> 28
   <211> 50
   <212> DNA
   <213> Artificial Sequence
<220>
   <223> Oligonucleotide
<400> 28
   gcagagctgt gcttcctgga cgtgattccc ttttggaagc tggacctgga 50
<210> 29
   <211> 50
   <212> DNA
   <213> Artificial Sequence
<220>
   <223> Oligonucleotide
<400> 29
   catcatgctg ggggagattc tcagacactc gatggatcca cccacattca 50
<210> 30
   <211> 50
   <212> DNA
   <213> Artificial Sequence
<220>
   <223> Oligonucleotide
<400> 30
   atgatggcgg tggaggtggt ggttgtagtg tgatggatcc cctttaggtt 50
<210> 31
   <211> 50
   <212> DNA
   <213> Artificial Sequence
<220>
   <223> Oligonucleotide
<400> 31
   tcattggact catggtgggc ggtgttgtca tagcgacagt gatcgtcatc 50
<210> 32
   <211> 50
   <212> DNA
   <213> Artificial Sequence
<220>
   <223> Oligonucleotide
<400> 32
   gcagaactag acccccgcca cagcagcctc tgaagttgga cagcaaaacc 50
<210> 33
   <211> 50
   <212> DNA
   <213> Artificial Sequence
<220>
   <223> Oligonucleotide
<400> 33
   gagttacaag caccagggga tgctctacat caagggatgc accttcagtc 50
<210> 34
   <211> 50
   <212> DNA
   <213> Artificial Sequence
<220>
   <223> Oligonucleotide
<400> 34
   gctggttgaa aagtaccact cccactctga acatctggcc gtccctgcaa 50
<210> 35
   <211> 50
   <212> DNA
   <213> Artificial Sequence
<220>
   <223> Oligonucleotide
<400> 35
   agggaccaat ctggggctgg aaatgttagg aggttgcctt ggtgctgccc 50
<210> 36
   <211> 50
   <212> DNA
   <213> Artificial Sequence
<220>
   <223> Oligonucleotide
<400> 36
   tgggacagtg tggtggtacc aggaagaaag aggattggaa aggccagtac 50
<210> 37
   <211> 50
   <212> DNA
   <213> Artificial Sequence
<220>
   <223> Oligonucleotide
<400> 37
   cctaacaaga attaagcaga gttgggggaa gtgggagggg tgacaagcat 50
<210> 38
   <211> 50
   <212> DNA
   <213> Artificial Sequence
<220>
   <223> Oligonucleotide
<400> 38
   tctgctctcc gccctccttt gtgtatcaag tgtcgctcac agccccattc 50
<210> 39
   <211> 50
   <212> DNA
   <213> Artificial Sequence
<220>
   <223> Oligonucleotide
<400> 39
   gtgactgctg gctctgtcac ctcatcaaac tggatgtgac ccatgccgcc 50
<210> 40
   <211> 50
   <212> DNA
   <213> Artificial Sequence
<220>
   <223> Oligonucleotide
<400> 40
   ggagcagggg caggcgaacc tctttctttg cagaccgaac agtgaaaagc 50
<210> 41
   <211> 50
   <212> DNA
   <213> Artificial Sequence
<220>
   <223> Oligonucleotide
<400> 41
   caggccttcc ctgacccagc caggaacaaa caagggacca agtgcacaca 50
<210> 42
   <211> 50
   <212> DNA
   <213> Artificial Sequence
<220>
   <223> Oligonucleotide
<400> 42
   actgcagaac tgacattttg acggtctacc agcgtggcgg ctggtgttgg 50
<210> 43
   <211> 50
   <212> DNA
   <213> Artificial Sequence
<220>
   <223> Oligonucleotide
<400> 43
   ttgctccggg acgatgagag tatttcccat gctggagtcg gcgagaaggg 50
<210> 44
   <211> 50
   <212> DNA
   <213> Artificial Sequence
<220>
   <223> Oligonucleotide
<400> 44
   cagtgtttcg tgaaggtgtt ggagaggggc tgtgtctggg tgagggatgg 50
<210> 45
   <211> 50
   <212> DNA
   <213> Artificial Sequence
<220>
   <223> Oligonucleotide
<400> 45
   gagtcgtgat tgtaccactg cattcctgct gagcaacaga gtgagacccc 50
<210> 46
   <211> 50
   <212> DNA
   <213> Artificial Sequence
<220>
   <223> Oligonucleotide
<400> 46
   cgaagtcaga aaactcatca tcaggcgacg ccctggcggc tgggtggaga 50
<210> 47
   <211> 50
   <212> DNA
   <213> Artificial Sequence
<220>
   <223> Oligonucleotide
<400> 47
   ggagtttgga actctaccct ggtaggaaag caccgcagca tgtggggaag 50
<210> 48
   <211> 50
   <212> DNA
   <213> Artificial Sequence
<220>
   <223> Oligonucleotide
<400> 48
   ccctggctta tgagcaggct gagagtgaca tcatgaagag acagcccaga 50
<210> 49
   <211> 50
   <212> DNA
   <213> Artificial Sequence
<220>
   <223> Oligonucleotide
<400> 49
   cccaaccctg ctgttaggcc tgctgttccc tttgctcttg attaggagag 50
<210> 50
   <211> 50
   <212> DNA
   <213> Artificial Sequence
<220>
   <223> Oligonucleotide
<400> 50
   gcctcttcct ctgaatagac cagacgccct ttcacttagt tcagtgccag 50
<210> 51
   <211> 50
   <212> DNA
   <213> Artificial Sequence
<220>
   <223> Oligonucleotide
<400> 51
   gcattcatcg tgaggggtct ttgtcctctg tactgtctct ctccttgccc 50
<210> 52
   <211> 50
   <212> DNA
   <213> Artificial Sequence
<220>
   <223> Oligonucleotide
<400> 52
   cagcttgtct cttgcctgcc actgtgtgaa tcggcgacgg agcactgcac 50
<210> 53
   <211> 50
   <212> DNA
   <213> Artificial Sequence
<220>
   <223> Oligonucleotide
<400> 53
   ggtccagagt acttgttttc ccgatgtgtc cagccagctc cgcagcagct 50
<210> 54
   <211> 50
   <212> DNA
   <213> Artificial Sequence
<220>
   <223> Oligonucleotide
<400> 54
   cctgcctctg tttttccctc gagaccagcc aactctcaca tttcagtccg 50
<210> 55
   <211> 50
   <212> DNA
   <213> Artificial Sequence
<220>
   <223> Oligonucleotide
<400> 55
   tgtcttcacg gcagcgtttt gctcacacag cagcttttgc acgccccagg 50
<210> 56
   <211> 50
   <212> DNA
   <213> Artificial Sequence
<220>
   <223> Oligonucleotide
<400> 56
   gccttaggct tcgatggttc ttccaacccc cttaatatgg cttagggtgg 50
<210> 57
   <211> 50
   <212> DNA
   <213> Artificial Sequence
<220>
   <223> Oligonucleotide
<400> 57
   ggcctgaaga tgggagattc ctaagtggag gagaactgtg ccttactgac 50
<210> 58
   <211> 50
   <212> DNA
   <213> Artificial Sequence
<220>
   <223> Oligonucleotide
<400> 58
   gtactgcttt cagtgtgttc cccctcagcc cctccggcgt gtcaggcata 50
<210> 59
   <211> 50
   <212> DNA
   <213> Artificial Sequence
<220>
   <223> Oligonucleotide
<400> 59
   cagagctggc atttgcacaa acacggcaac actgggtggc atccaagtct 50
<210> 60
   <211> 50
   <212> DNA
   <213> Artificial Sequence
<220>
   <223> Oligonucleotide
<400> 60
   acgtcatagc tccttagttc tgctcctgtc gccctaactt ggcatgggca 50
<210> 61
   <211> 50
   <212> DNA
   <213> Artificial Sequence
<220>
   <223> Oligonucleotide
<400> 61
   ccctcctgcc ctctgtccat tcttagagca taccttcatc cactccatgc 50
<210> 62
   <211> 50
   <212> DNA
   <213> Artificial Sequence
<220>
   <223> Oligonucleotide
<400> 62
   ccaaacacca agtgttagct gccttaaggt ttatccgggg cattggtggc 50
<210> 63
   <211> 50
   <212> DNA
   <213> Artificial Sequence
<220>
   <223> Oligonucleotide
<400> 63
   acctggtcag cctaaatctt cccagtcccg ctgtggagct gtcagtcacc 50
<210> 64
   <211> 50
   <212> DNA
   <213> Artificial Sequence
<220>
   <223> Oligonucleotide
<400> 64
   aaagaagtgg agcgtgtcct gaaggagttc caccaggccg ggaagcccat 50
<210> 65
   <211> 50
   <212> DNA
   <213> Artificial Sequence
<220>
   <223> Oligonucleotide
<400> 65
   cagccacatt tcagacctgc tgagactgct gagtgaggaa tggcagtgag 50
<210> 66
   <211> 50
   <212> DNA
   <213> Artificial Sequence
<220>
   <223> Oligonucleotide
<400> 66
   gaccagaaaa acaaggtggt cacgacccca gccttcatgt gcgagacggc 50
<210> 67
   <211> 50
   <212> DNA
   <213> Artificial Sequence
<220>
   <223> Oligonucleotide
<400> 67
   gcccctttag tccccgctct ggaaggccag gcagtttagg tgtaaatagg 50
<210> 68
   <211> 50
   <212> DNA
   <213> Artificial Sequence
<220>
   <223> Oligonucleotide
<400> 68
   accaagatcg caccactgca ttccagccta agcaatagag cgagactccc 50
<210> 69
   <211> 50
   <212> DNA
   <213> Artificial Sequence
<220>
   <223> Oligonucleotide
<400> 69
   gtggatggca gttttctgta gttttgggga ctgtggtagc tcttggattg 50
<210> 70
   <211> 50
   <212> DNA
   <213> Artificial Sequence
<220>
   <223> Oligonucleotide
<400> 70
   ggcagcactt atgctctgtg acagtattgt gtgtcatagt tgagcagtag 50
<210> 71
   <211> 50
   <212> DNA
   <213> Artificial Sequence
<220>
   <223> Oligonucleotide
<400> 71
   cttcttggcc aggggaaagg accacaaggc aatctggggt gtggacagac 50
<210> 72
   <211> 50
   <212> DNA
   <213> Artificial Sequence
<220>
   <223> Oligonucleotide
<400> 72
   tgctgtctgt atcccttgga gtaagaaggt agtggcatgg gtggagtgtg 50
<210> 73
   <211> 50
   <212> DNA
   <213> Artificial Sequence
<220>
   <223> Oligonucleotide
<400> 73
   ggtctcagcc agccctagag actgcttctt gtgtttgtgt cattctgtcc 50
<210> 74
   <211> 50
   <212> DNA
   <213> Artificial Sequence
<220>
   <223> Oligonucleotide
<400> 74
   tcccctgtac ctgtgccaag cctagcactt gtgatgcctc catgccccga 50
<210> 75
   <211> 50
   <212> DNA
   <213> Artificial Sequence
<220>
   <223> Oligonucleotide
<400> 75
   aggagtggct gcagctgact atgtattcct gaactggagc cccagacccg 50
<210> 76
   <211> 50
   <212> DNA
   <213> Artificial Sequence
<220>
   <223> Oligonucleotide
<400> 76
   acttgagtgg aatcctttcc tcacgtactc ccacagacgt ctgggcctgg 50
<210> 77
   <211> 50
   <212> DNA
   <213> Artificial Sequence
<220>
   <223> Oligonucleotide
<400> 77
   actccaaggg ccaaagctca aatgcccacc atagaacgac tgtccatgac 50
<210> 78
   <211> 50
   <212> DNA
   <213> Artificial Sequence
<220>
   <223> Oligonucleotide
<400> 78
   aaggatctgt gttagtccct gggatggctc caaggcctgc tctaggaagg 50
<210> 79
   <211> 50
   <212> DNA
   <213> Artificial Sequence
<220>
   <223> Oligonucleotide
<400> 79
   tgacttgagt ccacaaggac acaaacacct gagtagctgg gcagcccttg 50
<210> 80
   <211> 50
   <212> DNA
   <213> Artificial Sequence
<220>
   <223> Oligonucleotide
<400> 80
   gggagcagag cttttcccta gcacccactt tcccaaacca gtctctgcag 50
<210> 81
   <211> 50
   <212> DNA
   <213> Artificial Sequence
<220>
   <223> Oligonucleotide
<400> 81
   agggcggcac cgatcaccga gcagccgtgc gtgtatctca aggaactaaa 50
<210> 82
   <211> 50
   <212> DNA
   <213> Artificial Sequence
<220>
   <223> Oligonucleotide
<400> 82
   agatgtcctc agacgggaag gtttgagaag ggtcagatgg taggcgggcc 50
<210> 83
   <211> 50
   <212> DNA
   <213> Artificial Sequence
<220>
   <223> Oligonucleotide
<400> 83
   aagctttcgt gtgggagcca gctatggtgc ggatcaatgc gctgacagca 50
<210> 84
   <211> 50
   <212> DNA
   <213> Artificial Sequence
<220>
   <223> Oligonucleotide
<400> 84
   gcccaggggg gtacatggta tggagtagac atcaacaacg aggacattgc 50
<210> 85
   <211> 50
   <212> DNA
   <213> Artificial Sequence
<220>
   <223> Oligonucleotide
<400> 85
   aaagtggagc agaggaagct gctggaaaag tgtgccatga ccgctctgag 50
<210> 86
   <211> 50
   <212> DNA
   <213> Artificial Sequence
<220>
   <223> Oligonucleotide
<400> 86
   agagtcctgg ggagcttctg tccacctgtc ctgcagagga gtcgtttcca 50
<210> 87
   <211> 50
   <212> DNA
   <213> Artificial Sequence
<220>
   <223> Oligonucleotide
<400> 87
   cactgctgta cccagatgcc tacaaccatc cctgccacat acaggtgctc 50
<210> 88
   <211> 50
   <212> DNA
   <213> Artificial Sequence
<220>
   <223> Oligonucleotide
<400> 88
   gttcccagca gggggagaaa cccttcacac cccaggccct tcaggaactg 50
<210> 89
   <211> 50
   <212> DNA
   <213> Artificial Sequence
<220>
   <223> Oligonucleotide
<400> 89
   ccccgttcct gacatcacag cagcctccaa cacaaggctc caagacctag 50
<210> 90
   <211> 50
   <212> DNA
   <213> Artificial Sequence
<220>
   <223> Oligonucleotide
<400> 90
   tatgggagca tcggctactg ctggtgtgtc ttccccaacg gcacggaggt 50
<210> 91
   <211> 50
   <212> DNA
   <213> Artificial Sequence
<220>
   <223> Oligonucleotide
<400> 91
   aaggcacagg gagaagggat aaccctacac ccagacccca ggctggacat 50
<210> 92
   <211> 50
   <212> DNA
   <213> Artificial Sequence
<220>
   <223> Oligonucleotide
<400> 92
   caggacgagc tactgctttg gagcgagggt ttcctgcttt tgagttgacc 50
<210> 93
   <211> 50
   <212> DNA
   <213> Artificial Sequence
<220>
   <223> Oligonucleotide
<400> 93
   aagaggctgc ggtgagccaa tttagagccc aaagagcccc gagggaacct 50
<210> 94
   <211> 50
   <212> DNA
   <213> Artificial Sequence
<220>
   <223> Oligonucleotide
<400> 94
   cactgtctca gagaggtttt cctgtgctcg ccctgtttct ctcaggaagc 50
<210> 95
   <211> 50
   <212> DNA
   <213> Artificial Sequence
<220>
   <223> Oligonucleotide
<400> 95
   actctgcttc tctctgtcag cgtcctgctg ctctagaaga ctgtccgtgg 50
<210> 96
   <211> 50
   <212> DNA
   <213> Artificial Sequence
<220>
   <223> Oligonucleotide
<400> 96
   gtacaggctt ttagcaccga agtgtggtcc tcagaccagt gcctgccaac 50
<210> 97
   <211> 50
   <212> DNA
   <213> Artificial Sequence
<220>
   <223> Oligonucleotide
<400> 97
   cacgtgtgaa catctgtctt ggtcacagag ctgggtgctg ccggtcacct 50
<210> 98
   <211> 50
   <212> DNA
   <213> Artificial Sequence
<220>
   <223> Oligonucleotide
<400> 98
   gaccctgact gctagttctg aggacactgg tggctgtgct atgtgtggcc 50
<210> 99
   <211> 50
   <212> DNA
   <213> Artificial Sequence
<220>
   <223> Oligonucleotide
<400> 99
   gccccgctgg tgcactgaag agccaccctg tggaaacact acatctgcaa 50
<210> 100
   <211> 50
   <212> DNA
   <213> Artificial Sequence
<220>
   <223> Oligonucleotide
<400> 100
   tgggaacggg tggcccggct gtgtgacttt aaccccaagt ctagcaagca 50
<210> 101
   <211> 50
   <212> DNA
   <213> Artificial Sequence
<220>
   <223> Oligonucleotide
<400> 101
   gaggaaagaa attagggcct cctctgatct ctcgctatct gcgggtcctg 50
<210> 102
   <211> 50
   <212> DNA
   <213> Artificial Sequence
<220>
   <223> Oligonucleotide
<400> 102
   tgacgcggat ggtggggaag aacgtgaagc tgtacgacat ggtgctgcag 50
<210> 103
   <211> 50
   <212> DNA
   <213> Artificial Sequence
<220>
   <223> Oligonucleotide
<400> 103
   ccagggcaga gcctctcctt gtactttggc agccatagaa agcgtgctca 50
<210> 104
   <211> 50
   <212> DNA
   <213> Artificial Sequence
<220>
   <223> Oligonucleotide
<400> 104
   aaagcgaggc acactgctta ctgccttggg gttgtggaga tggacccgtg 50
<210> 105
   <211> 50
   <212> DNA
   <213> Artificial Sequence
<220>
   <223> Oligonucleotide
<400> 105
   acgtgctcct gctgaccttc tgcggctccg ggctgtgtcc taaatgcaaa 50
<210> 106
   <211> 50
   <212> DNA
   <213> Artificial Sequence
<220>
   <223> Oligonucleotide
<400> 106
   ggaaacctgc ttctcctact ccggttattg tggcctccca cacagccaac 50
<210> 107
   <211> 50
   <212> DNA
   <213> Artificial Sequence
<220>
   <223> Oligonucleotide
<400> 107
   tgcccctctg tctgctgaag gacctgttgc tgcttctgtc ttttcacccc 50
<210> 108
   <211> 50
   <212> DNA
   <213> Artificial Sequence
<220>
   <223> Oligonucleotide
<400> 108
   ctcccagttc tagagcaatc tacagctgtt tatgtgaggt gcccaacacc 50
<210> 109
   <211> 50
   <212> DNA
   <213> Artificial Sequence
<220>
   <223> Oligonucleotide
<400> 109
   cacagaggtg ttctgacctg caacttgact gggaagcccc tgggtaacac 50
<210> 110
   <211> 50
   <212> DNA
   <213> Artificial Sequence
<220>
   <223> Oligonucleotide
<400> 110
   gcagaattgc ttgaacccag gaggcagagg gttgcagtga gccgagatag 50
<210> 111
   <211> 50
   <212> DNA
   <213> Artificial Sequence
<220>
   <223> Oligonucleotide
<400> 111
   tgttaggtta taatttctca tttggagccg ggcgcagtgg ctcacgcctg 50
<210> 112
   <211> 50
   <212> DNA
   <213> Artificial Sequence
<220>
   <223> Oligonucleotide
<400> 112
   gtccctacag gaggaacagt ggccttgctt cttagacggt cttcactgtg 50
<210> 113
   <211> 50
   <212> DNA
   <213> Artificial Sequence
<220>
   <223> Oligonucleotide
<400> 113
   aagatacact cctgctgtgc ccccatcttt cctccaaact cctgcctgtg 50
<210> 114
   <211> 50
   <212> DNA
   <213> Artificial Sequence
<220>
   <223> Oligonucleotide
<400> 114
   cctcaggcag aggatgttct ggacctcccc ctcttggtcc ctactagaga 50
<210> 115
   <211> 50
   <212> DNA
   <213> Artificial Sequence
<220>
   <223> Oligonucleotide
<400> 115
   cgtgtctcct cggtcgcccc gtgtttgcgc ttgaccatgt tgcactgttt 50
<210> 116
   <211> 50
   <212> DNA
   <213> Artificial Sequence
<220>
   <223> Oligonucleotide
<400> 116
   cctaaccctt gaatgactca aatcagtgcc aggtggagga ctcccatcac 50
<210> 117
   <211> 50
   <212> DNA
   <213> Artificial Sequence
<220>
   <223> Oligonucleotide
<400> 117
   gctcgagaca attaaggacg tgggatgagg ctccgagaca ggacgcggtt 50
<210> 118
   <211> 50
   <212> DNA
   <213> Artificial Sequence
<220>
   <223> Oligonucleotide
<400> 118
   aaaccgtcaa gcccgaggcg gatagagacc acgccagtga ccagttgtag 50
<210> 119
   <211> 50
   <212> DNA
   <213> Artificial Sequence
<220>
   <223> Oligonucleotide
<400> 119
   gaagacggca tcacgaagca gctccaaaag gaaaagcttg ggcggtgccc 50
<210> 120
   <211> 50
   <212> DNA
   <213> Artificial Sequence
<220>
   <223> Oligonucleotide
<400> 120
   atgtagcaga atggcaccca gaccactgcc caccagtgac ggacatgcac 50
<210> 121
   <211> 50
   <212> DNA
   <213> Artificial Sequence
<220>
   <223> Oligonucleotide
<400> 121
   ctgcaggggt cctctgtgaa cttgctcagg acaaggaagc tgcagaagct 50
<210> 122
   <211> 50
   <212> DNA
   <213> Artificial Sequence
<220>
   <223> Oligonucleotide
<400> 122
   agctgcaggg gtcctctgtg aacttgctca ggacaaggaa gctgcagaag 50
<210> 123
   <211> 50
   <212> DNA
   <213> Artificial Sequence
<220>
   <223> Oligonucleotide
<400> 123
   cctggatgcg gggctctttg ttctccagca catctgctac atcatggccg 50
<210> 124
   <211> 50
   <212> DNA
   <213> Artificial Sequence
<220>
   <223> Oligonucleotide
<400> 124
   gacacttgca cagcatggct ctgcctcaca atgatgcagt cagccacctg 50
<210> 125
   <211> 50
   <212> DNA
   <213> Artificial Sequence
<220>
   <223> Oligonucleotide
<400> 125
   gttaggagga gactcttgat gtcaccttca gtatcttgaa agcgggtccc 50
<210> 126
   <211> 50
   <212> DNA
   <213> Artificial Sequence
<220>
   <223> Oligonucleotide
<400> 126
   tgtctgtcat ccagctccta tgtctgttat ccagctccaa gtacagcttg 50
<210> 127
   <211> 50
   <212> DNA
   <213> Artificial Sequence
<220>
   <223> Oligonucleotide
<400> 127
   attgtcattc ctgtattcac ccgtccagac cttgttcaca ctctcacatg 50
<210> 128
   <211> 50
   <212> DNA
   <213> Artificial Sequence
<220>
   <223> Oligonucleotide
<400> 128
   ccatctggga aaatacccca tcattcatgc tactgccaac ctggggagcc 50
<210> 129
   <211> 50
   <212> DNA
   <213> Artificial Sequence
<220>
   <223> Oligonucleotide
<400> 129
   gggcacgcgc tgcactccgt aactcaacat ggcatgcctt tctctccgta 50
<210> 130
   <211> 50
   <212> DNA
   <213> Artificial Sequence
<220>
   <223> Oligonucleotide
<400> 130
   taggttttgt ggcatccacg gtcaggtgta gaggaagctg ccccttgcag 50
<210> 131
   <211> 50
   <212> DNA
   <213> Artificial Sequence
<220>
   <223> Oligonucleotide
<400> 131
   gtcaccatcc acgaagctgc tgtctctggc acatctccac aattaactcc 50
<210> 132
   <211> 50
   <212> DNA
   <213> Artificial Sequence
<220>
   <223> Oligonucleotide
<400> 132
   ggaaagccag ctccccttct cctctaacta ttggaacgcc agaaagtcag 50
<210> 133
   <211> 50
   <212> DNA
   <213> Artificial Sequence
<220>
   <223> Oligonucleotide
<400> 133
   cttccaaaga cccactagaa tgtcagctgt actctgtact ctccactgag 50
<210> 134
   <211> 50
   <212> DNA
   <213> Artificial Sequence
<220>
   <223> Oligonucleotide
<400> 134
   tacctgccct cctactacca cctgcatgtg cacttcaccg ccctgggctt 50
<210> 135
   <211> 50
   <212> DNA
   <213> Artificial Sequence
<220>
   <223> Oligonucleotide
<400> 135
   gacagcttac tgcagcactg ttggtgttcg gagctcttct gtgccctggc 50
<210> 136
   <211> 50
   <212> DNA
   <213> Artificial Sequence
<220>
   <223> Oligonucleotide
<400> 136
   accaagggag aaccaggaaa cggaaacaga gtggtcattc cccagcccgg 50
<210> 137
   <211> 50
   <212> DNA
   <213> Artificial Sequence
<220>
   <223> Oligonucleotide
<400> 137
   gttcagggca caggccccga catcacccca aggacaacgg cacaagtaga 50
<210> 138
   <211> 50
   <212> DNA
   <213> Artificial Sequence
<220>
   <223> Oligonucleotide
<400> 138
   agtccaggtt caagactagc ctgggcaaca tggcaagacc ctgtccctat 50
<210> 139
   <211> 50
   <212> DNA
   <213> Artificial Sequence
<220>
   <223> Oligonucleotide
<400> 139
   gtctgttgct gctgaaagat gtctgtgtgc ctgtatcaac atgtgacttc 50
<210> 140
   <211> 50
   <212> DNA
   <213> Artificial Sequence
<220>
   <223> Oligonucleotide
<400> 140
   aagcaaaagg atggcttgag cccaagagtt cagagcagcc tggccaccat 50
<210> 141
   <211> 50
   <212> DNA
   <213> Artificial Sequence
<220>
   <223> Oligonucleotide
<400> 141
   ctcctggctt cagtccttac aatgtcagta aaacagcctt gctgggcctc 50
<210> 142
   <211> 50
   <212> DNA
   <213> Artificial Sequence
<220>
   <223> Oligonucleotide
<400> 142
   cagccccatt ccagggtccc atctgtccgg agttgtgtat gtcaagtccg 50
<210> 143
   <211> 50
   <212> DNA
   <213> Artificial Sequence
<220>
   <223> Oligonucleotide
<400> 143
   agcgcctcca cctggcctac tctgttattt ccacctgttt gggtagggcc 50
<210> 144
   <211> 50
   <212> DNA
   <213> Artificial Sequence
<220>
   <223> Oligonucleotide
<400> 144
   gcagcagagg acccaggacc acagtgacac acgaaaggaa acaggctaag 50
<210> 145
   <211> 50
   <212> DNA
   <213> Artificial Sequence
<220>
   <223> Oligonucleotide
<400> 145
   catttctgta aggcaatctt ggcacacgtg gggcttacca gtggcccagg 50
<210> 146
   <211> 50
   <212> DNA
   <213> Artificial Sequence
<220>
   <223> Oligonucleotide
<400> 146
   gctacaagtc tatcttcttt cttgacccat ttcaggaggg agccctctcc 50
<210> 147
   <211> 50
   <212> DNA
   <213> Artificial Sequence
<220>
   <223> Oligonucleotide
<400> 147
   gccagggttg gagccgacga ccagaaaatt gcagcaggca ctttaaggca 50
<210> 148
   <211> 50
   <212> DNA
   <213> Artificial Sequence
<220>
   <223> Oligonucleotide
<400> 148
   aattcccagg tgttgtggta ggtaattgaa tcatgggggc agtttccctc 50
<210> 149
   <211> 50
   <212> DNA
   <213> Artificial Sequence
<220>
   <223> Oligonucleotide
<400> 149
   ctaaaggcta cgtcttccag atggagatga ttgttcgggc aagacagttg 50
<210> 150
   <211> 50
   <212> DNA
   <213> Artificial Sequence
<220>
   <223> Oligonucleotide
<400> 150
   agccgtgggg ccaatttttt aactcagatc ttgctgagac caggagcatc 50
<210> 151
   <211> 50
   <212> DNA
   <213> Artificial Sequence
<220>
   <223> Oligonucleotide
<400> 151
   ggacgaagaa gattacgact cctagcgcct tctgcccccc agaccatagc 50
<210> 152
   <211> 50
   <212> DNA
   <213> Artificial Sequence
<220>
   <223> Oligonucleotide
<400> 152
   aatatgacta ttctaaaggc tgtgaggcca tggggtattg gttaagttgc 50
<210> 153
   <211> 50
   <212> DNA
   <213> Artificial Sequence
<220>
   <223> Oligonucleotide
<400> 153
   gcgctgggtc aagcagacaa acaccgagaa gaaggccagt gtggtaacct 50
<210> 154
   <211> 50
   <212> DNA
   <213> Artificial Sequence
<220>
   <223> Oligonucleotide
<400> 154
   agccttagtc caggggtgtg gctctgtccg ggtgcagtat gcagtcatgt 50
<210> 155
   <211> 50
   <212> DNA
   <213> Artificial Sequence
<220>
   <223> Oligonucleotide
<400> 155
   catggcagtc gcttggaacc cactcacacc aatccagtga ccgtgtgtgg 50
<210> 156
   <211> 50
   <212> DNA
   <213> Artificial Sequence
<220>
   <223> Oligonucleotide
<400> 156
   agaaaggcac aggactcgct aagtgttcgc tacgcggggc taccggatcg 50
<210> 157
   <211> 50
   <212> DNA
   <213> Artificial Sequence
<220>
   <223> Oligonucleotide
<400> 157
   atggcagagg tggaggagac actgaagcga ctgcagagcc agaagggagt 50
<210> 158
   <211> 50
   <212> DNA
   <213> Artificial Sequence
<220>
   <223> Oligonucleotide
<400> 158
   cattcggtgg ccgagagcct caactacgtg gcgtcctgga acatgagcat 50
<210> 159
   <211> 50
   <212> DNA
   <213> Artificial Sequence
<220>
   <223> Oligonucleotide
<400> 159
   agcaggtcag aggctcctaa ctgggcaact caagattctg gcttctactg 50
<210> 160
   <211> 50
   <212> DNA
   <213> Artificial Sequence
<220>
   <223> Oligonucleotide
<400> 160
   cagcagatca gtgggatgag ggagactgtt cacctgctgt gtactcctgt 50
<210> 161
   <211> 50
   <212> DNA
   <213> Artificial Sequence
<220>
   <223> Oligonucleotide
<400> 161
   aagtggctcc gtgaagcaga ggaggagtct gaccacaact gagggctggt 50
<210> 162
   <211> 50
   <212> DNA
   <213> Artificial Sequence
<220>
   <223> Oligonucleotide
<400> 162
   cggtggcagt gggtgcctgt agtgtgatgt gtctgaacta ataaagtggc 50
<210> 163
   <211> 50
   <212> DNA
   <213> Artificial Sequence
<220>
   <223> Oligonucleotide
<400> 163
   gcacccagcg gaatgtgctt agtatttggt caccagccgt catcctgggc 50
<210> 164
   <211> 50
   <212> DNA
   <213> Artificial Sequence
<220>
   <223> Oligonucleotide
<400> 164
   tgcagctgtc taggtctgcg gccacatctt ggggacacac tggactgttc 50
<210> 165
   <211> 50
   <212> DNA
   <213> Artificial Sequence
<220>
   <223> Oligonucleotide
<400> 165
   aagagcacgg tcccccagga ggcagctcag gataggtggt atggagctgt 50
<210> 166
   <211> 50
   <212> DNA
   <213> Artificial Sequence
<220>
   <223> Oligonucleotide
<400> 166
   agcgtttggt gttaccttct cctgggaggt cctgctgcaa ctcaagttcc 50
<210> 167
   <211> 50
   <212> DNA
   <213> Artificial Sequence
<220>
   <223> Oligonucleotide
<400> 167
   agtgattttg gtggccagta aatgccagcc atttctcaaa cccacctcgg 50
<210> 168
   <211> 50
   <212> DNA
   <213> Artificial Sequence
<220>
   <223> Oligonucleotide
<400> 168
   gcctctctcc ctggacatac gttagcacat tggcattcag tattggtggc 50
<210> 169
   <211> 50
   <212> DNA
   <213> Artificial Sequence
<220>
   <223> Oligonucleotide
<400> 169
   gaaggcggtg caggtcctca tggtgctctc cctcattctc tgctgtctct 50
<210> 170
   <211> 50
   <212> DNA
   <213> Artificial Sequence
<220>
   <223> Oligonucleotide
<400> 170
   gaacttctac agaagccaag ctccctggag ccctgttggc agctctagct 50
<210> 171
   <211> 50
   <212> DNA
   <213> Artificial Sequence
<220>
   <223> Oligonucleotide
<400> 171
   gtgcaagcgc cacggacctg ggactcagca ccgataactc agacttgaat 50
<210> 172
   <211> 50
   <212> DNA
   <213> Artificial Sequence
<220>
   <223> Oligonucleotide
<400> 172
   tcttctgtgt ccctaaggcc tggtacagtg ccaagcacat acttggtatc 50
<210> 173
   <211> 50
   <212> DNA
   <213> Artificial Sequence
<220>
   <223> Oligonucleotide
<400> 173
   gttcgacacc cagtacccct acggtgagaa gcaggatgag ttcaagcgtc 50
<210> 174
   <211> 50
   <212> DNA
   <213> Artificial Sequence
<220>
   <223> Oligonucleotide
<400> 174
   gctggtctgg ggatagctgg agcacttact caggtggctg gtgaaatgac 50
<210> 175
   <211> 50
   <212> DNA
   <213> Artificial Sequence
<220>
   <223> Oligonucleotide
<400> 175
   gaccttgcaa tttagaatca agcaggtgag acagggagaa gtatgcctgc 50
<210> 176
   <211> 50
   <212> DNA
   <213> Artificial Sequence
<220>
   <223> Oligonucleotide
<400> 176
   cctggaaaaa tggataaagg cgagcaccgt caggagcgca gagatcggcc 50
<210> 177
   <211> 50
   <212> DNA
   <213> Artificial Sequence
<220>
   <223> Oligonucleotide
<400> 177
   cctggacctt tgatggaaca gatgggagga agaagaggag gacgtggagg 50
<210> 178
   <211> 50
   <212> DNA
   <213> Artificial Sequence
<220>
   <223> Oligonucleotide
<400> 178
   gggaacccct tgtgagcatg ctcagtatca ttgtggagaa ccaagagggc 50
<210> 179
   <211> 50
   <212> DNA
   <213> Artificial Sequence
<220>
   <223> Oligonucleotide
<400> 179
   gtgcgagggt agatggttcc tgcacacaga agttaccaca ggggtcaggt 50
<210> 180
   <211> 50
   <212> DNA
   <213> Artificial Sequence
<220>
   <223> Oligonucleotide
<400> 180
   actcaaagct aaggagcagt caggaaccca gataagaaag ccatcctagt 50
<210> 181
   <211> 50
   <212> DNA
   <213> Artificial Sequence
<220>
   <223> Oligonucleotide
<400> 181
   gacagaggct tcaggtacaa ctggccacag agatagtcct ggaagacacg 50
<210> 182
   <211> 50
   <212> DNA
   <213> Artificial Sequence
<220>
   <223> Oligonucleotide
<400> 182
   gacaccgtgc tcccgtctct caggcagcga agttcgatcc atcaaccaaa 50
<210> 183
   <211> 50
   <212> DNA
   <213> Artificial Sequence
<220>
   <223> Oligonucleotide
<400> 183
   ggggccctgg taggctcctt tagaaggacc atttctgttc ctagagctta 50
<210> 184
   <211> 50
   <212> DNA
   <213> Artificial Sequence
<220>
   <223> Oligonucleotide
<400> 184
   gtgcctggtg ccaggtacac ggtcctcttc tcgcacggca atgccgtgga 50
<210> 185
   <211> 50
   <212> DNA
   <213> Artificial Sequence
<220>
   <223> Oligonucleotide
<400> 185
   ccagaaacct cttgtgttct tgcctaggcc caggtgttcc tggcagccaa 50
<210> 186
   <211> 50
   <212> DNA
   <213> Artificial Sequence
<220>
   <223> Oligonucleotide
<400> 186
   catccccaga gacctcttgt gttcctgcca catagctgcc agggcttaag 50
<210> 187
   <211> 50
   <212> DNA
   <213> Artificial Sequence
<220>
   <223> Oligonucleotide
<400> 187
   gctgcattgc tctgctgagc tgtattgaaa ccatgactgg gcccactgtc 50
<210> 188
   <211> 50
   <212> DNA
   <213> Artificial Sequence
<220>
   <223> Oligonucleotide
<400> 188
   ggctgactcc cagccctgac ttgaaaccat tagcgctaac ttgctctgtt 50
<210> 189
   <211> 50
   <212> DNA
   <213> Artificial Sequence
<220>
   <223> Oligonucleotide
<400> 189
   gaggccctga ctaccctgga agtagcaggc cgcatgcttg gaggtaaagt 50
<210> 190
   <211> 50
   <212> DNA
   <213> Artificial Sequence
<220>
   <223> Oligonucleotide
<400> 190
   gactcctgcc ccggttcaac cctaccagct tgtggtaact tactgtcaca 50
<210> 191
   <211> 50
   <212> DNA
   <213> Artificial Sequence
<220>
   <223> Oligonucleotide
<400> 191
   gccatgtcac cgagccccat tgattcccag agggtcttag tcctggaaag 50
<210> 192
   <211> 50
   <212> DNA
   <213> Artificial Sequence
<220>
   <223> Oligonucleotide
<400> 192
   tccccatctt cctggttctg ctccttcgtg gcctagtctt gtggacacca 50
<210> 193
   <211> 50
   <212> DNA
   <213> Artificial Sequence
<220>
   <223> Oligonucleotide
<400> 193
   cctgggcatg gaatcctgtg gcatccacaa aactaccttc aactccatag 50
<210> 194
   <211> 50
   <212> DNA
   <213> Artificial Sequence
<220>
   <223> Oligonucleotide
<400> 194
   gttctacaac ttgcacaggg taacagagga agtggctgag gcctagagtc 50
<210> 195
   <211> 50
   <212> DNA
   <213> Artificial Sequence
<220>
   <223> Oligonucleotide
<400> 195
   gctgtggtca gtggcttagc tcggacaagg agatgagagc ccatgtgttg 50
<210> 196
   <211> 50
   <212> DNA
   <213> Artificial Sequence
<220>
   <223> Oligonucleotide
<400> 196
   tggaggcatt ttgctgtgtg aggccgatcg ccactgtaaa ggtcctagag 50
<210> 197
   <211> 50
   <212> DNA
   <213> Artificial Sequence
<220>
   <223> Oligonucleotide
<400> 197
   gaatgctggt gagatgcttc atgggactgg gggtctcctg ctcagtctgg 50
<210> 198
   <211> 50
   <212> DNA
   <213> Artificial Sequence
<220>
   <223> Oligonucleotide
<400> 198
   cttttgggtg tggggcaggc agagagggat ggtgtccaga gatacatcac 50
<210> 199
   <211> 50
   <212> DNA
   <213> Artificial Sequence
<220>
   <223> Oligonucleotide
<400> 199
   cagcggaacc gcccaggatc agattgcatg tgactctgaa gctgacgaac 50
<210> 200
   <211> 50
   <212> DNA
   <213> Artificial Sequence
<220>
   <223> Oligonucleotide
<400> 200
   gctggaaaaa ggaccctgaa gaacgcccca cttttgagta cttgcagagc 50
<210> 201
   <211> 50
   <212> DNA
   <213> Artificial Sequence
<220>
   <223> Oligonucleotide
<400> 201
   gttgtgtctg gagaagaagc tgggtcaggg gtgtttcgct gaagtgtggc 50
<210> 202
   <211> 50
   <212> DNA
   <213> Artificial Sequence
<220>
   <223> Oligonucleotide
<400> 202
   gccgcctagt agttccctgt cacaaaggga tgccaaggct taccgatctg 50
<210> 203
   <211> 50
   <212> DNA
   <213> Artificial Sequence
<220>
   <223> Oligonucleotide
<400> 203
   gttttgtaca gtgcctggca ctctgtgggt gctcaataaa tggataggag 50
<210> 204
   <211> 50
   <212> DNA
   <213> Artificial Sequence
<220>
   <223> Oligonucleotide
<400> 204
   ctctgggtca atcggaaggc gctatgccag gactgatgag attggcgtgg 50
<210> 205
   <211> 50
   <212> DNA
   <213> Artificial Sequence
<220>
   <223> Oligonucleotide
<400> 205
   gccatgctga gagctgggct ttcctctgtg accatcccgg cctgtaacat 50
<210> 206
   <211> 50
   <212> DNA
   <213> Artificial Sequence
<220>
   <223> Oligonucleotide
<400> 206
   gctgagagct gggctttcct ctgtgaccat cccggcctgt aacatatctg 50
<210> 207
   <211> 50
   <212> DNA
   <213> Artificial Sequence
<220>
   <223> Oligonucleotide
<400> 207
   gcctgtccag ctccctctcc ccaagaaaca acatgaatga gcaacttcag 50
<210> 208
   <211> 50
   <212> DNA
   <213> Artificial Sequence
<220>
   <223> Oligonucleotide
<400> 208
   agttggtctt ggtgtcatat ggatcagagg cacaagtgca gaggctgtgg 50
<210> 209
   <211> 50
   <212> DNA
   <213> Artificial Sequence
<220>
   <223> Oligonucleotide
<400> 209
   tcctcaggtg actggggact tggaacccta ggacctgaac aaccaagact 50
<210> 210
   <211> 50
   <212> DNA
   <213> Artificial Sequence
<220>
   <223> Oligonucleotide
<400> 210
   gcgcatgcac cttcgtcagt acgagctgct ctaagaaggg aacccccaaa 50
<210> 211
   <211> 50
   <212> DNA
   <213> Artificial Sequence
<220>
   <223> Oligonucleotide
<400> 211
   gctgcctcct cctggctgtt tttgtgcctg tttgaagcta ctgctgcctc 50
<210> 212
   <211> 50
   <212> DNA
   <213> Artificial Sequence
<220>
   <223> Oligonucleotide
<400> 212
   ggtgccacag ttttaaacca gaaggtggca ctctgtggct ccttgtagta 50
<210> 213
   <211> 50
   <212> DNA
   <213> Artificial Sequence
<220>
   <223> Oligonucleotide
<400> 213
   gccccaggag ctgctgagac ggctgaaaag tcttccacta agaaggcagt 50
<210> 214
   <211> 50
   <212> DNA
   <213> Artificial Sequence
<220>
   <223> Oligonucleotide
<400> 214
   ctaaaggaag ggcctctgct gactcctacc agagcatccg tccagctcag 50
<210> 215
   <211> 50
   <212> DNA
   <213> Artificial Sequence
<220>
   <223> Oligonucleotide
<400> 215
   ctggatcaga gaccctgcct ctgtttgacc ccgcactgac tgaataaagc 50
<210> 216
   <211> 50
   <212> DNA
   <213> Artificial Sequence
<220>
   <223> Oligonucleotide
<400> 216
   agcagcgcga ggccagagtc caataaactc gtgctcatct gcagcctcct 50
<210> 217
   <211> 50
   <212> DNA
   <213> Artificial Sequence
<220>
   <223> Oligonucleotide
<400> 217
   ggccctgcca ccagaaagtc gagcactggt cctagtcagg ctgtgatgaa 50
<210> 218
   <211> 50
   <212> DNA
   <213> Artificial Sequence
<220>
   <223> Oligonucleotide
<400> 218
   gccaacattc agtctggtat gtgaggcgtg cgtgaagcaa gaactcctgg 50
<210> 219
   <211> 50
   <212> DNA
   <213> Artificial Sequence
<220>
   <223> Oligonucleotide
<400> 219
   gtagattgct ggcctgttgt aggtggtagg gacacagatg accgacctgg 50
<210> 220
   <211> 50
   <212> DNA
   <213> Artificial Sequence
<220>
   <223> Oligonucleotide
<400> 220
   ggccacacag ccaagaagag cccatgcaac tcagtccagg attttactgg 50
<210> 221
   <211> 50
   <212> DNA
   <213> Artificial Sequence
<220>
   <223> Oligonucleotide
<400> 221
   ttcctggcct cccctgagta cgtgaacctc cccatcaatg gcaacgggaa 50
<210> 222
   <211> 50
   <212> DNA
   <213> Artificial Sequence
<220>
   <223> Oligonucleotide
<400> 222
   tctagggcta gtacttagtt tcacacccgg gagctgggag aaaaaacctg 50
<210> 223
   <211> 50
   <212> DNA
   <213> Artificial Sequence
<220>
   <223> Oligonucleotide
<400> 223
   gagcactcaa cccagaaggc gaagatagct tttggttgta ggcggcttcc 50
<210> 224
   <211> 50
   <212> DNA
   <213> Artificial Sequence
<220>
   <223> Oligonucleotide
<400> 224
   aggtggcagc agccatccgt tattatttcc aatggagacc tagcccaggc 50
<210> 225
   <211> 50
   <212> DNA
   <213> Artificial Sequence
<220>
   <223> Oligonucleotide
<400> 225
   tctttatggt gggggcagac tttgcactta ctgcagtgca acacttgcac 50
<210> 226
   <211> 50
   <212> DNA
   <213> Artificial Sequence
<220>
   <223> Oligonucleotide
<400> 226
   aattagccgg gcgtggtggc aggctcctcg ggaggctgag gcagaaaaat 50
<210> 227
   <211> 50
   <212> DNA
   <213> Artificial Sequence
<220>
   <223> Oligonucleotide
<400> 227
   ccaggaactg agcctaggga gctcatctgg cagcaatggc ttttactcat 50
<210> 228
   <211> 50
   <212> DNA
   <213> Artificial Sequence
<220>
   <223> Oligonucleotide
<400> 228
   cagcgaagtc cgctcccgcg cccaagaagg gctcgaagaa agccgtgact 50
<210> 229
   <211> 50
   <212> DNA
   <213> Artificial Sequence
<220>
   <223> Oligonucleotide
<400> 229
   cccactgggg ggttggggta atattctgtg gtcctcagcc ctgtacctta 50
<210> 230
   <211> 50
   <212> DNA
   <213> Artificial Sequence
<220>
   <223> Oligonucleotide
<400> 230
   cgtgtgtccg tggaaccagt cctagccgcg tgtgacagtc ttgcattctg 50
<210> 231
   <211> 50
   <212> DNA
   <213> Artificial Sequence
<220>
   <223> Oligonucleotide
<400> 231
   ctgatgtgtc tctcacagct tgaaaagcct gagacagctg tcttgtgagg 50
<210> 232
   <211> 50
   <212> DNA
   <213> Artificial Sequence
<220>
   <223> Oligonucleotide
<400> 232
   accgtcctca tcataaagtc tctgcgttct ggccatgacc cccgggccca 50
<210> 233
   <211> 50
   <212> DNA
   <213> Artificial Sequence
<220>
   <223> Oligonucleotide
<400> 233
   cttggatgga cccacgaacg ctcttagctt tctcaggggg tcagcagagt 50
<210> 234
   <211> 50
   <212> DNA
   <213> Artificial Sequence
<220>
   <223> Oligonucleotide
<400> 234
   tacgactact cgccctatgg ctattacggc tacggccccg gctacgacta 50
<210> 235
   <211> 50
   <212> DNA
   <213> Artificial Sequence
<220>
   <223> Oligonucleotide
<400> 235
   gcggcagcag gagcgaccaa ctgatcgcac acatgctttg tttggatatg 50
<210> 236
   <211> 50
   <212> DNA
   <213> Artificial Sequence
<220>
   <223> Oligonucleotide
<400> 236
   ccagagctct aggtgtttag gcagcgtgtg gtgtctgaga ggccatagcg 50
<210> 237
   <211> 50
   <212> DNA
   <213> Artificial Sequence
<220>
   <223> Oligonucleotide
<400> 237
   ggtgaccagc agagtggtta tgggaaggta tccaggcgag gtggtcatca 50
<210> 238
   <211> 50
   <212> DNA
   <213> Artificial Sequence
<220>
   <223> Oligonucleotide
<400> 238
   cccccagtat tgtagagcaa gtcttgtgtt aaaagcccag tgtgacagtg 50
<210> 239
   <211> 50
   <212> DNA
   <213> Artificial Sequence
<220>
   <223> Oligonucleotide
<400> 239
   gcctgccgga tgatgaatgg catgaagctg agtggccgag agattgacgt 50
<210> 240
   <211> 50
   <212> DNA
   <213> Artificial Sequence
<220>
   <223> Oligonucleotide
<400> 240
   acaaccaaca gaactggggt tcccaaccca tcgctcagca gccgcttcag 50
<210> 241
   <211> 50
   <212> DNA
   <213> Artificial Sequence
<220>
   <223> Oligonucleotide
<400> 241
   caagataagc atttctttcc tgagttcagg tgactgagga agagccacaa 50
<210> 242
   <211> 50
   <212> DNA
   <213> Artificial Sequence
<220>
   <223> Oligonucleotide
<400> 242
   tcccactgcc tcctctccag tggtctccca ggtgccagac ccaaaagctt 50
<210> 243
   <211> 50
   <212> DNA
   <213> Artificial Sequence
<220>
   <223> Oligonucleotide
<400> 243
   tccagaaact ggggatggaa tctagacttg tgagcggcgg tggtgcctgc 50
<210> 244
   <211> 50
   <212> DNA
   <213> Artificial Sequence
<220>
   <223> Oligonucleotide
<400> 244
   cctggaggct tgcttgggac tggaggcttg cttggacagt tcctctgtgt 50
<210> 245
   <211> 50
   <212> DNA
   <213> Artificial Sequence
<220>
   <223> Oligonucleotide
<400> 245
   ctgtgcagca gtggctctgt gtgtaaatgc tatgcactga ggatacacaa 50
<210> 246
   <211> 50
   <212> DNA
   <213> Artificial Sequence
<220>
   <223> Oligonucleotide
<400> 246
   aagcctttgt atgtgtcctc agggggcaga ccgactttaa gagggaccag 50
<210> 247
   <211> 50
   <212> DNA
   <213> Artificial Sequence
<220>
   <223> Oligonucleotide
<400> 247
   ctgaggcatc ctgctgtcat gggaaggtct ccgcccaaat gtcagatgca 50
<210> 248
   <211> 50
   <212> DNA
   <213> Artificial Sequence
<220>
   <223> Oligonucleotide
<400> 248
   gaccatccga aacctgcgtc cctggtgatg ttctcaagcc tcggaagtgg 50
<210> 249
   <211> 50
   <212> DNA
   <213> Artificial Sequence
<220>
   <223> Oligonucleotide
<400> 249
   ggagattcac agcaactgat caaagggagt ccagtcaacg tgagcaagcg 50
<210> 250
   <211> 50
   <212> DNA
   <213> Artificial Sequence
<220>
   <223> Oligonucleotide
<400> 250
   aatgctgcag ttcctgatga gatcccccct ctcgagggcg atgaggatgc 50
<210> 251
   <211> 50
   <212> DNA
   <213> Artificial Sequence
<220>
   <223> Oligonucleotide
<400> 251
   gtcccaagta tttccagcag gaagtaatgt cttcctcagc ctcaaccagg 50
<210> 252
   <211> 50
   <212> DNA
   <213> Artificial Sequence
<220>
   <223> Oligonucleotide
<400> 252
   gaacaccacg gacttcctcg acaccatcaa gagcaacctg gacagagccc 50
<210> 253
   <211> 50
   <212> DNA
   <213> Artificial Sequence
<220>
   <223> Oligonucleotide
<400> 253
   gggatttagc caagagcaca gacttggatt ccttctgtcc ctccccacct 50
<210> 254
   <211> 50
   <212> DNA
   <213> Artificial Sequence
<220>
   <223> Oligonucleotide
<400> 254
   tggaagatca gacccagctc cttacccttg tctgccagtt gtaccagggc 50
<210> 255
   <211> 50
   <212> DNA
   <213> Artificial Sequence
<220>
   <223> Oligonucleotide
<400> 255
   gaaccgacag aacatgggct gatcttccca caacaccaca ggactgcagg 50
<210> 256
   <211> 50
   <212> DNA
   <213> Artificial Sequence
<220>
   <223> Oligonucleotide
<400> 256
   ggggccctta ggccgttggg actttgatac ccaggaagaa tacagcgagt 50
<210> 257
   <211> 50
   <212> DNA
   <213> Artificial Sequence
<220>
   <223> Oligonucleotide
<400> 257
   gcagctgctt cggatccaca ctgtatctgt gtcatcccca catgggtcct 50
<210> 258
   <211> 50
   <212> DNA
   <213> Artificial Sequence
<220>
   <223> Oligonucleotide
<400> 258
   ggggctccac acctttgctg tgtgttctgg ggcaacctac taatcctctc 50
<210> 259
   <211> 50
   <212> DNA
   <213> Artificial Sequence
<220>
   <223> Oligonucleotide
<400> 259
   caagaggggc gggctcagag ctttgtcact tgccacatgg tgtctcccaa 50
<210> 260
   <211> 50
   <212> DNA
   <213> Artificial Sequence
<220>
   <223> Oligonucleotide
<400> 260
   ggattcatcc tgggagaggg ggcaaggtgg aatgcagata actcacatgt 50
<210> 261
   <211> 50
   <212> DNA
   <213> Artificial Sequence
<220>
   <223> Oligonucleotide
<400> 261
   cccaaaggcc acatccaaga caggcaataa tgagcagagt ttacagctcc 50
<210> 262
   <211> 50
   <212> DNA
   <213> Artificial Sequence
<220>
   <223> Oligonucleotide
<400> 262
   cctcaacagg cccagggagg gaagtgtgag cgccttggta tgacttaaaa 50
<210> 263
   <211> 50
   <212> DNA
   <213> Artificial Sequence
<220>
   <223> Oligonucleotide
<400> 263
   ggagggcttg aggttggtga ggttaggtgc gtgtttcctg tgcaagtcag 50
<210> 264
   <211> 50
   <212> DNA
   <213> Artificial Sequence
<220>
   <223> Oligonucleotide
<400> 264
   ggagacttga ggagggcttg aggttggtga ggttaggtgc gtgtttcctg 50
<210> 265
   <211> 50
   <212> DNA
   <213> Artificial Sequence
<220>
   <223> Oligonucleotide
<400> 265
   attgcctctg acgtctggtc ttttggagtc actctgcatg agctgctgac 50
<210> 266
   <211> 50
   <212> DNA
   <213> Artificial Sequence
<220>
   <223> Oligonucleotide
<400> 266
   aaaacttctg tactgccctg gaatatgggc tgccccccac agctggctgg 50
<210> 267
   <211> 50
   <212> DNA
   <213> Artificial Sequence
<220>
   <223> Oligonucleotide
<400> 267
   cctctgtgcc tgagttctcc ctgttgtctc aaagcggtac ccatcctccc 50
<210> 268
   <211> 50
   <212> DNA
   <213> Artificial Sequence
<220>
   <223> Oligonucleotide
<400> 268
   gccctgccgg aagcagattg accagcagaa ctgtacctgt tgaggcactt 50
<210> 269
   <211> 50
   <212> DNA
   <213> Artificial Sequence
<220>
   <223> Oligonucleotide
<400> 269
   cctggacagt gtggagtgtt acgacccaga tacagacacc tggagcgagg 50
<210> 270
   <211> 50
   <212> DNA
   <213> Artificial Sequence
<220>
   <223> Oligonucleotide
<400> 270
   gcagatgttg tgggtgccct tctgttcctg gaggattatg ttcggtacac 50
<210> 271
   <211> 50
   <212> DNA
   <213> Artificial Sequence
<220>
   <223> Oligonucleotide
<400> 271
   ctgggccctg taaaatagtg ttactgtaat actctgtttt gcctcctgcc 50
<210> 272
   <211> 50
   <212> DNA
   <213> Artificial Sequence
<220>
   <223> Oligonucleotide
<400> 272
   tcctgcgtct gtcttccctg cttttcagtc gtcgggctta gagaagcctg 50
<210> 273
   <211> 50
   <212> DNA
   <213> Artificial Sequence
<220>
   <223> Oligonucleotide
<400> 273
   gtcaccctgt gcttcctgcc caagatactg acccattgaa cccccaaagc 50
<210> 274
   <211> 50
   <212> DNA
   <213> Artificial Sequence
<220>
   <223> Oligonucleotide
<400> 274
   ttgcttgtgt gcatgtgttg ggtgcatgct tccgggtctc agctgcccca 50
<210> 275
   <211> 50
   <212> DNA
   <213> Artificial Sequence
<220>
   <223> Oligonucleotide
<400> 275
   cggcacgtcc ttggcgtctc taatgtctgc agctcaaggg ctggcacttt 50
<210> 276
   <211> 50
   <212> DNA
   <213> Artificial Sequence
<220>
   <223> Oligonucleotide
<400> 276
   ccaacaaaga tgaagttccc tatctacgga aaggcatgac tgggaggccc 50
<210> 277
   <211> 50
   <212> DNA
   <213> Artificial Sequence
<220>
   <223> Oligonucleotide
<400> 277
   tctggcaccc tcctgaatgg aaccccagag tacctcctgt gtggaagggt 50
<210> 278
   <211> 50
   <212> DNA
   <213> Artificial Sequence
<220>
   <223> Oligonucleotide
<400> 278
   gtgtgtgcgg gcatggatgt gactgggagc tctgctgggc acccacatct 50
<210> 279
   <211> 50
   <212> DNA
   <213> Artificial Sequence
<220>
   <223> Oligonucleotide
<400> 279
   cctcttccaa gccctgcgtc cagcgagcgt cacagcacaa cctgcaaaaa 50
<210> 280
   <211> 50
   <212> DNA
   <213> Artificial Sequence
<220>
   <223> Oligonucleotide
<400> 280
   caagctgacc atttcgaagc ttgctcctgg tgggcacgtg ggacgtttct 50
<210> 281
   <211> 50
   <212> DNA
   <213> Artificial Sequence
<220>
   <223> Oligonucleotide
<400> 281
   accctctgcg accggaggac tatgccccta ctcccggaag ccctcggaca 50
<210> 282
   <211> 50
   <212> DNA
   <213> Artificial Sequence
<220>
   <223> Oligonucleotide
<400> 282
   gtggtagatc acttgaggtc aagagttgtg acaccagcct ggccaacctg 50
<210> 283
   <211> 50
   <212> DNA
   <213> Artificial Sequence
<220>
   <223> Oligonucleotide
<400> 283
   tgatggttct tgctgggcag cttggagaag gcgtgatact ctccagctcc 50
<210> 284
   <211> 50
   <212> DNA
   <213> Artificial Sequence
<220>
   <223> Oligonucleotide
<400> 284
   tagaactatt attgaccacg cctcctccaa gtcccagcga gcccgtgtac 50
<210> 285
   <211> 50
   <212> DNA
   <213> Artificial Sequence
<220>
   <223> Oligonucleotide
<400> 285
   gaatgagctc agtgaccact tggatgctat ggactccaac ctggataacc 50
<210> 286
   <211> 50
   <212> DNA
   <213> Artificial Sequence
<220>
   <223> Oligonucleotide
<400> 286
   cacttaacat agtgacctcc agttccatcc atgctgtcgc aagtgacagg 50
<210> 287
   <211> 50
   <212> DNA
   <213> Artificial Sequence
<220>
   <223> Oligonucleotide
<400> 287
   agataaaggg gccaccaagg agtcgagtga gaaggatcgc ggccgggaca 50
<210> 288
   <211> 50
   <212> DNA
   <213> Artificial Sequence
<220>
   <223> Oligonucleotide
<400> 288
   tgcagccagt cactgcacct ccgtcctacc ctcctaccag ctatgatcag 50
<210> 289
   <211> 50
   <212> DNA
   <213> Artificial Sequence
<220>
   <223> Oligonucleotide
<400> 289
   cagagctatt gcaccatgag cgtccttcct ccttcctctc cgggctgcca 50
<210> 290
   <211> 50
   <212> DNA
   <213> Artificial Sequence
<220>
   <223> Oligonucleotide
<400> 290
   ccctttcctc tccctcagaa tttgtgtttg ctgcctctat cttgtttttt 50
<210> 291
   <211> 50
   <212> DNA
   <213> Artificial Sequence
<220>
   <223> Oligonucleotide
<400> 291
   tcctttccaa ctctacctcc ctcactcgag ctcctttccc ctgatcagag 50
<210> 292
   <211> 50
   <212> DNA
   <213> Artificial Sequence
<220>
   <223> Oligonucleotide
<400> 292
   ccacgcgcga aaattcggcc agggttctcg ctcttgtcgc gtctgctcaa 50
<210> 293
   <211> 50
   <212> DNA
   <213> Artificial Sequence
<220>
   <223> Oligonucleotide
<400> 293
   agtggcgagg atggcaagaa aagctggcaa cttctatgta cctgcagaac 50
<210> 294
   <211> 50
   <212> DNA
   <213> Artificial Sequence
<220>
   <223> Oligonucleotide
<400> 294
   gtagaaggtg gagatgctgg caacagggag gaccaggtca acaggcttat 50
<210> 295
   <211> 50
   <212> DNA
   <213> Artificial Sequence
<220>
   <223> Oligonucleotide
<400> 295
   agccttgcat gtgcagaaag taaaagccag ggtaggcttg taacctgccc 50
<210> 296
   <211> 50
   <212> DNA
   <213> Artificial Sequence
<220>
   <223> Oligonucleotide
<400> 296
   tttgctcgaa gccttcagtc cgttgcagag gagcgagttg gacgccactg 50
<210> 297
   <211> 50
   <212> DNA
   <213> Artificial Sequence
<220>
   <223> Oligonucleotide
<400> 297
   agggtcctga attcttactg ggttggtgaa gattccacat acaaattttt 50
<210> 298
   <211> 50
   <212> DNA
   <213> Artificial Sequence
<220>
   <223> Oligonucleotide
<400> 298
   tgcttccaac tgcgggacag ggagtggccg tagcggcttg ttggataagt 50
<210> 299
   <211> 50
   <212> DNA
   <213> Artificial Sequence
<220>
   <223> Oligonucleotide
<400> 299
   cggagacggc aaatggcgga cttcgacacc tacgacgatc gggcctacag 50
<210> 300
   <211> 50
   <212> DNA
   <213> Artificial Sequence
<220>
   <223> Oligonucleotide
<400> 300
   tggcttcacg gctggctatg tggacagcaa gagtcgtttt cgcggaagcc 50
<210> 301
   <211> 50
   <212> DNA
   <213> Artificial Sequence
<220>
   <223> Oligonucleotide
<400> 301
   ccctcctgtg agagtctgaa ggatactatt gccagagctc tgccttctgg 50
<210> 302
   <211> 50
   <212> DNA
   <213> Artificial Sequence
<220>
   <223> Oligonucleotide
<400> 302
   gtgcgacctc gatctgtccc aagcattcat gttccctcac cagctgtagt 50
<210> 303
   <211> 50
   <212> DNA
   <213> Artificial Sequence
<220>
   <223> Oligonucleotide
<400> 303
   ggacccggac accctgtggg acctggcctc aaactcacca aatcgctcat 50
<210> 304
   <211> 50
   <212> DNA
   <213> Artificial Sequence
<220>
   <223> Oligonucleotide
<400> 304
   gtcatcaaga gccgctgcca ctggtcctcc gtttactgac ctggctgtgt 50
<210> 305
   <211> 50
   <212> DNA
   <213> Artificial Sequence
<220>
   <223> Oligonucleotide
<400> 305
   tgagcaggga cagtcatttt ttaaatgttt ttggccgggc gtggtggctc 50
<210> 306
   <211> 50
   <212> DNA
   <213> Artificial Sequence
<220>
   <223> Oligonucleotide
<400> 306
   tgggagccca gaagaaatgc tcttttgctt ggagtttgtc atcctacacc 50
<210> 307
   <211> 50
   <212> DNA
   <213> Artificial Sequence
<220>
   <223> Oligonucleotide
<400> 307
   cagccagtgc caacttcgct gccaactttg gtgccattgg tttcttctgg 50
<210> 308
   <211> 50
   <212> DNA
   <213> Artificial Sequence
<220>
   <223> Oligonucleotide
<400> 308
   gtgtctgggc cctggagctg ggatgacatt gagtttgagc tgctgacctg 50
<210> 309
   <211> 50
   <212> DNA
   <213> Artificial Sequence
<220>
   <223> Oligonucleotide
<400> 309
   tgctgtgtga ctatgattcc taagatttcc agggcttaag ggctaacttc 50
<210> 310
   <211> 50
   <212> DNA
   <213> Artificial Sequence
<220>
   <223> Oligonucleotide
<400> 310
   ggccctggtt gaaaagtact catctcctgg tctgacatcc aaagagtcac 50
<210> 311
   <211> 50
   <212> DNA
   <213> Artificial Sequence
<220>
   <223> Oligonucleotide
<400> 311
   gcaggattct gcaaaatgtg tctcacccac tactgagatt gttcagcccc 50
<210> 312
   <211> 50
   <212> DNA
   <213> Artificial Sequence
<220>
   <223> Oligonucleotide
<400> 312
   cctgtttgga tcctggtcct ttttaactgt tccttggtaa ttctgagcat 50
<210> 313
   <211> 50
   <212> DNA
   <213> Artificial Sequence
<220>
   <223> Oligonucleotide
<400> 313
   cctgagccag aagtggggtg cttatactcc caaaccttga gtgtccagcc 50
<210> 314
   <211> 50
   <212> DNA
   <213> Artificial Sequence
<220>
   <223> Oligonucleotide
<400> 314
   caggatgaca atcaggtcat ggtgtctgag ggcatcatct tcctcatctg 50
<210> 315
   <211> 50
   <212> DNA
   <213> Artificial Sequence
<220>
   <223> Oligonucleotide
<400> 315
   ggctttgggt ggttccaatt ggtggagaga agctctgagg cacgtcatgc 50
<210> 316
   <211> 50
   <212> DNA
   <213> Artificial Sequence
<220>
   <223> Oligonucleotide
<400> 316
   ccatctgctg gcacctgagg agagtgagca gcctggacca caagcccagt 50
<210> 317
   <211> 50
   <212> DNA
   <213> Artificial Sequence
<220>
   <223> Oligonucleotide
<400> 317
   cctctagcgg cttccagttc cccgctcctg actcctgacc tccaggatgt 50
<210> 318
   <211> 50
   <212> DNA
   <213> Artificial Sequence
<220>
   <223> Oligonucleotide
<400> 318
   tgcagaagga atggtggatc caagtctcaa tcccatttca gcctttcgac 50
<210> 319
   <211> 50
   <212> DNA
   <213> Artificial Sequence
<220>
   <223> Oligonucleotide
<400> 319
   cttctgccat gattgtgagg cctccccagc catgtggaac tgtgaatcca 50
<210> 320
   <211> 50
   <212> DNA
   <213> Artificial Sequence
<220>
   <223> Oligonucleotide
<400> 320
   gtaggggttt ccagcttccc caggctccgg ccttgtcagt ctctttgcat 50
<210> 321
   <211> 50
   <212> DNA
   <213> Artificial Sequence
<220>
   <223> Oligonucleotide
<400> 321
   ccctctgtgg ttctgactgg agaccccagt gtgggggagg tcttaccatt 50
<210> 322
   <211> 50
   <212> DNA
   <213> Artificial Sequence
<220>
   <223> Oligonucleotide
<400> 322
   caagatggca gcggcgctgc gcgtgcgttg ttgagtgttc gggacgccgg 50
<210> 323
   <211> 50
   <212> DNA
   <213> Artificial Sequence
<220>
   <223> Oligonucleotide
<400> 323
   ggcctgcagg cgccatggtc ttcctcaccg cgcagctctg gctgcggaat 50
<210> 324
   <211> 50
   <212> DNA
   <213> Artificial Sequence
<220>
   <223> Oligonucleotide
<400> 324
   tgctgctcct gctgccccat gagctgtgcc aagtgtgccc agggctgcat 50
<210> 325
   <211> 50
   <212> DNA
   <213> Artificial Sequence
<220>
   <223> Oligonucleotide
<400> 325
   gaacccgcgt gcaacctgtc ccgactctag ccgcctcttc agcacgccat 50
<210> 326
   <211> 50
   <212> DNA
   <213> Artificial Sequence
<220>
   <223> Oligonucleotide
<400> 326
   agaaggaggg tttctggctg tggttctaaa tggagcccca ggaagctgcc 50
<210> 327
   <211> 50
   <212> DNA
   <213> Artificial Sequence
<220>
   <223> Oligonucleotide
<400> 327
   ctatccagaa gaagctggct gcaaaagggc taagggatcc atggggccgc 50
<210> 328
   <211> 50
   <212> DNA
   <213> Artificial Sequence
<220>
   <223> Oligonucleotide
<400> 328
   ccagaagaag ctggctgcaa aagggctaag ggatccatgg ggccgcaatg 50
<210> 329
   <211> 50
   <212> DNA
   <213> Artificial Sequence
<220>
   <223> Oligonucleotide
<400> 329
   ctgagcctgg gtgctcactg tggcggtccc cgtcctggct atgaaacctt 50
<210> 330
   <211> 50
   <212> DNA
   <213> Artificial Sequence
<220>
   <223> Oligonucleotide
<400> 330
   cccatataag ctgctgccac tgcagaggtt tttacctcga cctccaggtg 50
<210> 331
   <211> 50
   <212> DNA
   <213> Artificial Sequence
<220>
   <223> Oligonucleotide
<400> 331
   agctccgggt ggctggttct cagtggttgt ctcatgtctc tttttctgtc 50
<210> 332
   <211> 50
   <212> DNA
   <213> Artificial Sequence
<220>
   <223> Oligonucleotide
<400> 332
   cccggttcat tttatgcgtg cgagaagtca gtggtaactg ctgcagggct 50
<210> 333
   <211> 50
   <212> DNA
   <213> Artificial Sequence
<220>
   <223> Oligonucleotide
<400> 333
   tatcacaatt gccacccatc gggttttggg tgtgtgtttt catagcgtgg 50
<210> 334
   <211> 50
   <212> DNA
   <213> Artificial Sequence
<220>
   <223> Oligonucleotide
<400> 334
   gccagtcttt tttcattgac gccccagatt ccccagccac gttagcctac 50
<210> 335
   <211> 50
   <212> DNA
   <213> Artificial Sequence
<220>
   <223> Oligonucleotide
<400> 335
   catcaggaga aaggctgggt cttgggacct tgtcctcccc agttggccta 50
<210> 336
   <211> 50
   <212> DNA
   <213> Artificial Sequence
<220>
   <223> Oligonucleotide
<400> 336
   cagcttccag tggtggccgt agacttggct cggaacttag tggcaccaga 50
<210> 337
   <211> 50
   <212> DNA
   <213> Artificial Sequence
<220>
   <223> Oligonucleotide
<400> 337
   cgaggaccgc gactctcaac tccgaagtca agcccgcact ctgattacct 50
<210> 338
   <211> 50
   <212> DNA
   <213> Artificial Sequence
<220>
   <223> Oligonucleotide
<400> 338
   agagagactt cctgaagcag cgccccacca agctcaagag cctcatccgc 50
<210> 339
   <211> 50
   <212> DNA
   <213> Artificial Sequence
<220>
   <223> Oligonucleotide
<400> 339
   ctcctgcttc ctccctgcca ttcatccctg cccctctcca tgaagcttga 50
<210> 340
   <211> 50
   <212> DNA
   <213> Artificial Sequence
<220>
   <223> Oligonucleotide
<400> 340
   aaagggttgg aggcagctgg cacaagaggc tgaggcctgg ctgaattacc 50
<210> 341
   <211> 50
   <212> DNA
   <213> Artificial Sequence
<220>
   <223> Oligonucleotide
<400> 341
   ggctattcct ccccagaacc tgacattcaa gactcctctg gaagtgaagc 50
<210> 342
   <211> 50
   <212> DNA
   <213> Artificial Sequence
<220>
   <223> Oligonucleotide
<400> 342
   gttgccaact gttgttccag ccatccacac aggagtctgt tctgaggtgg 50
<210> 343
   <211> 50
   <212> DNA
   <213> Artificial Sequence
<220>
   <223> Oligonucleotide
<400> 343
   cctggagatc agactgttgc tttcgcatga tgtatgtagt gtctcatgac 50
<210> 344
   <211> 50
   <212> DNA
   <213> Artificial Sequence
<220>
   <223> Oligonucleotide
<400> 344
   ggggccccca ccttcaatgt cactgtcacc aagactgaca agacgctagt 50
<210> 345
   <211> 50
   <212> DNA
   <213> Artificial Sequence
<220>
   <223> Oligonucleotide
<400> 345
   ggctggatgg acagacacct ccccctaccc atatccctcc cgtgtgtggt 50
<210> 346
   <211> 50
   <212> DNA
   <213> Artificial Sequence
<220>
   <223> Oligonucleotide
<400> 346
   gctaatccca gtcggtgccg catccccagc ccgccgccat ggccgcctac 50
<210> 347
   <211> 50
   <212> DNA
   <213> Artificial Sequence
<220>
   <223> Oligonucleotide
<400> 347
   attgtcaagc atctggaggg tctctctgaa gaggctatca tggagctgaa 50
<210> 348
   <211> 50
   <212> DNA
   <213> Artificial Sequence
<220>
   <223> Oligonucleotide
<400> 348
   ccatgttgag gggctccatt cccaattcct gggtcaaggt gaattaaccc 50
<210> 349
   <211> 50
   <212> DNA
   <213> Artificial Sequence
<220>
   <223> Oligonucleotide
<400> 349
   cttgcagcag ctctggtggc agctgtcctt gaggaacctt tggtgtgtgg 50
<210> 350
   <211> 50
   <212> DNA
   <213> Artificial Sequence
<220>
   <223> Oligonucleotide
<400> 350
   tagttggctt tgtctgtcag gtgcagtctg gcgggagtcc aggaggcagc 50
<210> 351
   <211> 50
   <212> DNA
   <213> Artificial Sequence
<220>
   <223> Oligonucleotide
<400> 351
   tcctcggacc cgagaacccg aaaattgcca aggacccatc ctggatcatc 50
<210> 352
   <211> 50
   <212> DNA
   <213> Artificial Sequence
<220>
   <223> Oligonucleotide
<400> 352
   agcccaggtc taaatgtaat ggttggttta ttgttctata accccagccc 50
<210> 353
   <211> 50
   <212> DNA
   <213> Artificial Sequence
<220>
   <223> Oligonucleotide
<400> 353
   ccacttgcac ctctccacct ttggcactag aactcctgag acaccacttc 50
<210> 354
   <211> 50
   <212> DNA
   <213> Artificial Sequence
<220>
   <223> Oligonucleotide
<400> 354
   gagtaacggc tctgctgcca gggtttctct gggctcattc ttccactgac 50
<210> 355
   <211> 50
   <212> DNA
   <213> Artificial Sequence
<220>
   <223> Oligonucleotide
<400> 355
   tggctcggat cctcagtgcc tgtgtcttgt caaagcggtg cccgccagac 50
<210> 356
   <211> 50
   <212> DNA
   <213> Artificial Sequence
<220>
   <223> Oligonucleotide
<400> 356
   cgttcaaact gtccactctg atccaaccct gtactgatag tacttcccag 50
<210> 357
   <211> 50
   <212> DNA
   <213> Artificial Sequence
<220>
   <223> Oligonucleotide
<400> 357
   gtaatcctag cacttttgtc gcctgggcga cacaccaagg ctctgtctca 50
<210> 358
   <211> 50
   <212> DNA
   <213> Artificial Sequence
<220>
   <223> Oligonucleotide
<400> 358
   ctccatggcc ctcggccgct tgcacccgct ctctgttgta cactttcaat 50
<210> 359
   <211> 50
   <212> DNA
   <213> Artificial Sequence
<220>
   <223> Oligonucleotide
<400> 359
   tggccaagtt cctccacaag cacgacttgg acctcatctg ccgagcacac 50
<210> 360
   <211> 50
   <212> DNA
   <213> Artificial Sequence
<220>
   <223> Oligonucleotide
<400> 360
   gccaagaaat agcccccgca caccaccctg tgccccagat gatggattga 50
<210> 361
   <211> 50
   <212> DNA
   <213> Artificial Sequence
<220>
   <223> Oligonucleotide
<400> 361
   gctgcccctg agaagagact taatccaagc ctgattgtac tagtggcatc 50
<210> 362
   <211> 50
   <212> DNA
   <213> Artificial Sequence
<220>
   <223> Oligonucleotide
<400> 362
   cgagttcctg aaaaaggaga ctgcacagcg tcgggttctg gaggagtcgg 50
<210> 363
   <211> 50
   <212> DNA
   <213> Artificial Sequence
<220>
   <223> Oligonucleotide
<400> 363
   tccagtcctc acaacctgtc cttcacctag tccctcctga cccagggatg 50
<210> 364
   <211> 50
   <212> DNA
   <213> Artificial Sequence
<220>
   <223> Oligonucleotide
<400> 364
   cttcttccta ggggcctcgt gatctgaggg gtggtgccta cttccactgt 50
<210> 365
   <211> 50
   <212> DNA
   <213> Artificial Sequence
<220>
   <223> Oligonucleotide
<400> 365
   tgatcagctc tgaggtgcaa cttcttcaca tactgtacat acctgtgacc 50
<210> 366
   <211> 50
   <212> DNA
   <213> Artificial Sequence
<220>
   <223> Oligonucleotide
<400> 366
   cagccctgtg tgtgaatcgt ttgtgacgtg tgcaaatggg aaaggagggg 50
<210> 367
   <211> 50
   <212> DNA
   <213> Artificial Sequence
<220>
   <223> Oligonucleotide
<400> 367
   cccagaagaa cctcaggagg taaccttggg cccttccctg ctatcctttt 50
<210> 368
   <211> 50
   <212> DNA
   <213> Artificial Sequence
<220>
   <223> Oligonucleotide
<400> 368
   gattcaccct gtccaaactg cctaagccct ccgccattct caagccctgc 50
<210> 369
   <211> 50
   <212> DNA
   <213> Artificial Sequence
<220>
   <223> Oligonucleotide
<400> 369
   gggaacggat gtggaaggaa gaactgtcac cctcttaagg cccagggtcg 50
<210> 370
   <211> 50
   <212> DNA
   <213> Artificial Sequence
<220>
   <223> Oligonucleotide
<400> 370
   ggagaagctg aatgcaacaa acattgagct agccacagtg cagcctggcc 50
<210> 371
   <211> 50
   <212> DNA
   <213> Artificial Sequence
<220>
   <223> Oligonucleotide
<400> 371
   accctggagc tagtggagga aactgtgcag gctatggagg tggagtaagc 50
<210> 372
   <211> 50
   <212> DNA
   <213> Artificial Sequence
<220>
   <223> Oligonucleotide
<400> 372
   tgcagcccac ggctatggtg ccttcctgac tctcagtatc ctcgaccgat 50
<210> 373
   <211> 50
   <212> DNA
   <213> Artificial Sequence
<220>
   <223> Oligonucleotide
<400> 373
   accagtgaag acattgagga gctggtggaa cctgtggcag cacatggccc 50
<210> 374
   <211> 50
   <212> DNA
   <213> Artificial Sequence
<220>
   <223> Oligonucleotide
<400> 374
   accgggacat ccggctgatg gtcatggaga tccgcaatgc ttatgctgtg 50
<210> 375
   <211> 50
   <212> DNA
   <213> Artificial Sequence
<220>
   <223> Oligonucleotide
<400> 375
   gctcagcttc tccacaaggc tagaaatggg gcacagagcc actggaggcc 50
<210> 376
   <211> 50
   <212> DNA
   <213> Artificial Sequence
<220>
   <223> Oligonucleotide
<400> 376
   tgattttggg gtagcaatcc aggagaaggt gctggagagg gtgaatgccg 50
<210> 377
   <211> 50
   <212> DNA
   <213> Artificial Sequence
<220>
   <223> Oligonucleotide
<400> 377
   ctgcagcttc gtagtactcc cttccggtac ctacttacac cttccatgca 50
<210> 378
   <211> 50
   <212> DNA
   <213> Artificial Sequence
<220>
   <223> Oligonucleotide
<400> 378
   taatctggac attcgaggaa ttggccgctg tcactgcttg ttgtttgcgc 50
<210> 379
   <211> 50
   <212> DNA
   <213> Artificial Sequence
<220>
   <223> Oligonucleotide
<400> 379
   accctgcaga gctatggtga ggtgtggata aggcttaggt gccaggctgt 50
<210> 380
   <211> 50
   <212> DNA
   <213> Artificial Sequence
<220>
   <223> Oligonucleotide
<400> 380
   ggagaagagc aagggttccc tcaagaggaa gtgagcggtg ctgtcctcag 50
<210> 381
   <211> 50
   <212> DNA
   <213> Artificial Sequence
<220>
   <223> Oligonucleotide
<400> 381
   cctagtcttc cttcatcctt gccctctgtt ggcacaggca ttatctctgc 50
<210> 382
   <211> 50
   <212> DNA
   <213> Artificial Sequence
<220>
   <223> Oligonucleotide
<400> 382
   cctgtcccat gttggaagtt gctctgaagg ggtggtagat gctggaagcc 50
<210> 383
   <211> 50
   <212> DNA
   <213> Artificial Sequence
<220>
   <223> Oligonucleotide
<400> 383
   ggtgtcatgt tggatcgctt tgtgactgtt catctgtcct tgacagtggc 50
<210> 384
   <211> 50
   <212> DNA
   <213> Artificial Sequence
<220>
   <223> Oligonucleotide
<400> 384
   aatctcccca gccgacttcc actgggctga cagactttgc tgaccacagg 50
<210> 385
   <211> 50
   <212> DNA
   <213> Artificial Sequence
<220>
   <223> Oligonucleotide
<400> 385
   ccctgtagtc cagtggtgct gccctgttgt gcaaactgct cctttttctc 50
<210> 386
   <211> 50
   <212> DNA
   <213> Artificial Sequence
<220>
   <223> Oligonucleotide
<400> 386
   gctttgagac ctttcctctc ctgggtactg aggtgctatg aagccaactg 50
<210> 387
   <211> 50
   <212> DNA
   <213> Artificial Sequence
<220>
   <223> Oligonucleotide
<400> 387
   agagtcgcgg ggacacagga gtcttcctac agtacacaca cgcccgcctc 50
<210> 388
   <211> 50
   <212> DNA
   <213> Artificial Sequence
<220>
   <223> Oligonucleotide
<400> 388
   catcctaaaa atggggtcca ggcagacccc tccagacctc acactgccga 50
<210> 389
   <211> 50
   <212> DNA
   <213> Artificial Sequence
<220>
   <223> Oligonucleotide
<400> 389
   gctcctgctg caaccgctgt gaatgctgct gagaacctcc ctctatgggg 50
<210> 390
   <211> 50
   <212> DNA
   <213> Artificial Sequence
<220>
   <223> Oligonucleotide
<400> 390
   ggctcacaca gcttaagagt agctgtctct caaacgtgcg ctcacagttg 50
<210> 391
   <211> 50
   <212> DNA
   <213> Artificial Sequence
<220>
   <223> Oligonucleotide
<400> 391
   agcttcgcaa gagcatgtgg aaggaccgga atctggacgt ggtccgcaag 50
<210> 392
   <211> 50
   <212> DNA
   <213> Artificial Sequence
<220>
   <223> Oligonucleotide
<400> 392
   gtgcaaacag acattccaga gagcctgatc cacatccagc agcagagccc 50
<210> 393
   <211> 50
   <212> DNA
   <213> Artificial Sequence
<220>
   <223> Oligonucleotide
<400> 393
   gttgggaatg tgcttggcgc tgaccctgcg ggcatctgac tggtcttcca 50
<210> 394
   <211> 50
   <212> DNA
   <213> Artificial Sequence
<220>
   <223> Oligonucleotide
<400> 394
   atacctgtgg ccccccacac aactgagccc atgctgatgg agtaccctga 50
<210> 395
   <211> 50
   <212> DNA
   <213> Artificial Sequence
<220>
   <223> Oligonucleotide
<400> 395
   ctggcgaacc ttggagaggg aatgctgatt gtcttgacca aacccacagc 50
<210> 396
   <211> 50
   <212> DNA
   <213> Artificial Sequence
<220>
   <223> Oligonucleotide
<400> 396
   ccctccttta tgacctttgg gacattggga atacccagcc aactctccac 50
<210> 397
   <211> 50
   <212> DNA
   <213> Artificial Sequence
<220>
   <223> Oligonucleotide
<400> 397
   ccatggacat tgctgctctt ggtggtgtta tctaattttt gtgataggga 50
<210> 398
   <211> 50
   <212> DNA
   <213> Artificial Sequence
<220>
   <223> Oligonucleotide
<400> 398
   cagaaattca gaaagggagc cagccaccct ggggcagtga agtgccactg 50
<210> 399
   <211> 50
   <212> DNA
   <213> Artificial Sequence
<220>
   <223> Oligonucleotide
<400> 399
   cccagggagt gctcgaggcg catcaggccc gttttttacc agtttatatc 50
<210> 400
   <211> 50
   <212> DNA
   <213> Artificial Sequence
<220>
   <223> Oligonucleotide
<400> 400
   ctcagttcct aatatcccgc tccttgctga gaccatctcc tggggcaggg 50
<210> 401
   <211> 50
   <212> DNA
   <213> Artificial Sequence
<220>
   <223> Oligonucleotide
<400> 401
   tgctcgaggc gcatcaggcc cgttttttac cagtttatat cacggtcttc 50
<210> 402
   <211> 50
   <212> DNA
   <213> Artificial Sequence
<220>
   <223> Oligonucleotide
<400> 402
   ataccggctt ccagagaccc cttttctcca gccatattac atcaggctag 50
<210> 403
   <211> 50
   <212> DNA
   <213> Artificial Sequence
<220>
   <223> Oligonucleotide
<400> 403
   aattctcagg gctctacccc cctttcctgg tcctaggtgg ccagtgggta 50
<210> 404
   <211> 50
   <212> DNA
   <213> Artificial Sequence
<220>
   <223> Oligonucleotide
<400> 404
   ccgcagctct catcattgtg atgtgtagca tgtctgccct ctgactggac 50
<210> 405
   <211> 50
   <212> DNA
   <213> Artificial Sequence
<220>
   <223> Oligonucleotide
<400> 405
   cactgtcgtc cttcctcaga gggcctcacg ccaaacaaac ggccttttcg 50
<210> 406
   <211> 50
   <212> DNA
   <213> Artificial Sequence
<220>
   <223> Oligonucleotide
<400> 406
   agcctggctc tgtgctgcgg gtgctctggt tggccgactg cgatgtgagt 50
<210> 407
   <211> 50
   <212> DNA
   <213> Artificial Sequence
<220>
   <223> Oligonucleotide
<400> 407
   ggtcctgtac gacatttact ggtctgagga gatggaggac cggctgcagg 50
<210> 408
   <211> 50
   <212> DNA
   <213> Artificial Sequence
<220>
   <223> Oligonucleotide
<400> 408
   gcaccgatgc acacaccgca ccccaccact gtactctgaa attggcgagt 50
<210> 409
   <211> 50
   <212> DNA
   <213> Artificial Sequence
<220>
   <223> Oligonucleotide
<400> 409
   gcattggggc caaacacaga atcagcaaag aggaggccat gcgctggttc 50
<210> 410
   <211> 50
   <212> DNA
   <213> Artificial Sequence
<220>
   <223> Oligonucleotide
<400> 410
   ttggggccaa acacagaatc agcaaagagg aggccatgcg ctggttccag 50
<210> 411
   <211> 50
   <212> DNA
   <213> Artificial Sequence
<220>
   <223> Oligonucleotide
<400> 411
   caggggattt ggggctttct tgaaagacag tccaagccct ggataatgct 50
<210> 412
   <211> 50
   <212> DNA
   <213> Artificial Sequence
<220>
   <223> Oligonucleotide
<400> 412
   ggttacccac tctgtccact cccataggct acagaaaaag tcacaagcgc 50
<210> 413
   <211> 50
   <212> DNA
   <213> Artificial Sequence
<220>
   <223> Oligonucleotide
<400> 413
   tcctttcggc cggaaccgcc atcttccagt aattcgccaa aatgacgaac 50
<210> 414
   <211> 50
   <212> DNA
   <213> Artificial Sequence
<220>
   <223> Oligonucleotide
<400> 414
   tccgggtgga taaggcagct gctgcagcag cggcactaca agccaaatca 50
<210> 415
   <211> 50
   <212> DNA
   <213> Artificial Sequence
<220>
   <223> Oligonucleotide
<400> 415
   aggcagaggt ccaagtaaac cgctagcttg ttgcaccgtg gaggccacag 50
<210> 416
   <211> 50
   <212> DNA
   <213> Artificial Sequence
<220>
   <223> Oligonucleotide
<400> 416
   ggagcgggct gctgagagct aaacccagca attttctatg attttttcag 50
<210> 417
   <211> 50
   <212> DNA
   <213> Artificial Sequence
<220>
   <223> Oligonucleotide
<400> 417
   gccaacctcc ctgtccagat gcagctattt tggtatctcc tatcacatgc 50
<210> 418
   <211> 50
   <212> DNA
   <213> Artificial Sequence
<220>
   <223> Oligonucleotide
<400> 418
   ctggacaaca agctccgtga agacctggag cgactgaaga agattcgggc 50
<210> 419
   <211> 50
   <212> DNA
   <213> Artificial Sequence
<220>
   <223> Oligonucleotide
<400> 419
   cctcgttgca ctgctgagag caagatgggt caccagcagc tgtactggag 50
<210> 420
   <211> 50
   <212> DNA
   <213> Artificial Sequence
<220>
   <223> Oligonucleotide
<400> 420
   agagcagccc aggtggtcat cagagtcacc aatgccaatg ccagcctgca 50
<210> 421
   <211> 50
   <212> DNA
   <213> Artificial Sequence
<220>
   <223> Oligonucleotide
<400> 421
   gcgaaaattc ggccagggtt ctcgctcttg tcgtgtctgt tcaaaccggc 50
<210> 422
   <211> 50
   <212> DNA
   <213> Artificial Sequence
<220>
   <223> Oligonucleotide
<400> 422
   tggactaaat gctcttcctt cagaggatta tccggggcat ctactcaatg 50
<210> 423
   <211> 50
   <212> DNA
   <213> Artificial Sequence
<220>
   <223> Oligonucleotide
<400> 423
   ctggggacga gacaggtgct aaagttgaac gagctgatgg atatgaacca 50
<210> 424
   <211> 50
   <212> DNA
   <213> Artificial Sequence
<220>
   <223> Oligonucleotide
<400> 424
   ccaacaggtc cgccaaatcc ggaagaagat gatggaaatc atgacccgag 50
<210> 425
   <211> 50
   <212> DNA
   <213> Artificial Sequence
<220>
   <223> Oligonucleotide
<400> 425
   gggacgagac aggtgctaaa gttgaacgag ctgatggata tgaaccacca 50
<210> 426
   <211> 50
   <212> DNA
   <213> Artificial Sequence
<220>
   <223> Oligonucleotide
<400> 426
   ggggcatgtt gtgtcatgta gtcagccact tatgcaccaa tgtgaggaaa 50
<210> 427
   <211> 50
   <212> DNA
   <213> Artificial Sequence
<220>
   <223> Oligonucleotide
<400> 427
   gtcctataaa tgcacctcct gtcaaaacca tgcctgagag gtcccggctg 50
<210> 428
   <211> 50
   <212> DNA
   <213> Artificial Sequence
<220>
   <223> Oligonucleotide
<400> 428
   cctggccgct gccttcattg agtttaaagg gacaggattg cccttccgtc 50
<210> 429
   <211> 50
   <212> DNA
   <213> Artificial Sequence
<220>
   <223> Oligonucleotide
<400> 429
   ccagcattga tctagaagca gaggaatccc agcgcctttt aaaagttgtt 50
<210> 430
   <211> 50
   <212> DNA
   <213> Artificial Sequence
<220>
   <223> Oligonucleotide
<400> 430
   cgtggctagg cctttcctgc cgagtgctct gatgcaatag tggaaatcgc 50
<210> 431
   <211> 50
   <212> DNA
   <213> Artificial Sequence
<220>
   <223> Oligonucleotide
<400> 431
   aaccatccca gagctggcga gaggatggag ctgggtggaa actgctttgc 50
<210> 432
   <211> 50
   <212> DNA
   <213> Artificial Sequence
<220>
   <223> Oligonucleotide
<400> 432
   gagcagttcg ctcctccctg ataagagttg tcccaaaggg tcgcttaagg 50
<210> 433
   <211> 50
   <212> DNA
   <213> Artificial Sequence
<220>
   <223> Oligonucleotide
<400> 433
   ggcagaaatg agcagttcgc tcctccctga taagagttgt ccaaagggtc 50
<210> 434
   <211> 50
   <212> DNA
   <213> Artificial Sequence
<220>
   <223> Oligonucleotide
<400> 434
   taacttccag gagttcctca ttctggtgat aaagatgggc gtggcagccc 50
<210> 435
   <211> 50
   <212> DNA
   <213> Artificial Sequence
<220>
   <223> Oligonucleotide
<400> 435
   gggcctccct tgaccccagt acgaagtcta tgccctgaat ccccagagta 50
<210> 436
   <211> 50
   <212> DNA
   <213> Artificial Sequence
<220>
   <223> Oligonucleotide
<400> 436
   cagagcccca gcccctcatg tcttgccgcc cttcctccat gtgtttgtaa 50
<210> 437
   <211> 50
   <212> DNA
   <213> Artificial Sequence
<220>
   <223> Oligonucleotide
<400> 437
   gccaaccgcc tcggggcaaa ctcgctcttg gacctggttg tctttggtcg 50
<210> 438
   <211> 50
   <212> DNA
   <213> Artificial Sequence
<220>
   <223> Oligonucleotide
<400> 438
   ctgtgccacc atcccgccag ccattcgctc ctactgatga gacaagatgt 50
<210> 439
   <211> 50
   <212> DNA
   <213> Artificial Sequence
<220>
   <223> Oligonucleotide
<400> 439
   gctgtctcca gtgcctgtta agctgaggat acaaccagga aatgcaacgg 50
<210> 440
   <211> 50
   <212> DNA
   <213> Artificial Sequence
<220>
   <223> Oligonucleotide
<400> 440
   gccacgttac atcaacacgg agcatggagg cagtcaggct cgattccttt 50
<210> 441
   <211> 50
   <212> DNA
   <213> Artificial Sequence
<220>
   <223> Oligonucleotide
<400> 441
   gcgctgcctt tcttcagcaa cagaccctca aaccaagagg aagctagatg 50
<210> 442
   <211> 50
   <212> DNA
   <213> Artificial Sequence
<220>
   <223> Oligonucleotide
<400> 442
   cccaggccaa taagctgggt gtctaaaagg acagcttctc ttccactcaa 50
<210> 443
   <211> 50
   <212> DNA
   <213> Artificial Sequence
<220>
   <223> Oligonucleotide
<400> 443
   gttcgtttca tcaggctctg ttcctcaatg gccttttgct acgtgcctcc 50
<210> 444
   <211> 50
   <212> DNA
   <213> Artificial Sequence
<220>
   <223> Oligonucleotide
<400> 444
   atggccatga cccagaagta tgaggagcat gtgcgggagc agcaggctca 50
<210> 445
   <211> 50
   <212> DNA
   <213> Artificial Sequence
<220>
   <223> Oligonucleotide
<400> 445
   gctgtagctc cttggggcaa aggtactaat ccctttcagc acccccactc 50
<210> 446
   <211> 50
   <212> DNA
   <213> Artificial Sequence
<220>
   <223> Oligonucleotide
<400> 446
   gcctgaggtg acagacaggg caggtggtaa caaaaccgtt gaacctccca 50
<210> 447
   <211> 50
   <212> DNA
   <213> Artificial Sequence
<220>
   <223> Oligonucleotide
<400> 447
   ctgctccgac agcagcccca ggaaatacgg gaatggttca gggaccaagt 50
<210> 448
   <211> 50
   <212> DNA
   <213> Artificial Sequence
<220>
   <223> Oligonucleotide
<400> 448
   tggcctttcc tacagggagc tcagtaacct ggacggctct aaggctggaa 50
<210> 449
   <211> 50
   <212> DNA
   <213> Artificial Sequence
<220>
   <223> Oligonucleotide
<400> 449
   aaggccttgg actcttccct gagggttgcc tgaaattcct tcatgctttc 50
<210> 450
   <211> 50
   <212> DNA
   <213> Artificial Sequence
<220>
   <223> Oligonucleotide
<400> 450
   atcaccacac tccccccagc cttcacctgg ccatgaagga ccttttgacc 50
<210> 451
   <211> 50
   <212> DNA
   <213> Artificial Sequence
<220>
   <223> Oligonucleotide
<400> 451
   ggtcattggc cacttcatct acagcagcct gttcccagtt ccctgaggcc 50
<210> 452
   <211> 50
   <212> DNA
   <213> Artificial Sequence
<220>
   <223> Oligonucleotide
<400> 452
   ccgtccactc ctcctactgt attttattgg acaggtcaga ctcgccgggg 50
<210> 453
   <211> 50
   <212> DNA
   <213> Artificial Sequence
<220>
   <223> Oligonucleotide
<400> 453
   atggagctcc tggaagcagc agagtccttt gacccaggaa gtgcttcagg 50
<210> 454
   <211> 50
   <212> DNA
   <213> Artificial Sequence
<220>
   <223> Oligonucleotide
<400> 454
   ccaccaaggc cctagactca tcttggccct cctcagctcc ctgcctgttt 50
<210> 455
   <211> 50
   <212> DNA
   <213> Artificial Sequence
<220>
   <223> Oligonucleotide
<400> 455
   gcgtgggctc gattcctcag ggccacgtta ccacagacct gtttgtttct 50
<210> 456
   <211> 50
   <212> DNA
   <213> Artificial Sequence
<220>
   <223> Oligonucleotide
<400> 456
   ccccatttca tggttttcag tggcaactta ctgacccttg tttttgcctg 50
<210> 457
   <211> 50
   <212> DNA
   <213> Artificial Sequence
<220>
   <223> Oligonucleotide
<400> 457
   ggtccccatg tgcctgttgt tcagccctct ctcttgttcc ctttctgagc 50
<210> 458
   <211> 50
   <212> DNA
   <213> Artificial Sequence
<220>
   <223> Oligonucleotide
<400> 458
   cacctccctg atgcctgctt tcagttgagg gttgggggca atgatgagca 50
<210> 459
   <211> 50
   <212> DNA
   <213> Artificial Sequence
<220>
   <223> Oligonucleotide
<400> 459
   ctgctattag agcccatcct ggagccccac ctctgaacca cctcctacca 50
<210> 460
   <211> 50
   <212> DNA
   <213> Artificial Sequence
<220>
   <223> Oligonucleotide
<400> 460
   tggagcccgc gttgctgttc ccacagggcc tcggtttttc ctaacttgct 50
<210> 461
   <211> 50
   <212> DNA
   <213> Artificial Sequence
<220>
   <223> Oligonucleotide
<400> 461
   gatacctgta atcccagcta cgtgggaggc tgaggtggga gaattgcttc 50
<210> 462
   <211> 50
   <212> DNA
   <213> Artificial Sequence
<220>
   <223> Oligonucleotide
<400> 462
   ggtgggtttg cctagggacg tgtaactaca ggcttttact aagccaagga 50
<210> 463
   <211> 50
   <212> DNA
   <213> Artificial Sequence
<220>
   <223> Oligonucleotide
<400> 463
   ggaggccagt gttgtgggct tcctgctggg actgagaagg ctcacgaagg 50
<210> 464
   <211> 50
   <212> DNA
   <213> Artificial Sequence
<220>
   <223> Oligonucleotide
<400> 464
   acccaagtct tctcccgtcc attccagtca aatctgggct cactcacccc 50
<210> 465
   <211> 50
   <212> DNA
   <213> Artificial Sequence
<220>
   <223> Oligonucleotide
<400> 465
   tatgtgcagc gacccttggt gtttcccttc ctcggtggct ctggggtatg 50
<210> 466
   <211> 50
   <212> DNA
   <213> Artificial Sequence
<220>
   <223> Oligonucleotide
<400> 466
   gcctttggtc agtaatgcgt tcaggagtcc acaccaggca cagatggggc 50
<210> 467
   <211> 50
   <212> DNA
   <213> Artificial Sequence
<220>
   <223> Oligonucleotide
<400> 467
   ggcagctgta gatcttgatc ttccaggtac cccatgtacc tttattgagc 50
<210> 468
   <211> 50
   <212> DNA
   <213> Artificial Sequence
<220>
   <223> Oligonucleotide
<400> 468
   tcttggcctc ccaactcttc ccactcccag aatccagaag taagctctgc 50
<210> 469
   <211> 50
   <212> DNA
   <213> Artificial Sequence
<220>
   <223> Oligonucleotide
<400> 469
   cgacagcagg acatacatgt tggtgtgaag actgggacga cactgggtag 50
<210> 470
   <211> 50
   <212> DNA
   <213> Artificial Sequence
<220>
   <223> Oligonucleotide
<400> 470
   tcaacctcac cagggctgtc tcttggtcca cacctcgctc cctgttagtg 50
<210> 471
   <211> 50
   <212> DNA
   <213> Artificial Sequence
<220>
   <223> Oligonucleotide
<400> 471
   ggagcagtac cttcccggag tccacgcatg tgagttgggt caagtgcatt 50
<210> 472
   <211> 50
   <212> DNA
   <213> Artificial Sequence
<220>
   <223> Oligonucleotide
<400> 472
   ctctgatttt tgcctctgga tagtagatct cgagcgttta tctcgggctt 50
<210> 473
   <211> 50
   <212> DNA
   <213> Artificial Sequence
<220>
   <223> Oligonucleotide
<400> 473
   ttgccagagt tttgcctgct gctttcctcg tggcctcttc ttgggtagtg 50
<210> 474
   <211> 50
   <212> DNA
   <213> Artificial Sequence
<220>
   <223> Oligonucleotide
<400> 474
   atggaggaga ccgaggaggt ggctatggag gagatcgagg tggctatgga 50
<210> 475
   <211> 50
   <212> DNA
   <213> Artificial Sequence
<220>
   <223> Oligonucleotide
<400> 475
   gtaacggagt ttagagccag ggctgatgct ttggtgtggc cagcactctg 50
<210> 476
   <211> 50
   <212> DNA
   <213> Artificial Sequence
<220>
   <223> Oligonucleotide
<400> 476
   aaagaagagc aacacgattc tgggatccca ggaggggaac accatgaaga 50
<210> 477
   <211> 50
   <212> DNA
   <213> Artificial Sequence
<220>
   <223> Oligonucleotide
<400> 477
   gtctgcagcc agcctgctcg aaactttagt cggcctgatg gcttagagga 50
<210> 478
   <211> 50
   <212> DNA
   <213> Artificial Sequence
<220>
   <223> Oligonucleotide
<400> 478
   gagcggatgc tttcatgcac cctttactgc actttctgac caggagctac 50
<210> 479
   <211> 50
   <212> DNA
   <213> Artificial Sequence
<220>
   <223> Oligonucleotide
<400> 479
   cactctctgc cagtggagcc agaaatgaca gcccaacaca gataccagtg 50
<210> 480
   <211> 50
   <212> DNA
   <213> Artificial Sequence
<220>
   <223> Oligonucleotide
<400> 480
   cgctcgtcac caagtcttta gaatagcttt agcgtcgtga accccgctgc 50
<210> 481
   <211> 50
   <212> DNA
   <213> Artificial Sequence
<220>
   <223> Oligonucleotide
<400> 481
   ctgtattaaa aaggcaaatc gaaggccggg cgcggtgact cacgcctgtc 50
<210> 482
   <211> 50
   <212> DNA
   <213> Artificial Sequence
<220>
   <223> Oligonucleotide
<400> 482
   cagccccctg cagctaagaa ttgtattgac tgtcctcaca gcggcttttc 50
<210> 483
   <211> 50
   <212> DNA
   <213> Artificial Sequence
<220>
   <223> Oligonucleotide
<400> 483
   agggctgcag ggcctcccac cttccaacag acaggctctg ctgtatctgt 50
<210> 484
   <211> 50
   <212> DNA
   <213> Artificial Sequence
<220>
   <223> Oligonucleotide
<400> 484
   gggctatgta ggcaggttaa tcctccactt ctcatgtggt tgaaccagtg 50
<210> 485
   <211> 50
   <212> DNA
   <213> Artificial Sequence
<220>
   <223> Oligonucleotide
<400> 485
   cttcagtgag aaactgccct tacaaacagt cccttctctg ctgtcaatcc 50
<210> 486
   <211> 50
   <212> DNA
   <213> Artificial Sequence
<220>
   <223> Oligonucleotide
<400> 486
   catcgcaggt accatcaaaa cggaaggcga gcatgaccct gtgacggagt 50
<210> 487
   <211> 50
   <212> DNA
   <213> Artificial Sequence
<220>
   <223> Oligonucleotide
<400> 487
   tcgctctggt cgcagcttag gaacagcaga cgtgcacttt gagcggaagg 50
<210> 488
   <211> 50
   <212> DNA
   <213> Artificial Sequence
<220>
   <223> Oligonucleotide
<400> 488
   gctgcaaccc ctcattatcc accacgcaca gatggtacag ctggggctga 50
<210> 489
   <211> 50
   <212> DNA
   <213> Artificial Sequence
<220>
   <223> Oligonucleotide
<400> 489
   gacaaaacgg gcgcgatgat gccctggctt tcagggtggt cagaactgga 50
<210> 490
   <211> 50
   <212> DNA
   <213> Artificial Sequence
<220>
   <223> Oligonucleotide
<400> 490
   gcagcgccaa gcggcatcca ccaagcatca agttggagaa aagggaaccc 50
<210> 491
   <211> 50
   <212> DNA
   <213> Artificial Sequence
<220>
   <223> Oligonucleotide
<400> 491
   cttctctccc catcgctcca caacctgaaa ccgagaagga gttgctgacc 50
<210> 492
   <211> 50
   <212> DNA
   <213> Artificial Sequence
<220>
   <223> Oligonucleotide
<400> 492
   ctgagaacct ttcccgttac tgcgttttca ccacctgtct tccccatgct 50
<210> 493
   <211> 50
   <212> DNA
   <213> Artificial Sequence
<220>
   <223> Oligonucleotide
<400> 493
   gctggagggc gctgtcattg tctatcagct gtactcccta atgtcctctg 50
<210> 494
   <211> 50
   <212> DNA
   <213> Artificial Sequence
<220>
   <223> Oligonucleotide
<400> 494
   atattccatc ctgcccaacc cttcctctcc catcctcaaa aaagggccat 50
<210> 495
   <211> 50
   <212> DNA
   <213> Artificial Sequence
<220>
   <223> Oligonucleotide
<400> 495
   tgactcaagg gctgtagatg ttccctttcc accccccaca cttggtgcgt 50
<210> 496
   <211> 50
   <212> DNA
   <213> Artificial Sequence
<220>
   <223> Oligonucleotide
<400> 496
   gtagtgtatc acagtagtag cctccaggtt tccttaaggg acaacatcct 50
<210> 497
   <211> 50
   <212> DNA
   <213> Artificial Sequence
<220>
   <223> Oligonucleotide
<400> 497
   ggcatgagtt agggagactg aagagtattg tagactgtac atgtgccttc 50
<210> 498
   <211> 50
   <212> DNA
   <213> Artificial Sequence
<220>
   <223> Oligonucleotide
<400> 498
   agtacctggg gaggttagat gtgtgtttca ggcttggagt gtatgagtgg 50
<210> 499
   <211> 50
   <212> DNA
   <213> Artificial Sequence
<220>
   <223> Oligonucleotide
<400> 499
   cagtgcttgg cacctggaag taggtggcag atgttaacgc ccttcctccc 50
<210> 500
   <211> 50
   <212> DNA
   <213> Artificial Sequence
<220>
   <223> Oligonucleotide
<400> 500
   ggtgaaccag aacagcacct cctcccactt aggaagctcg tgatttccag 50
<210> 501
   <211> 50
   <212> DNA
   <213> Artificial Sequence
<220>
   <223> Oligonucleotide
<400> 501
   ccttaatttg caggactgcc ttggtggctt tgtttgctgg gacaaggccc 50
<210> 502
   <211> 50
   <212> DNA
   <213> Artificial Sequence
<220>
   <223> Oligonucleotide
<400> 502
   tattagtgcg ctgtgaggtc tccacccgct ttgacatggg tagccttcgg 50
<210> 503
   <211> 50
   <212> DNA
   <213> Artificial Sequence
<220>
   <223> Oligonucleotide
<400> 503
   cagtaacgag gcttttgatg tgttgagctg gaggtgagtg gaccgggggc 50
<210> 504
   <211> 50
   <212> DNA
   <213> Artificial Sequence
<220>
   <223> Oligonucleotide
<400> 504
   tgatctccca gtcttgtcct tacccattcc aagtgctctg ccagcccctg 50
<210> 505
   <211> 50
   <212> DNA
   <213> Artificial Sequence
<220>
   <223> Oligonucleotide
<400> 505
   caccaatgaa tgagtcctta gccctgtgtc agtttaccct cgatgccctt 50
<210> 506
   <211> 50
   <212> DNA
   <213> Artificial Sequence
<220>
   <223> Oligonucleotide
<400> 506
   tgtcctggct tcccctccca aggaggatga ggatggtgcc tctgaggaaa 50
<210> 507
   <211> 50
   <212> DNA
   <213> Artificial Sequence
<220>
   <223> Oligonucleotide
<400> 507
   ccctggggat agctggggca tttgtctagc tgggctacct tctaacactt 50
<210> 508
   <211> 50
   <212> DNA
   <213> Artificial Sequence
<220>
   <223> Oligonucleotide
<400> 508
   tttgcccctc tcaccagccg tggaagccag cagtatcgag ctctcacagt 50
<210> 509
   <211> 50
   <212> DNA
   <213> Artificial Sequence
<220>
   <223> Oligonucleotide
<400> 509
   ctctggagtg gtgtatactg ccacatcagt gtttgagtca gtccccagag 50
<210> 510
   <211> 50
   <212> DNA
   <213> Artificial Sequence
<220>
   <223> Oligonucleotide
<400> 510
   gcatgcgtgt gcaggactgg ctgtgtgctt ggactcggct ccaggtggaa 50
<210> 511
   <211> 50
   <212> DNA
   <213> Artificial Sequence
<220>
   <223> Oligonucleotide
<400> 511
   agagggaagg caggggtgga ccgccatgag catgaaaaga cccgaagcaa 50
<210> 512
   <211> 50
   <212> DNA
   <213> Artificial Sequence
<220>
   <223> Oligonucleotide
<400> 512
   cctccagcat tcagtccagg gggagccacg gaaaccatgt tcttgcttaa 50
<210> 513
   <211> 50
   <212> DNA
   <213> Artificial Sequence
<220>
   <223> Oligonucleotide
<400> 513
   tcggtcctgc tggaggccac gggtgccaca cactcggtcc cgacatgatg 50
<210> 514
   <211> 50
   <212> DNA
   <213> Artificial Sequence
<220>
   <223> Oligonucleotide
<400> 514
   gcctgaatga tgagcctatg tccctgccta acactggtgt ctcactcatc 50
<210> 515
   <211> 50
   <212> DNA
   <213> Artificial Sequence
<220>
   <223> Oligonucleotide
<400> 515
   aggctccctt ctgagcctct cctgctgctg acctgatcac ctctggcttt 50
<210> 516
   <211> 50
   <212> DNA
   <213> Artificial Sequence
<220>
   <223> Oligonucleotide
<400> 516
   ttctcctagg gttatgtcca gttggggttt ttaaggcagc acagactgcc 50
<210> 517
   <211> 50
   <212> DNA
   <213> Artificial Sequence
<220>
   <223> Oligonucleotide
<400> 517
   cccagtgacg tggaagtcat cagaacccca cggtacttgg agtacctctc 50
<210> 518
   <211> 50
   <212> DNA
   <213> Artificial Sequence
<220>
   <223> Oligonucleotide
<400> 518
   ccttggttcc ctaaccctaa ttgatgagag gctcgctgct tgatggtgtg 50
<210> 519
   <211> 50
   <212> DNA
   <213> Artificial Sequence
<220>
   <223> Oligonucleotide
<400> 519
   ctgatatttt tccttggggg cgtaaccttc gctgaaattg ctgccctgcg 50
<210> 520
   <211> 50
   <212> DNA
   <213> Artificial Sequence
<220>
   <223> Oligonucleotide
<400> 520
   cacttgaccc caggagacgt aggttgtggt gagctgagat cgcgccattg 50
<210> 521
   <211> 50
   <212> DNA
   <213> Artificial Sequence
<220>
   <223> Oligonucleotide
<400> 521
   gttctgtgtg ctgtgacgac tgtcaaagag tatctggcca tggcggacac 50
<210> 522
   <211> 50
   <212> DNA
   <213> Artificial Sequence
<220>
   <223> Oligonucleotide
<400> 522
   tccagtctgt caccctcctt tcctgctccc atacacccaa ggcttgtttc 50
<210> 523
   <211> 50
   <212> DNA
   <213> Artificial Sequence
<220>
   <223> Oligonucleotide
<400> 523
   gctaacatcc attccctttc ataccaccat tttcaccctg tttcttcccc 50
<210> 524
   <211> 50
   <212> DNA
   <213> Artificial Sequence
<220>
   <223> Oligonucleotide
<400> 524
   gcgctaacat tcactcttgt ttgtccctgg actggccatg aagtgaggag 50
<210> 525
   <211> 50
   <212> DNA
   <213> Artificial Sequence
<220>
   <223> Oligonucleotide
<400> 525
   cataccggct ggccacggga agcgatgata actgcgcggc attctttgag 50
<210> 526
   <211> 50
   <212> DNA
   <213> Artificial Sequence
<220>
   <223> Oligonucleotide
<400> 526
   ggcaccgtgt ccaagttttt agaacccttg ttagccagac cgaggtgtcc 50
<210> 527
   <211> 50
   <212> DNA
   <213> Artificial Sequence
<220>
   <223> Oligonucleotide
<400> 527
   ctcagcccga ctggatcgcc atctgctaca acaactgcct ggagatactc 50
<210> 528
   <211> 50
   <212> DNA
   <213> Artificial Sequence
<220>
   <223> Oligonucleotide
<400> 528
   ggaagatgcc ccgacttctt tggccagtga tggggaatca gtgagtgctc 50
<210> 529
   <211> 50
   <212> DNA
   <213> Artificial Sequence
<220>
   <223> Oligonucleotide
<400> 529
   aaaaaatgat ttctggccgg gcgtggtggc tcaagcctgt aatcccagca 50
<210> 530
   <211> 50
   <212> DNA
   <213> Artificial Sequence
<220>
   <223> Oligonucleotide
<400> 530
   tggaagccct caccaagcac ttccaggact gaccagaggc cgcgcgtcca 50
<210> 531
   <211> 50
   <212> DNA
   <213> Artificial Sequence
<220>
   <223> Oligonucleotide
<400> 531
   gggtggggta cttctccata aggcatctca gtcaaatccc catcactgtc 50
<210> 532
   <211> 50
   <212> DNA
   <213> Artificial Sequence
<220>
   <223> Oligonucleotide
<400> 532
   ctgcctaacg tttgcttctg tgatggttat attgcctagc aagcacaccc 50
<210> 533
   <211> 50
   <212> DNA
   <213> Artificial Sequence
<220>
   <223> Oligonucleotide
<400> 533
   ttgcccattc ggctgtggat agagaagcag gaagagcact ggacttggag 50
<210> 534
   <211> 50
   <212> DNA
   <213> Artificial Sequence
<220>
   <223> Oligonucleotide
<400> 534
   ctgaggcaaa caggcatggg aaaatggaag ggttgaggat ggaccggaga 50
<210> 535
   <211> 50
   <212> DNA
   <213> Artificial Sequence
<220>
   <223> Oligonucleotide
<400> 535
   ccagaatgtg gtggttctgg gcaacaaatg agattgtggc gacgtggaga 50
<210> 536
   <211> 50
   <212> DNA
   <213> Artificial Sequence
<220>
   <223> Oligonucleotide
<400> 536
   tgtgtcacag ccagagggac aaagtgtggg tgatcctgga gacgccagtt 50
<210> 537
   <211> 50
   <212> DNA
   <213> Artificial Sequence
<220>
   <223> Oligonucleotide
<400> 537
   gggatcaact gtacgccttt ggtatctgac cataaagtct tttgctccgc 50
<210> 538
   <211> 50
   <212> DNA
   <213> Artificial Sequence
<220>
   <223> Oligonucleotide
<400> 538
   agccaaggac agagacctgg aacagatgct ttcattatgg cctccagagg 50
<210> 539
   <211> 50
   <212> DNA
   <213> Artificial Sequence
<220>
   <223> Oligonucleotide
<400> 539
   ggtgctggct ctctgtcaca atgcctcaaa agacatggaa cccaggccta 50
<210> 540
   <211> 50
   <212> DNA
   <213> Artificial Sequence
<220>
   <223> Oligonucleotide
<400> 540
   ctcactatga ggaacaagag aactagggga gctgctctgg tggccgtgtg 50
<210> 541
   <211> 50
   <212> DNA
   <213> Artificial Sequence
<220>
   <223> Oligonucleotide
<400> 541
   agtctgtagc ctccccgatc caagttccta gacctcatgg ctgtcccctc 50
<210> 542
   <211> 50
   <212> DNA
   <213> Artificial Sequence
<220>
   <223> Oligonucleotide
<400> 542
   aggccttgtt gcttaagaca ccttcagtct ttgcaggagg gcatggaagc 50
<210> 543
   <211> 50
   <212> DNA
   <213> Artificial Sequence
<220>
   <223> Oligonucleotide
<400> 543
   ttccagcctg ccagtcatga atctcagaca gcctgccacc tattgccctg 50
<210> 544
   <211> 50
   <212> DNA
   <213> Artificial Sequence
<220>
   <223> Oligonucleotide
<400> 544
   gagctgaaag ctgcggcgcc actggtgcca gagtcagatg tcacagatgt 50
<210> 545
   <211> 50
   <212> DNA
   <213> Artificial Sequence
<220>
   <223> Oligonucleotide
<400> 545
   aggcgtggtg gcctgcttct gtaatcctag ctagttggga ggctggcaca 50

## Description

### FIELD OF THE INVENTION

The present invention provides a method for diagnosing, identifying or monitoring proliferative disorders due to e.g. cancer preferably breast cancer in a subject by measuring the change of gene expression in a sample e.g. a blood sample. The present invention also encompasses oligonucleotide probes and primers corresponding to genes differentially expressed compared to the expression pattern in a normal cell. The use of the method and the oligonucleotides is also an aspect of the invention together with a kit comprising said oligonucleotides.

### BACKGROUND OF THE INVENTION

Carcinomas or solid tumors are composed of neoplastic epithelial cells, which form the heart of the tumor, as well as a variety of mesenchymal cell types and extracellular matrix components that comprise the tumor stroma, often termed the tumor microenvironment (TME)¹. Historically, it was believed that leukocytic infiltrates, in and around developing neoplasms, were representative of the host's attempt to eradicate neoplastic cells. However, it is now clear that infiltrating immune cells regulate and exert complex, paradoxical roles that are both pro- and anti-tumor during each stage of cancer development².

Recent advances have revealed that tumor-host interactions extend well beyond the local TME (i.e. interactions between the neoplastic cells and the nearby stroma) and that cancer development largely depends on the ability of malignant cells to hijack and exploit the normal physiological processes of the host^{2,3}. Although there is evidence that pronounced abnormalities in a host's systemic immune system caused by aging, stress, immuno-suppressive medications, autoimmune disease or chronic inflammation are associated with increased cancer risk⁴, we lack a precise understanding of how a tumor influences circulating immune cells and *vice versa.*

Blood pervades the entire body and is in a constant state of renewal⁵. It is the vehicle by which immune cells circulate between central and peripheral lymphoid organs, and migrate to and from tumor sites. Such a crucial role for blood cells as enhancers of tumor physiology raises questions about how they convey their tumor-promoting effects and whether, if understood, it is reflected in the gene expression of circulating cells.

Advances in blood RNA processing and transcriptomics allow whole genome gene expression profiling of circulating blood cells⁶. The inventors work within the unique Norwegian Women and Cancer study (NOWAC)⁷ proposed to use transcriptional profiling of cells preferably blood cells to investigate the genesis of cancer preferably breast cancer (BC)⁷ when compared to population-based controls.

The successful treatment of cancer depends in part on early detection and diagnosis. It is however, well established knowledge that a diagnostic method will be dependent on the prevalence of the disease in the population from which the cases (persons diagnosed with the disease) originate.

Unlike gene expression profiles from breast cancer that has been reported previously^{49,50} which were determined using cases and control group based on what is called a "workout" which means that the tests are based on control groups with positive findings (abnormal mammograms) and the prevalence of breast cancer in the populations involved will be about 50 %, while the prevalence using samples from the control group of the present invention wherein population-based controls from the NOWAC cohort (healthy controls matched by time of follow-up and birth year within the cohort) were studied will be less than 0,5 ‰.

It is believed that the method according to the present invention will be a more reliable diagnostic method, as the gene expression profile has been built up as a result of genes differentially expressed in samples from cases compared to samples from healthy people from the population wherein the test is to be used. It is therefore an objection of the present invention to provide an *in vitro* method for diagnosing, identifying or monitoring breast cancer in a human being.

The differences in the control groups as described above between WO 2011/086174 A2 and Aarøe et al. and the present invention may explain the differences between the identified gene profiles.

It should also be noted that our list is based on a study run as an epidemiological study and not with the potentials for quality controls as in a clinical setting. This is an important aspect for improving the test.

The inventors have disclosed how the presence of breast cancer is reflected in the gene expression of the host's cells, preferably circulating blood cells, and further how to utilize this in diagnosing, identifying or monitoring breast cancer. It has further been shown that specific functional "groups" of genes are specifically expressed. The method of the present invention may be able to detect the expression pattern reflecting a breast cancer long before the onset of symptoms appear.

The primary benefit of breast cancer screening is early detection, which typically results in simpler treatment, a lower chance of recurrence and a greater chance of survival. For younger woman, sensitivity of mammography screening can be as low as 58% and false negatives results cause a delay in diagnosis for women who think they are safe of breast cancer. The cumulative risk of false positive reading after 10 mammographic screens ranges 21% to 49 % for all women. In population offered mammography screening, the risk of unnecessary surgeries was increased by 31% compared to women not using mammography.

More specific technologies complementing existing screening will clearly be beneficial and we believe that the present invention providing a robust new diagnostic method may be such a complement. The method may however, further be used separately for diagnosing, identifying or monitoring proliferative disorder without being part of a screening program.

### SUMMARY OF THE INVENTION

The present invention encompasses in a first aspect, an *in vitro* method for diagnosing, identifying or monitoring breast cancer in a human, which method comprises the following step:
a) measuring the level of gene expression of all of the genes set forth in Table 1 or all of the genes set forth in Table 2 in a blood sample from said human;
b) comparing the level of gene expression of said genes in the sample from said human with the level of gene expression of said genes in a standard gene expression pattern extracted from healthy subjects;
c) wherein change of gene expression of each and one said gene in said sample as compared to a standard gene expression pattern being indicative for breast cancer .

A second aspect of the present invention comprises an in vitro method for preparing a standard gene expression pattern reflecting breast cancer in a human, which method comprises the following step:
a) measuring the level of gene expression in a sample from said human,
b) measuring the level of gene expression in a control sample from a healthy human;
c) comparing level of gene expression of the sample from said human with the level of gene expression in a control sample from the healthy human (not suffering from breast cancer) to produce a characteristic standard gene expression pattern reference from genes reflecting breast cancer as set forth in Table 1 or 2.

A third aspect of the present invention comprises a set of oligonucleotide probes, wherein said set consists of the oligonucleotides of Table 1 or 2 or oligonucleotides derived from the sequences set forth in Table 1 or 2, or an oligonucleotide with a complementary sequence, for use in the method.

In a further aspect the invention comprises a kit for *in vitro* diagnosing, identifying or monitoring proliferative disorder consisting of:
a collection of oligonucleotide probes and primers capable of detecting the level of expression of all of the genes set forth in Table 1 or all of the genes set forth in Table 2.

The present invention also provides, in one aspect use of the method as described, or the set of oligonucleotide probes, or the kit for diagnosing, identifying or monitoring breast cancer in a human.

Preferred embodiments are set forth in the dependent claims and in the detailed description of the invention.

### DESCRIPTION OF THE FIGURES

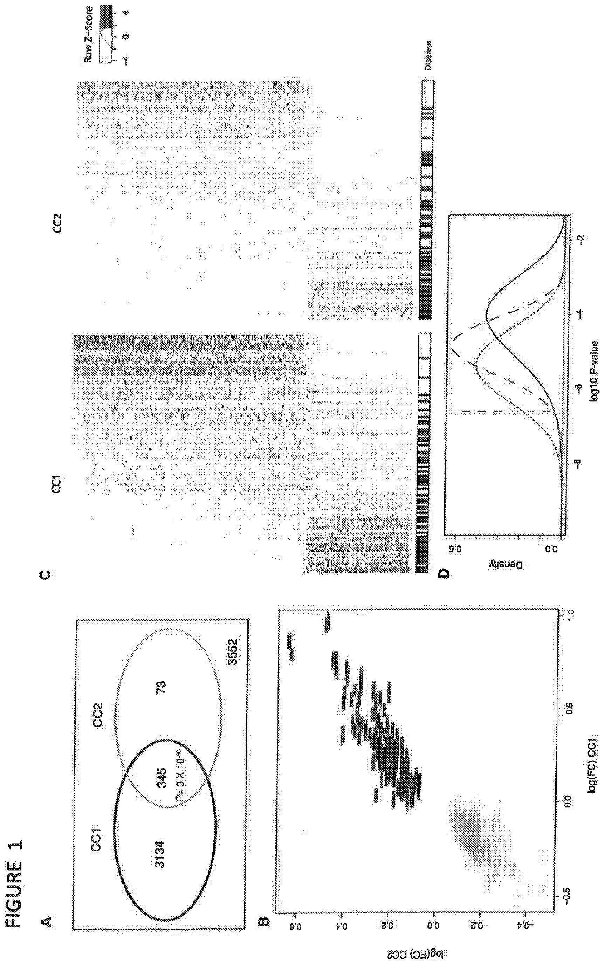

**Figure 1****.** Gene expression changes in circulating blood cells of breast cancer patients compared to controls.
   **A.** Venn diagram depicting the overlap between genes differentially expressed in circulating blood cells of breast cancer patients compared to controls in the primary (CC1) and the secondary (CC2) dataset. Differential expression was assessed at an FDR < 0.005 in the paired linear analyses. **B.** Expression fold changes for the 345 overlapping genes differentially expressed in CC1 and CC2. Log fold-changes (logFC) in CC1 are plotted on the x-axis against the log FCs for the same genes in CC2 on the y-axis. Light grey, genes downregulated in the breast cancer group in both data sets; dark grey genes upregulated in the breast cancer group in both data sets. **C.** Ordering of blood samples according to the sum of expression over those 345 overlapping genes. Heat map colors represent mean-centered fold change expression in log-space **D.** Accuracy significances of predictors in the validation dataset (CC3). Vertical line represents the accuracy significance of a predictor based on the expression of the 345 overlapping genes. The stippled line represents the distribution of accuracy significances that can be obtained from 100,000 predictors built using 345 random genes present in CC3 (N = 8529). The dotted line represents the distribution of accuracy significances that can be obtained from 100,000 predictors built using 50 genes among the 341 expressed in CC3 of the 345 overlapping genes. Plane line represents the distribution of accuracy significances that can be obtained from 100,000 predictors built using 50 random genes present in the dataset (N = 8529).
**Figure 2****.** Ordering of blood samples based on the enrichment scores of the 38 significant gene sets differentially expressed between breast cancer cases (in dark grey) and controls (in light grey) in the primary (CC1), secondary (CC2) and validation (CC3) case-control serie. Heat map colors represent mean-centered fold change enrichment score in log-space.
**Figure 3****.** Gene set variation analysis of the antigen processing and presentation pathway and the natural killer cell gene set.
   **A.** The antigen processing and presentation pathway from KEGG. Overlapping core genes driving the observed association of the Ag processing and presentation pathway with the presence of breast cancer within CC1 and CC2 are colored according to their up- (dark grey) or down- (light grey) regulation in breast cancer patients.
   **B.** Boxplot indicating the enrichment scores from gene set variation analysis for the natural killer (NK) cell gene set associated with the gene expression profiles from breast patients (dark grey) and controls (light grey) included in CC1, CC2 and CC3 (left). List of genes included in the NK gene set significantly associated to disease status in paired linear analysis with FDR<0.10 in at least two of the three datasets and their corresponding median fold-changes over all datasets (right).

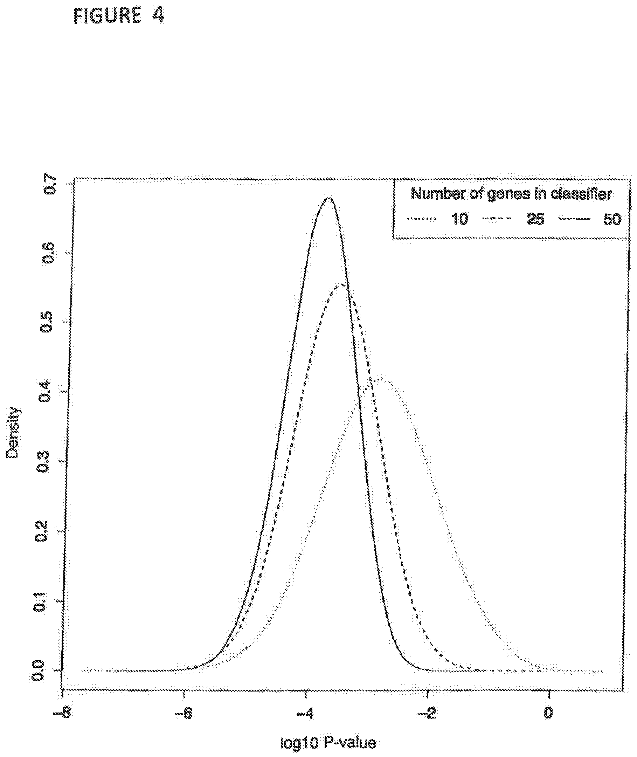

**Figure 4****.** Accuracy significances of predictors in the validation dataset (CC3) that can be obtained from 100,000 predictors built using 10, 25 or 50 genes among the 341 expressed in CC3 of the 345 overlapping genes.

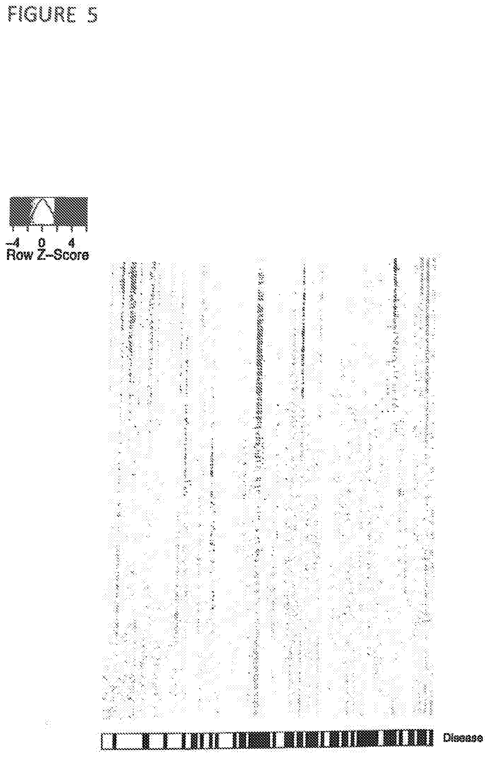

**Figure 5****.** Ordering of blood samples (dark grey: cases, light grey: controls) based on the top quintile of most variable genes in the primary case-control serie (CC1). Heat map colors represent mean-centered fold-change expression in log-space.

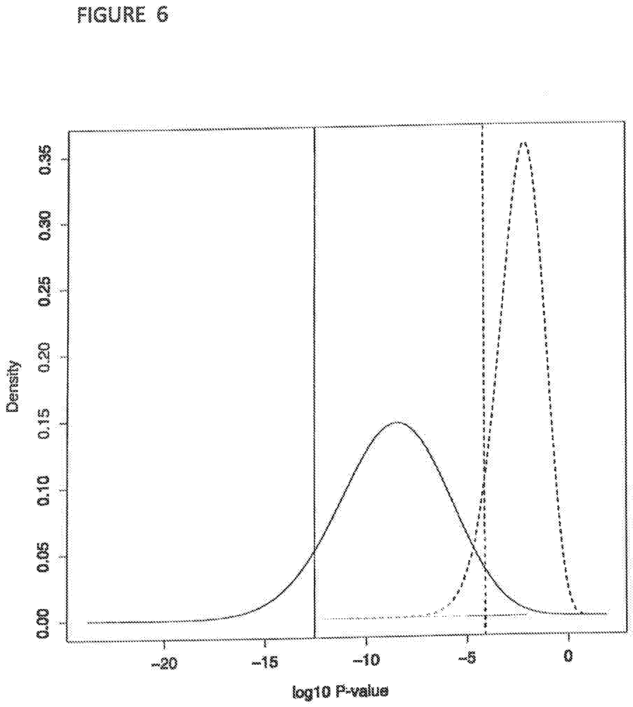

**Figure 6****.** Accuracy significances obtained to predict breast cancer diagnosis in the publicly available dataset including gene expression profiles of peripheral blood mononuclear cells (PBMC) from BC patients, pre- and post-surgery samples, patients from benign breast diseases, controls, gastrointestinal and brain cancer patients (GSE27562). Vertical lines represent the accuracy significances of a predictor based on the expression of the 49 genes present in the dataset among our 50-gene best predictor list to predict breast cancer case from controls (plain line) and to predict breast cancer or benign breast abnormalities cases from controls (dotted line). The distribution curves represent the respective distribution of accuracy significances that can be obtained from 100,000 predictors built using 49 random genes present in CC3 (N =8242).

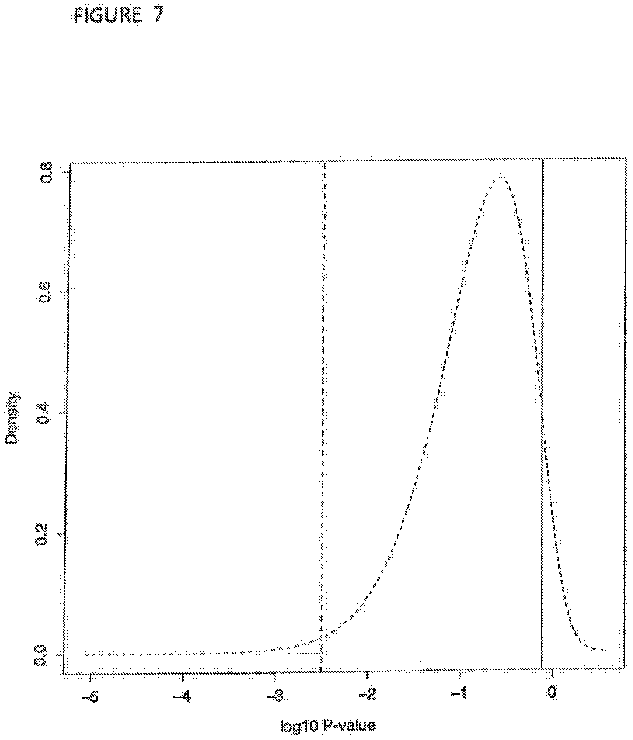

**Figure 7****.** Accuracy significances obtained to predict breast cancer diagnosis in the publicly available dataset including gene expression profiles of whole blood cells from BC patients and controls with suspect mammograms. Vertical lines represent the accuracy significances of a predictor based on the expression of the 32 genes present in the dataset among our 50-gene best predictor list (dotted line) and of the 63 genes present plain line). The dotted distribution curve represents the distribution of accuracy significances that can be obtained from 100,000 predictors built using 32 random genes present in CC3 (N =8242).

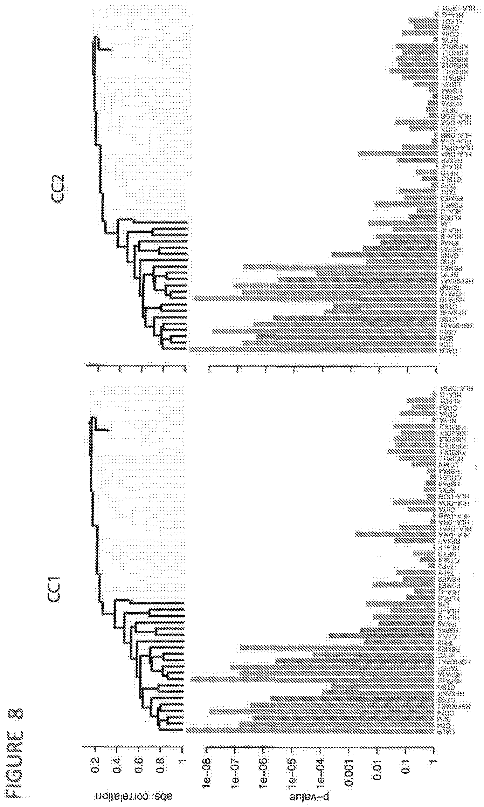

**Figure 8****.** Covariate plot displaying the individual contributions of each gene in the antigen processing and presentation pathway to the overall test statistics for differential expression in the primary (CC1) and secondary (CC2) case-control series. Significantly contributing genes (p<0.1) are indicated by the dark line in the hierarchical tree. The grey and the black colors represent association with the control and the breast cancer cases groups, respectively.

### DETAILED DESCRIPTION OF THE INVENTION

It is an object of the present invention to provide a method for diagnosing, identifying or monitoring breast cancer in a human by measuring the change of gene expression in a blood sample. The present invention also encompasses oligonucleotide probes and primers corresponding to genes differentially expressed compared to the expression pattern in a normal cell. The use of the method and oligonucleotides is also an aspect of the invention together with a kit comprising said oligonucleotides.

Norway has around 436,338 women born between 1943 and 1957, of whom 148,536 (34.4%) participate in the Norwegian Women and Cancer study (NOWAC)^{6,8,40}.
This unique material formed the basis of the study resulting in the present invention.

Blood samples were collected at time of diagnosis. The inventors first selected 96 blood samples from breast cancer patients and 116 blood samples from healthy controls matched by time of follow-up and birth year within the NOWAC cohort. From each sample, RNA was extracted from whole blood, and one control sample of two with the highest RNA quality and quantity was then amplified and hybridised along with its corresponding case sample. Using Illumina microarrays, whole blood gene-expression profiles for 96 breast cancer cases and 96 matched controls were generated. Probes fluorescence intensities of scanned images were quantified, trimmed, averaged and normalized to yield the transcript abundance of a gene as an intensity value.

In this primary dataset (CC1), some 9,338 unique genes were regulated across the group of 55 pairs of case/control. The inventors investigated blood gene expression profiles from an additional 63 pairs of BC cases and controls processed in the same way than CC1. To test whether these data findings where replicable in an independent data set a secondary dataset (CC2) was tested. After preprocessing of this secondary dataset (CC2), some 7898 unique genes and 49 pairs of case-control were included in the analyses. 818 of the 7898 genes passing the quality controls were differentially expressed in CC2, of which 345 were also differentially expressed in CC1. Remarkably, the directionality of differential expression between breast cancer cases and control of all 345 overlapping genes was conserved between the datasets. When patients were ranked according to the sum of expression over the 345 overlapping genes, the majority of blood samples from breast cancer cases were segregated from controls in both dataset.

These findings indicate that a basis of a reliable method for diagnosing, identifying and monitoring breast cancer, has been established. The overlapping 345 genes and alternative splice variants of some of said genes presented in Table 1 may serve as a source from where a subset of genes is chosen and the level of expression is measured and compared to a standard expression pattern. Also oligonucleotide probes and primers may be generated. A selection of preferred 50 genes is presented in Table 2. Each gene in Table 1 and 2 are denoted by international recognized gene symbols.

In order to validate the findings in CC1 and CC2 the validation dataset (CC3) consisted in 8529 unique genes expressed across 59 pairs of breast cancer cases and controls was investigated. Microarray data have been deposited at European Genome-phenome Archive (EGA; https://www.ebi.ac.uk/ega/).
In the 345-gene list, most differentially expressed transcription factors and genes that are part of the general transcription machinery (Table 4) are in blood cells from breast cancer patients compared to controls suggesting that down regulated steady state transcription rates are reduced.

Coordinate and sharp waves of expression of large sets of genes in response to a common stimulus imply that a broad regulatory mechanism is in place, possibly in response to the presence of breast cancer. Although transcription is an essential first step in the regulation of gene expression, the expression of key factors controlling immune cell differentiation and/or function are regulated at the translational level¹⁵. In our study, post-transcriptional regulons based on RNA binding proteins (RBPs) were identified as having essential roles in the control of blood cell gene expression changes associated with breast cancer (Table 4, Table 5). RBPs organize nascent RNA transcripts into groups in order to percolate them together down the chain of splicing, nuclear export, stability and translation so that proteins are efficiently produced to meet the needs of the cell¹⁶. The abundance of genes in the immune system that are alternatively spliced¹⁷, and the connections between splicing and disease in our study, imply that alternative splicing may be a crucial mechanism for regulating and fine-tuning the function of the circulating blood cells associated with the presence of breast cancer.

The inventors surprisingly detected that circulating blood cells in breast cancer patients are enriched in genes which are associated with, and may depend on, systemic immunosuppression, cell motility, metabolism and proliferation. These findings may further be determining when selecting the subset of genes and/or oligonucleotides or primers of the present invention.

In conclusion, the gene expression of circulating blood cells is markedly perturbed in a specific pattern by irregularities other places in the body as e.g. the presence of cancer, preferably breast cancer. These detectable changes form a reliable specific pattern not seen in blood cells form healthy persons and will serve as an excellent platform for diagnostic, identification or monitoring purposes. As a consequence the proliferative disorder may be detected long before the onset of other symptoms appears.

The blood-based gene expression method according to the present invention produced surprisingly uniquely robust and reproducible results across microarray platforms and external datasets to detect breast cancer from population-based controls.

Thus the invention comprises a method for diagnosing, identifying or monitoring breast cancer in a human by measuring a change in gene expression in cells from a blood sample

Accordingly a first aspect of the present invention relates to an *in vitro* method for diagnosing, identifying or monitoring breast cancer in a human , which method comprises the following step:
a) measuring the level of gene expression in all of the genes set forth in Table 1 or all of the genes set forth in Table 2 in a blood sample from said human;
b) comparing the level of gene expression of said genes in the sample from said human with the level of gene expression of said genes in a standard gene expression pattern extracted from healthy subjects; and
c) wherein change of gene expression of each and one said gene in said sample as compared to a standard gene expression pattern being indicative for breast cancer.

As used here in "subject" refers to a mammal. The term "subject" therefore includes for example primates (e.g. humans) and e.g. animals as cows, sheep goats, horses, dogs, cats.

As used here in "sample" refers to a biological sample (e.g. blood sample or fluid sample)
As used herein, "gene expression" refers to translation of information encoded in a gene into a gene product (e.g., RNA, protein). Expressed genes include genes that are transcribed into RNA (e.g., mRNA) that is subsequently translated into protein as well as genes that are transcribed into non-coding functional RNA that are not translated into protein (e.g., tRNA, rRNA ribozymes etc.)
As used herein "level of gene expression" or "expression level" refers to the level (e.g., amount) of one or more products (e.g. RNA, protein) encoded by a given gene in a sample or reference standard.

As used herein "healthy subject" refers to a subject from the NOWAC cohort representing population-based controls (healthy controls matched by time of follow-up and birth year within the cohort).

It is described that the expression from all 50 genes of Table 2 may be measured. It is described that the expression from all 345 genes of Table 1 may be measured.

In yet a further embodiment the change of gene expression is measured to be at least 10%, at least 20%, at least 30 % when measured as intensity value of scanned images. Other detection methods may be but are not limited to e.g. PCR.

It is described that the difference in gene expression may be measured by using oligonucleotide probes selected from a group set forth in Table 1 or 2, said oligonucleotide probes may be derived from a sequence set forth in Table 1 or 2, or may further be complementary to the sequence of the oligonucleotides set forth in Table 1 or 2, or be functional equivalent to the oligonucleotides of Table 1 or 2. The oligonucleotide may act as a detectable probe targeting the correspondent target sequence or may be used as a primer in the amplification step described in the Example section. The subset of genes, oligonucleotides probes or primers according to the present invention may be selected from Table 1 or 2 or any combination thereof.

It is further described that said oligonucleotide probes may be a set of probes of at least about 50, preferably at least about 30, more preferably at least about 20, most preferably at least about 10 and most preferably at least about 2 oligonucleotide probes.

As used herein "oligonucleotide" refers to a nucleic acid molecule and may be DNA, RNA or PNA (peptide nucleic acid) or hybrids thereof or modified forms thereof, e.g chemically modified by e.g. methylation. An oligonucleotide probe according to the invention has the ability to bind and probe complementary sequences in the target gene or gene product.

As used herein "derived" refers to oligonucleotides derived from the genes corresponding to the sequences set forth in Table 1 or 2. As the Tables provides Illumina identifiers and internationally recognized HUGO gene symbols, the probe or the primer sequences may be derived from anywhere on the gene to allow specific binding to that gene or its transcript.

As used herein "complementary sequences" refers to sequences with consecutive complementary bases, making it possible for the sequences to bind to one another through their complementarity.

As used herein "functional equivalent" refers to a oligonucleotide which is capable of identifying and binding to the transcript (or DNA) of the same gene as the oligonucleotides set forth in Table 1 or 2 or sequences derived from Table 1 or 2.

In a further embodiment the oligonucleotide may be a probe or a primer which hybridizes under stringent conditions, preferably under high stringency conditions. In further embodiments the oligonucleotide probe may be immobilized on one or more solid supports, which may be but are not limited to e.g. a membrane, a plate or a biochip.

As used herein "hybridize under high stringency condition" refers to a specific association of two complementary nucleotide sequences (e.g., DNA, RNA or a combination thereof) in a duplex under stringent conditions as a result of hydrogen bonding between complementary base pairs. Under high stringency conditions do not permit hybridization of two nucleic acid molecules that are not complementary (two nucleic acids molecules that have less than 70% sequence complementarities).

In yet another embodiment said oligonucleotides or primers may preferably be at least about 20, 50, 100 or 200 nucleotides in length.

It is described that the levels of gene expression in said subset of genes may be detected in said sample by determining the levels of RNA molecules encoded by said genes. The levels may but are not restricted to be determined by the use of microarray technique. Also other detection methods as e.g. PCR may be used.

As used herein "RNA" refers to total RNA in a cell.

In another embodiment the sample wherein the gene expression is measured is circulating blood cells e.g from whole blood, peripheral blood mononuclear cells or other subsets of blood cells. Also cells from body fluids or other tissues may be a sample source as the inventors surprisingly detected that the expression profile of differentially expressed genes found in blood cells also were seen in circulating mononuclear cells including monocytes, T-cells and natural killers cells. In one embodiments these cells may isolated and used as a sample source of the present invention.

In one embodiment the detection of differentially expressed genes may be obtained by translating the genes from Table 1 or 2 to PCR technology and identify the genes differentially expressed compared to control genes.

It is further described that the detection may be to identify every pair of genes in the subset of genes from Table 1 or 2 and identify the differentially expressed genes.

It is also described that the pair of genes in the subset of genes of Table 1 or 2 may be investigated and rules created as e.g. if gene 1 > gene 2 etc. then it's a positive case.

In order to decide if a sample from a subject reflects the specific expression pattern characteristic of a proliferative disorder as e.g. cancer, preferably breast cancer, a standard gene expression pattern may be provided.

One embodiment of the present invention comprises an *in vitro* method for preparing a standard gene expression pattern reflecting breast cancer in a human, which method comprises the following step:
a) measuring the level of gene expression in a sample from said human,
b) measuring level of gene expression in a control sample from a healthy human;
c) comparing level of gene expression of the sample from said human with the level of gene expression in a control sample from the healthy human (not suffering from breast cancer) to produce a characteristic standard gene expression pattern from genes reflecting breast cancer as set forth in Table 1 or 2.

According to a second aspect the present invention relates to a set of oligonucleotide probes, wherein said set consists of the oligonucleotides of Table 1 or 2 or oligonucleotides derived from the sequences set forth in Table 1 or 2 or an oligonucleotide with a complementary sequence.

It is described that the set of oligonucleotide probes may comprise at least about 50, preferably at least about 30, more preferably at least about 20, most preferably at least about 10 and most preferably at least about 2 oligonucleotide probes.

The oligonucleotide may act as a detectable probe targeting the correspondent target sequence or may be used as a primer in the amplification step described in the Example section. It is described that the oligonucleotide probes or primers according to the present invention may be selected from a group of Table 1 or 2 or any combination thereof.
In one or more embodiments the set of oligonucleotide probes consisting of the oligonucleotides of Table 1 or 2 may hybridize under high or medium stringency conditions with the corresponding genes of Table 1 or 2. Further the oligonucleotide probes may be immobilized on a solid support, which may be but are not limited to e.g. a membrane, a plate or a biochip.

The use of the oligonucleotides in a product e.g. a kit form further aspects of the present invention.

Accordingly a further aspect of the present invention relates to a kit for *in vitro* diagnosing, identifying or monitoring a proliferative disorderconsisting of :
the oligonucleotide probes and/or primers capable of detecting the level of expression of all of the genes set forth in Table 1 or of all of the genes set forth in Table 2.

The collection of oligonucleotides provided in the kit of the present invention consists of the oligonucleotides of Table 1 or 2 or an oligonucleotide with a complementary sequence.

In another embodiment the said probes may specifically hybridize to RNA transcripts of said genes, further the kit may comprise a set of oligonucleotide probes as defined above.

In further embodiments the oligonucleotide probes are preferably immobilized on one or more a solid supports wherein the solid support may be e.g. a membrane, a plate, or a biochip.

The present invention also provides, in one aspect use of the method as described, or the set of oligonucleotide probes, or the kit for diagnosing, identifying or monitoring breast cancer in a human.

Having now fully described the present invention in some detail by way of illustration and example for purpose of clarity of understanding, it will be obvious to one of ordinary skill in the art that same can be performed by modifying or changing the invention by with a wide and equivalent range of conditions and other parameters thereof, and that such modifications or changes are intended to be encompassed within the scope of the appended claims.

### EXAMPLES

### Example 1

### Blood-wide transcriptional signal of breast cancer and its diagnostic potential

NOWAC is a prospective cohort that follows a large representative sample of the Norwegian female population (∼34% of all women born between 1943-1957) and biobanks blood samples both prior to (n=50,000), and at the time of, breast cancer diagnosis (n=385 cases with age-matched controls)⁸. We selected from this cohort 96 blood samples from breast cancer patients and 116 blood samples from controls matched by time of follow-up and birth year. From each case sample, total RNA was extracted from whole blood, and a matched control sample was selected that exhibited the highest quality and quantity of RNA. Both case and control were amplified and hybridized simultaneously to ablate technical effects using Illumina microarrays. In total, we generated whole blood gene-expression profiles for 96 breast cancer cases and 96 matched controls. Samples received more than 4 days after collection (N=6), with low RNA quality (RIN<7, N=24), outliers (N=14), misdiagnosis (N=3) and unmatched samples (N=35) were excluded from our analyses. Probes intensities of scanned images were quantified, trimmed, normalized and averaged to yield the transcript abundance of each gene as an intensity value. In this first case-control dataset (CC1), these quality control steps identified 9,338 unique genes of sufficient quality across a group of 55 pairs of case/control (Table 3). Disease status was associated with substantial differences in blood gene expression profiles across the case/control pairs included in CC1 (*P* = 6 × 10⁻⁸, global test. This blood-wide signal of breast cancer diagnosis is illustrated by the grouping of samples according to disease status in an unsupervised clustering based on the top quintile of most variable genes (Figure 5). We identified 3479 genes showing significant differences in expression (False Discovery Rate (FDR) < 0.005; paired linear analysis) between the breast cancer and controls groups with a relatively low median absolute value of fold-change equal to 1.13 and ranging from 1.03 to 2.03. This indicates that gene expression changes associated with breast cancer are of relatively low amplitude but ubiquitous in circulating blood cells.

### Data analysis

The data analysis in the examples was performed using R (http://cran.r-project.org), an open-source-interpreted computer language for statistical computation and graphics, and tools from the Bioconductor project (http://www.bioconductor.org), adapted to our needs. To identify single gene differentially expressed between cases and controls, we conducted paired gene-wise linear analysis with application of empirical Bayes methods implemented in the software package Limma for the R computing environment. False discovery rate⁴¹ (FDR) was calculated to adjust for multiple testing. We trained naive Bayes' classifiers to predict outcome using the ranked log-odds that the gene is differentially expressed in the paired linear analysis and used internal leave-one-out cross-validation. We investigated the distribution of accuracy significances that can be obtained from 100,000 predictors built using a defined number of genes among the selected gene list, and compared it with predictors using the same number of genes randomly chosen within the dataset. Functional annotations were curated from the GeneCards encyclopedia⁴² (www.genecards.org). We applied functional clustering via the Database for Annotation, Visualization, and Integrated Discovery (DAVID) (ref) (http://david.abcc.ncifcrf.gov/). Gene set variation analysis package for the R computing environment implements a non-parametric unsupervised method for assessing gene set enrichment in each sample. We used several compendia and ontologies including the Gene Ontology⁴³ (GO), the KEGG⁴⁴ and Biocarta (http://www.biocarta.com/) pathway databases, and curated gene signatures databases obtained from the literature, all available from the Molecular Signature Database, MSigDB⁴⁵. Gene set specific to immune cell subsets were generated using CD markers of no more than 3 immune cell subtypes²¹ and transcripts specifically overexpressed in each differentiated immune cell subtype²². The global test {Goeman, 2004 24 /id} was used to test the influence of groups of genes on pairs of breast cancer cases and controls. Genes or cluster of genes driving the observed association of the gene set with the presence of BREAST CANCER were defined as core genes (multiplicity corrected P-value < 0.1).

### Example 2

To test whether these findings were replicable in an independent data set (CC2), we investigated blood gene expression profiles from an additional 49 pairs of breast cancer cases and controls subjected to the same data processing as CC1 (Table 3). 418 of the 7898 genes passing quality controls were differentially expressed in CC2 with a FDR<0.005, of which 345 were also differentially expressed in CC1 (*P* = 3 × 10⁻⁶⁰, hypergeometric test; Figure 1A, Table 4). Remarkably, the directionality of differential expression between breast cancer cases and controls of all 345 overlapping genes was conserved between datasets (Figure 1B). When patients were ranked according to the sum of expression over the 345 overlapping genes, the majority of blood samples from breast cancer cases were segregated from controls in both datasets (Figure 1C).

### Example 3

Using both CC1 and CC2 to select genes differentially expressed in blood samples from breast cancer patients compared to controls, we next asked whether we could accurately classify a third independent dataset encompassing (CC3). Data were subjected to the same processing as CC1 and CC2 (Table 3) and included the expressions of 8529 unique genes across 59 new case-control pairs from NOWAC. Of note, amplified RNA from the blood samples in CC3 was hybridized using a different version of the Illumina array system that includes 12 samples per array with about 40% less probes per signal. That can explain, at least partly, the higher FDR associated with the 345-gene list (Table 4). We built a predictor including all 341 expressed genes in CC3 of the 345-gene list and accurately predicted disease status in this validation dataset (*P* = 8.7 × 10⁻⁵; fisher test; Figure 1D). We investigated the distribution of accuracy significances that can be obtained from 100,000 predictors built using 50 genes among the 341 expressed genes, and compared it with predictors using 50 random genes present in CC3 (N = 8529, Figure 1D). The "best" 50-gene predictor chosen among the 341 significant genes (Table 4) had an accuracy of 72.9% to predict the presence of breast cancer (sensitivity = 83.1% and specificity = 62.7%; P = 3.0 × 10⁻⁹; fisher test). In all three datasets, we investigated whether RNA quality quantified by the RIN value and the use of menopausal hormone therapies or other specific medications could explain the misclassification of controls (i.e. false positives) and cases (i.e. false negatives). In CC2 only, true and false positives had a significantly lower RNA quality defined by the RIN value compared to false and true negatives, respectively (P = 1 × 10⁻³ and 0.02; student test). This indicates that gene expression changes in blood cells of breast cancer patients compared to controls can be confounded by RNA degradation although all samples were selected with a good RNA quality (RIN>7). Also, a significant proportion of controls misclassified as cases in CC3 (36.4%) were currently using of either a selective serotonin reuptake inhibitors (ATC N06AB) or a selective beta-blocking agent (ATC C07AB). Both drugs were previously found to inhibit the expression of T-cell and adaptive immunity-related genes ⁹⁻¹¹ that may explain the lower specificity in CC3 of the best 50-gene predictor identified. Notably, perturbagen signatures from the connectivity map associated with histone deacetylase, Hsp90, tyrosine kinase and immune response inhibitors were significantly enriched in our 50-gene predictor (Table 9, Table 5). Overall, this indicates that our 50-gene predictor contains cytostatic signals including the specific suppression of tumor immunity and that medications influencing transcription involved in those processes can be confounder of the blood-based signal associated with the presence of breast cancer. Considering breast cancer samples included in all 3 datasets, we did not observe tumor receptor specific or stage specific differences between true and false positives.

### Example 4

To further validate the results, we investigated whether we were able to predict breast cancer diagnosis in two external datasets deposited in NCBI's Gene Expression Omnibus¹² including gene expression profiles of peripheral blood mononuclear cells (PBMC) from breast cancer patients, pre- and post-surgery samples, patients from benign breast diseases, controls, gastrointestinal and brain cancer patients¹³ (GSE27562) and gene expression profiles of whole blood cells from breast cancer patients and controls with suspect mammograms¹⁴ (GSE164430). In the PBMC dataset, our 50-gene predictor was able to accurately predict the presence of breast cancer compared to controls from PBMC gene expression profiles (91.5% accuracy, Figure 6). This indicates that our diagnostic profile for breast cancer identified from whole blood cells is clearly found in circulating mononuclear cells including monocytes, T-cells, B-cells, and natural killer (NK) cells. Of note, the naive bayes classifier based on the top 3 factors published in LaBreche et al. (N = 77 genes) achieved a significantly lower accuracy (79.3%; P = 0.02 McNemar χ² test) in their own dataset. All PBMC samples from other cancer types were predicted as normal, which indicates that our predictor is specific for breast cancer. Since our predictor was not trained to differentiate malignant breast cancer from benign breast diseases, we obtained significantly lower accuracy when we included those samples (63.4% accuracy; Figure 6). The expressions of only 33 genes of our 50-gene predictor were available in the second dataset (GSE164430) although we were able to significantly predict breast cancer diagnosis compared to women with suspect screening mammograms (P = 0.008; fisher test, Figure 7). We were not granted access of the Breast Cancer Test gene list derived from this study. In conclusion, our blood-based gene expression analysis produced uniquely robust and reproducible results across microarray platforms and external datasets to specifically detect breast cancer from population-based controls, warranting further analysis of the processes found deregulated by the presence of a breast cancer in circulating blood cells including PBMC.

### Example 5

### Pathway and gene set variation analyses

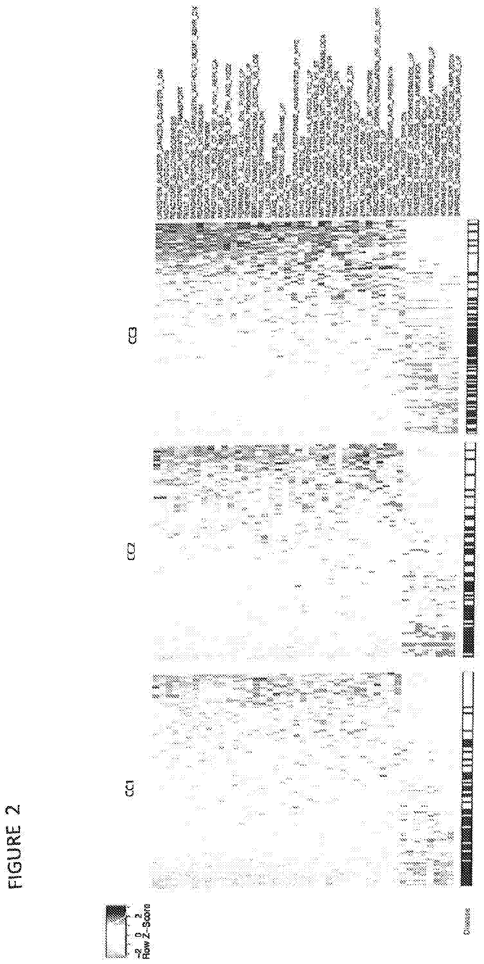

Pathway enrichment analysis showed that our 345-gene list differentially expressed in blood with the presence of breast cancer patients was enriched with a FDR < 0.10 for gene ontology categories related to apoptosis, RNA binding, spliceosome and RNA splicing, protein synthesis, RNA metabolism, transcriptional regulation, cell cycle, metabolism and signal transduction (Table 10). Expert functional annotation curated from GeneCards (Table 4) revealed additional grouping of genes involved in immune processes, cell growth/proliferation, cytoskeletal regulation, signal transduction, protein and cell metabolism. To further investigate how breast cancer affects gene expression in blood, we performed gene set variation analysis in CC1 and CC2 datasets and validate the results in CC3. The collection of gene sets used in the analysis consisted of release 3.0 of the C2 (curated gene sets) and C5 (Gene ontology gene sets) sub-collections of the Molecular Signatures Database (http://www.broad.mit.edu/gsea/msigdb/) of size ranging from 10 to 500 genes. We found 58 gene sets overlapping between the top 200 gene sets deregulated in CC1 and CC2 (FDR < 2 × 10⁻⁴; linear analysis). Although we previously identified a confounding factor with the current use of specific drugs by controls in CC3, forty-five of the 58 significant gene sets overlapping in CC1 and CC2 were validated in CC3 (FDR < 0.15, Table 6) and showed remarkably comparable enrichment scores according to disease status in all three datasets (Figure 2). Gene set variation analysis revealed similar processes seen after functional clustering of our 345-gene list including apoptosis, metabolism and transcription pathways, but also identified additional gene signatures notably involved in antigen processing and presentation and Myc target genes (Figure 2, Table 6).

### Example 6

### Circulating blood cells in breast cancer patients expressed changes in genes related to immune functions

### The antigen processing and presentation pathway and NK-cell mediated immunity

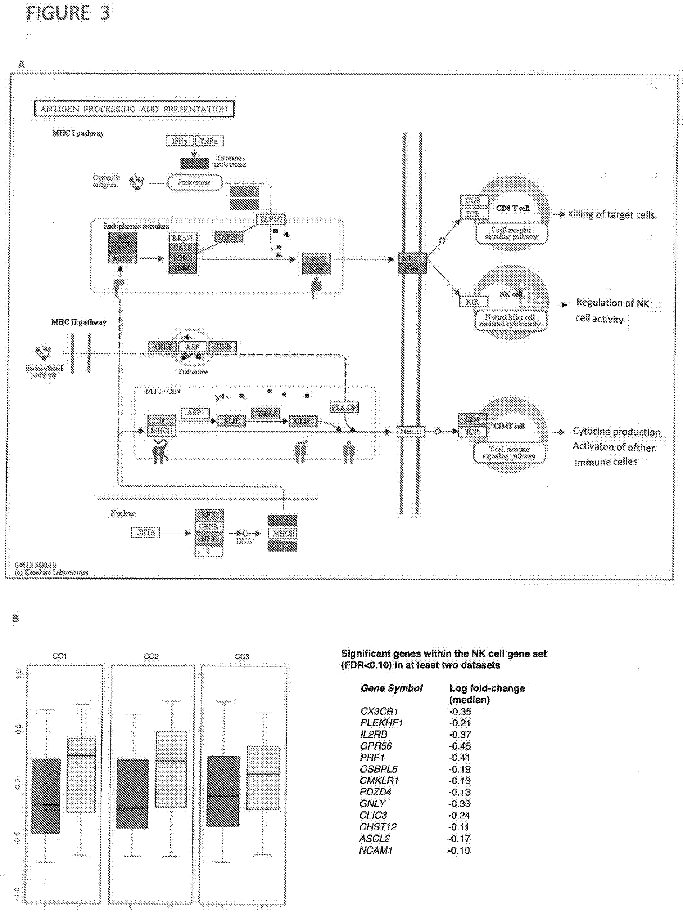

The antigen processing and presentation (APP) pathway downregulated in blood cells of BC patients (Figure 2, Table 6) was the most direct evidence that our signature is based specifically on the transcriptome of circulating immune effector cells. Mechanisms that regulate APP alter the form and the quantity of the epitopes that are presented by the major histocompatibility complex (MHC) molecules for immune recognition and can dictate tumor immunogenicity^{19,20}.
We investigated the overlapping core genes driving the observed association of the APP pathway with the presence of breast cancer within CC1 and CC2 (Figure 3A, Figure 8). All core genes that part of the MHC class II pathway were downregulated in blood samples from breast cancer patients compared to controls including the interferon gamma-inducible protein 30 (*IFI30*) and cathepsin S (*CTSS*) involved in the endocytic generation of MHC class II-restricted epitopes as well as *CD74* involved in the formation and transport of MHC class II protein and *CD4* a co-receptor that assists the T cell receptor (TCR) (Figure 3A). A recent study integrating measurements of copy number and gene expression in breast cancer tumor tissue identified trans-acting deletion hotspots localized to both TCR loci. Given that genomic copy number loss at the TCR loci drived a trans-acting immune response module, we asked whether cognate mRNAs modulated by both TCR loci in tumor-infiltrating lymphocytes (N= 114, Table 7) were differentially expressed in circulating blood cells of breast cancer patients included in CC1 and CC2. The gene set was significantly enriched when comparing the gene expression profiles of circulating blood cells from breast cancer patients compared to controls (P = 1.4 × 10⁻⁸ and 1.5 × 10⁻⁵ in CC1 and CC2, respectively; global test). Ranking the samples according to the sum of expression over 42 genes defined as core genes in at least one of the dataset, the majority of blood samples from breast cancer cases were segregated from controls in both datasets (Figure 8). This indicates that part of the signal from tumor-infiltrating lymphocytes with rearranged TCR loci was also found in circulating blood cells of breast cancer patients compared to controls.

Within the MHC class I pathway, *PSME3* of the immune-proteasome was defined as a core gene in the APP pathway downregulated in breast cancer patients (Figure 3A). In addition, three genes coding for the proteasome (*PSMB2, PSMB10, PSMD1*) within our 345-gene list (Table 5) were downregulated in blood cells of breast cancer patients compared to controls. These findings support that alterations in epitope processing occur in blood cells of breast cancer patients owing at least partly to changes in the proteasomal machinery. Also, core genes involved in peptide-loading onto MHC class I molecules (*TAPBP, CALR)* were downregulated in breast cancer patients (Figure 3A). Finally, several heat shock proteins within the APP pathway were also defined as core genes (Figure 3A) including the downregulation in breast cancer blood samples of *HSP70-1* and *-2* genes in breast cancer blood samples (HSPA1A and HSPA1B) that encode the major heat-inducible 70kDa heat shock protein (HSP70). In contrast, we observed upregulation in breast cancer blood samples of *HSPA5* with anti-inflammatory properties.
When investigating the expression of CD markers of no more than 3 immune cell subtype²¹ and transcripts specifically overexpressed in each differentiated immune cell subtypes²², genes specific to NK cells (Table 8) were consistently downregulated within the set and in samples from breast cancer patients compared to controls (Figure 3B). Consistent with this finding, the NK cell-mediated cytotoxicity pathway from KEGG was significantly downregulated in blood samples from breast cancer patients compared to controls (mean FDR = 0.08 across all three datasets; gene set variation analysis). Overall, this indicates that breast cancer evasion of anti-tumoe immunity is associated with the downregulation of the APP pathway and NK cell-mediated immunity not only in murine models or in the TME of good prognosis solid tumors²³ but also in the transcriptome of circulating blood cells. Remarkably, one epidemiological study has linked low peripheral blood NK cell cytotoxic activity with increased cancer risk²⁴.
Changes in expression of several cytoskeleton-regulating genes among our 345-gene list (Table 4) may imply further deregulation of the APP pathway as well as the disruption of the overall immune response against the damaged cells. For example, integrin-linked kinase (*ILK*), signaling modules of GTPase-mediated actin polymerization (*ABR, ABI3, ARHGAP1*), several actin-related genes (*ACTB, ACTG1, ARPC5L, PFN1*), several genes involved in microtubule-based motor proteins dynein and kinesin (*DYNLRB1, DYNC1H1, KIF13B*), and two engulfment and cell motility genes (*ELMO1*, *ELMO2*) were downregulated in blood cells of breast cancer patients (Table 6). A deregulated cellular cytoskeleton could disrupt the vacuolar transport of MHC-peptide complex in blood cells of breast cancer patients, as well as deregulate the cellular programs that lead to activation, proliferation, differentiation, secretion, cell-cell interaction and survival of immune cells, and phagocytosis²⁵. During the activation of a resting lymphocyte, large metabolic demands are also placed on the cell as it initiates proliferation and cytokine production^{26,27}. However, it has been shown that antigen stimulation signaling is required to control the ability of resting cells to take up and utilize nutrients at levels sufficient to maintain viability²⁸. Consistent with the defect in the APP pathway in blood cells from breast cancer patients, we observed a lowered cell metabolism with an overall downregulation of glycolysis and glucose metabolism (Table 10, Figure 2). In accordance with our results, altered plasma levels of enzymes involved in glucose, lipid, and amino acid metabolism were observed during tumor development in mice²⁹ indicating the presence of a tumor triggers systemic metabolic dysregulation. When extrinsic growth factors are limiting like it is suggested in blood cells of breast cancer patients, cells can activate pathways such as autophagy that promotes the degradation of intracellular constituents to provide a source of ATP. In accordance with this, two genes promoting autophagy (*ATG12, VPM1*) were upregulated in blood cells of breast cancer patients (Table 5).
If cells begin with lower energic levels and less biomass, they have to grow more to reach a size sufficient to enter a replicative division^{28,30}. Protein synthesis has been defined as a key determinant of cell growth and proliferation³¹ and is regulated by either the rates of translation initiation and elongation or ribosome biogenesis³²⁻³⁴. Consistent with this, three components of the eukaryotic initiation factor 4complex (*EIF4A1*, *EIF4A3, EIF4H*) were significantly downregulated in breast cancer patients compared to controls. Furthermore, several genes involved in ribosomal biogenesis (*CAR1, SURF6, RRS1*) were downregulated while several small *(RPS3A, RPS29*) and large (*RPL4, RPL5, RPL7, RPL11, RPL15, RPL21, RPL41*) ribosomal proteins (RPs) were upregulated in blood cells from breast cancer patients compared to controls. The accumulation of RPs can occur due to defects in ribosome assembly caused by an imbalance among RPs or caused by a defect in the assembly process³⁵. Recent findings have demonstrated that components of the translational apparatus are multifunctional and that several individual RPs play a role in regulating cell growth, transformation and death³⁵. Such an accumulation of any of several RPs can interface with the p53 system, leading to cell-cycle arrest or to apoptosis. For example, both *RPL5* and *RPL11* upregulated in blood cells from breast cancer patients have been found necessary for the accumulation of p53 and the consequent G1 arrest³⁶. Also, RPL11 can bind and sequester c-myc, itself a positive promoter of ribosome synthesis, and particularly of RPL11 synthesis^{36,37}.

Consistent with this, we found a decrease in gene set expression of the targets downregulated by Myc³⁸ (Figure 2) in blood cells from breast cancer patients. The downregulated Myc targets defined as core genes in all three datasets are involved in global gene regulatory networks with specific influence on cell growth and proliferation (*ILK, ARPC4, PP2R4, ERBB2, CEBPA*). Since some Myc target genes are regulators of cell growth while others function in cell differentiation and proliferation pathway, Myc is apparently poised at the interface of these processes in circulating blood cells from breast cancer patients compared to controls. The corresponding gene set including targets up regulated by Myc³⁸ was upregulated but not significant in blood samples of breast cancer patients compared to controls (mean FDR = 0.34 across all three datasets; gene set variation analysis). During immune cell proliferation, a fine regulation of the cell cycle is required to maintain immune cell homeostasis³⁹. In our 345-gene list, differentially expressed genes in the cell cycle regulation (*TERF2, CKAP5, CUL4B, MCM3, HBP1, NUDC, CTCF, TUBB, USP9X, H2AFX*) in circulating blood cells of breast cancer patients were mostly involved in the arrest at mitosis checkpoint. In GSVA, the gene set associated with the loss of Nlp from mitotic centrosomes required at the onset of mitosis was downregulated in the blood samples from breast cancer patients compared to controls (Figure 2, Table 6). Further, the integrin signaling pathway was downregulated in blood samples from breast cancer patients compared to controls. That indicates that integrin-mediated intracellular signals including cellular shape, mobility, and progression through the cell cycle were downregulated by the presence of breast cancer.
Together, these findings show that tumor development is associated with, and may depend on, systemic immunosuppression including the impairment of Ag processing/presentation pathway, and the metabolism, growth, motility, and proliferation of immune cells with a remarkable suppression of NK cell-mediated immunity.

The work leading up to the present invention has received funding from the European Research Council under the European Community's Seventh Framework Program (FPT/2007-2013)/ERC grant agreement no. 232997.

The human biological material applied in the study presented in present application has been approved by Regional Committees for Medical and Health Research Ethics in Norway; P REK NORD 27/2004 and P REK NORD 146/2006, and is in accordance with the "Biobankloven" (Lov av 21. February 2003 nr.12 om biobanker).

### References

1 Bissell, M. J. & Radisky, D. Putting tumours in context. Nature reviews. Cancer 1, 46-54, doi:10.1038/35094059 (2001).
2 de Visser, K. E., Eichten, A. & Coussens, L. M. Paradoxical roles of the immune system during cancer development. Nature reviews. Cancer 6, 24-37, doi:10.1038/nrc1782 (2006).
3 Hanahan, D. & Weinberg, R. A. Hallmarks of cancer: the next generation. Cell 144, 646-674, doi:10.1016/j.cell.2011.02.013 (2011).
4 Grivennikov, S. I., Greten, F. R. & Karin, M. Immunity, inflammation, and cancer. Cell 140, 883-899, doi:10.1016/j.cell.2010.01.025 (2010).
5 Ogawa, M. Differentiation and proliferation of hematopoietic stem cells. Blood 81, 2844-2853 (1993).
6 Dumeaux, V. et al. Gene expression analyses in breast cancer epidemiology: the Norwegian Women and Cancer postgenome cohort study. Breast cancer research : BCR 10, R13, doi:10.1186/bcr1859 (2008).
7 Lund, E. & Dumeaux, V. Systems epidemiology in cancer. Cancer epidemiology, biomarkers & prevention : a publication of the American Association for Cancer Research, cosponsored by the American Society of Preventive Oncology 17, 2954-2957, doi:10.1158/1055-9965.EPI-08-0519 (2008).
8 Lund, E. et al. Cohort profile: The Norwegian Women and Cancer Study-NOWAC--Kvinner og kreft. International journal of epidemiology 37, 36-41, doi:10.1093/ije/dym137 (2008).
9 Taler, M. et al. Immunomodulatory effect of selective serotonin reuptake inhibitors (SSRIs) on human T lymphocyte function and gene expression. European neuropsychopharmacology : the journal of the European College of Neuropsychopharmacology 17, 774-780, doi:10.1016/j.euroneuro.2007.03.010 (2007).
10 Sacre, S., Medghalchi, M., Gregory, B., Brennan, F. & Williams, R. Fluoxetine and citalopram exhibit potent antiinflammatory activity in human and murine models of rheumatoid arthritis and inhibit toll-like receptors. Arthritis and rheumatism 62, 683-693, doi:10.1002/art.27304 (2010).
11 Gasser, R. Myocardial ischemia and the immune system: some thoughts and changing views J Clin Basic Cardiol 14, 7 (2011).
12 Barrett, T. et al. NCBI GEO: archive for functional genomics data sets--10 years on. Nucleic acids research 39, D1005-1010, doi:10.1093/nar/gkq1184 (2011).
13 LaBreche, H. G., Nevins, J. R. & Huang, E. Integrating factor analysis and a transgenic mouse model to reveal a peripheral blood predictor of breast tumors. BMC medical genomics 4, 61, doi:10.1186/1755-8794-4-61 (2011).
14 Aaroe, J. et al. Gene expression profiling of peripheral blood cells for early detection of breast cancer. Breast cancer research : BCR 12, R7, doi:10.1186/bcr2472 (2010).
15 Rebhan, M., Chalifa-Caspi, V., Prilusky, J. & Lancet, D. GeneCards: integrating information about genes, proteins and diseases. Trends in genetics : TIG 13, 163 (1997).
16 Anderson, P. Post-transcriptional regulons coordinate the initiation and resolution of inflammation. Nature reviews. Immunology 10, 24-35, doi:10.1038/nri2685 (2010).
17 Keene, J. D. RNA regulons: coordination of post-transcriptional events. Nature reviews. Genetics 8, 533-543, doi:10.1038/nrg2111 (2007).
18 Lynch, K. W. Consequences of regulated pre-mRNA splicing in the immune system. Nature reviews. Immunology 4, 931-940, doi:10.1038/nri1497 (2004).
19 Garrido, C. et al. Alterations of HLA class I expression in human melanoma xenografts in immunodeficient mice occur frequently and are associated with higher tumorigenicity. Cancer immunology, immunotherapy : CII 59, 13-26, doi:10.1007/s00262-009-0716-5 (2010).
20 Neefjes, J., Jongsma, M. L., Paul, P. & Bakke, O. Towards a systems understanding of MHC class I and MHC class II antigen presentation. Nature reviews. Immunology 11, 823-836, doi:10.1038/nri3084 (2011).
21 Curtis, C. et al. The genomic and transcriptomic architecture of 2,000 breast tumours reveals novel subgroups. Nature 486, 346-352, doi:10.1038/nature10983 (2012).
22 Birnbaum, K. D. & Kussell, E. Measuring cell identity in noisy biological systems. Nucleic acids research 39, 9093-9107, doi:10.1093/nar/gkr591 (2011).
23 Watkins, N. A. et al. A HaemAtlas: characterizing gene expression in differentiated human blood cells. Blood 113, e1-9, doi:10.1182/blood-2008-06-162958 (2009).
24 Vivier, E., Ugolini, S., Blaise, D., Chabannon, C. & Brossay, L. Targeting natural killer cells and natural killer T cells in cancer. Nature reviews. Immunology 12, 239-252, doi:10.1038/nri3174 (2012).
25 Imai, K., Matsuyama, S., Miyake, S., Suga, K. & Nakachi, K. Natural cytotoxic activity of peripheral-blood lymphocytes and cancer incidence: an 11-year follow-up study of a general population. Lancet 356, 1795-1799, doi:10.1016/S0140-6736(00)03231-1 (2000).
26 Vicente-Manzanares, M. & Sanchez-Madrid, F. Role of the cytoskeleton during leukocyte responses. Nature reviews. Immunology 4, 110-122, doi:10.1038/nri1268 (2004).
27 Jones, R. G. & Thompson, C. B. Revving the engine: signal transduction fuels T cell activation. Immunity 27, 173-178, doi:10.1016/j.immuni.2007.07.008 (2007).
28 Fox, C. J., Hammerman, P. S. & Thompson, C. B. Fuel feeds function: energy metabolism and the T-cell response. Nature reviews. Immunology 5, 844-852, doi:10.1038/nri1710 (2005).
29 Rathmell, J. C., Vander Heiden, M. G., Harris, M. H., Frauwirth, K. A. & Thompson, C. B. In the absence of extrinsic signals, nutrient utilization by lymphocytes is insufficient to maintain either cell size or viability. Molecular cell 6, 683-692 (2000).
30 Pitteri, S. J. et al. Tumor microenvironment-derived proteins dominate the plasma proteome response during breast cancer induction and progression. Cancer research 71, 5090-5100, doi: 10.1158/0008-5472.CAN-11-0568 (2011).
31 Frauwirth, K. A. & Thompson, C. B. Activation and inhibition of lymphocytes by costimulation. The Journal of clinical investigation 109, 295-299, doi:10.1172/JCI14941 (2002).
32 Pardee, A. B. G1 events and regulation of cell proliferation. Science 246, 603-608 (1989).
33 Holland, E. C., Sonenberg, N., Pandolfi, P. P. & Thomas, G. Signaling control of mRNA translation in cancer pathogenesis. Oncogene 23, 3138-3144, doi:10.1038/sj.onc.1207590 (2004).
34 Thomas, G. An encore for ribosome biogenesis in the control of cell proliferation. Nature cell biology 2, E71-72, doi:10.1038/35010581 (2000).
35 Warner, J. R., Vilardell, J. & Sohn, J. H. Economics of ribosome biosynthesis. Cold Spring Harbor symposia on quantitative biology 66, 567-574 (2001).
36 Warner, J. R. & McIntosh, K. B. How common are extraribosomal functions of ribosomal proteins? Molecular cell 34, 3-11, doi:10.1016/j.molcel.2009.03.006 (2009).
37 Dai, M. S., Arnold, H., Sun, X. X., Sears, R. & Lu, H. Inhibition of c-Myc activity by ribosomal protein L11. The EMBO journal 26, 3332-3345, doi:10.1038/sj.emboj.7601776 (2007).
38 van Riggelen, J., Yetil, A. & Felsher, D. W. MYC as a regulator of ribosome biogenesis and protein synthesis. Nature reviews. Cancer 10, 301-309, doi:10.1038/nrc2819 (2010).
39 Zeller, K. I., Jegga, A. G., Aronow, B. J., O'Donnell, K. A. & Dang, C. V. An integrated database of genes responsive to the Myc oncogenic transcription factor: identification of direct genomic targets. Genome biology 4, R69, doi:10.1186/gb-2003-4-10-r69 (2003).
40 Baek, K. H. et al. p53 deficiency and defective mitotic checkpoint in proliferating T lymphocytes increase chromosomal instability through aberrant exit from mitotic arrest. Journal of leukocyte biology 73, 850-861 (2003).
41 Lund, E. et al. External validity in a population-based national prospective study--the Norwegian Women and Cancer Study (NOWAC). Cancer causes & control: CCC 14, 1001-1008 (2003).
42 Smyth, G. K. Linear models and empirical bayes methods for assessing differential expression in microarray experiments. Statistical applications in genetics and molecular biology 3, Article3, doi:10.2202/1544-6115.1027 (2004).
43 Benjamini, Y. & Hochberg, Y. Controlling the false discovery rate: a practical and powerful approach to multiple testing. Journal of the Royal Statistical Society Series B 57, 289-300 (1995).
44 Huang da, W., Sherman, B. T. & Lempicki, R. A. Systematic and integrative analysis of large gene lists using DAVID bioinformatics resources. Nature protocols 4, 44-57, doi:10.1038/nprot.2008.211 (2009).
45 Ashburner, M. et al. Gene ontology: tool for the unification of biology. The Gene Ontology Consortium. Nature genetics 25, 25-29, doi:10.1038/75556 (2000).
46 Ogata, H. et al. KEGG: Kyoto Encyclopedia of Genes and Genomes. Nucleic acids research 27, 29-34 (1999).
47 Subramanian, A. et al. Gene set enrichment analysis: a knowledge-based approach for interpreting genome-wide expression profiles. Proceedings of the National Academy of Sciences of the United States of America 102, 15545-15550, doi:10.1073/pnas.0506580102 (2005).
48 Goeman, J. J., van de Geer, S. A., de Kort, F. & van Houwelingen, H. C. A global test for groups of genes: testing association with a clinical outcome. Bioinformatics 20, 93-99 (2004).
49 WO 2011/086174 A2
50 Aarøe, J. et al. Gene expression profiling of peripheral blood cells for early detection of breast cancer. Breast Cancer Research 12, no. 1, R7 (2010)

**Table 1**

| **Illumina_Probe** | **50-base sequence** | **Gene_Symbol** | **SEQ ID NO** |
|---|---|---|---|
| ILMN_1766054 | GTGCTCTTTGTTCATCATTGGCCCTCATTCCAAGCACTTTACGCTGTCTG | ABCA1 | 1 |
| ILMN_1770031 | CCAGGCAGAGACTACCTTTGTGACCAGCTCCCAGTAAAAACCCCAGGCAC | ABHD10 | 2 |
| ILMN_1755658 | TGTCCTGTGACTGCCCCACAGAGATAAGGGGCCAGGAGGGATTGAAAGGC | ABI3 | 3 |
| ILMN_1672878 | AAACGCCAGGTCTGCCTGTTCTTGCTGGGCAATGGCTGATGGCTGCCAGT | ABR | 4 |
| ILMN_2152131 | TAAGGAGAATGGCCCAGTCCTCTCCCAAGTCCACACAGGGGAGGTGATAG | ACTB | 5 |
| ILMN_1777296 | CGGCTACAGCTTCACCACCACGGCCGAGCGGGAAATCGTGCGTGACATTA | ACTB | 6 |
| ILMN_2038777 | ACAGGAAGTCCCTTGCCATCCTAAAAGCCACCCCACTTCTCTCTAAGGAG | ACTB | 7 |
| ILMN_2053178 | GAGGCTGGCAAGAACCAGTTGTTTTGTCTTGCGGGTCTGTCAGGGTTGGA | ACTG1 | 8 |
| ILMN_1704961 | CGTTTCTCTGCCGGTCGCAATGGAAGAAGAGATCGCCGCGCTGGTCATTG | ACTG1 | 9 |
| ILMN_1654010 | AGCTCCTTTGTCGCTCTCATGGCTGTCAGATCCTGGTCCCTCCACACTGG | AGPAT3 | 10 |
| ILMN_2313821 | GTGGAATTGGCAAACCCACTGCAGCCCCTGAGAGGAGGTCGAATGGGTAA | AIFM1 | 11 |
| ILMN_1668408 | AGTTCCACAGGCTCCCGTCCAGGGGGAGGACTACGGCAAAGGTGTCATCT | AIFM1 | 12 |
| ILMN_1673252 | CAGTGTGACAGTGCCAGCCAATGTGCAGAGGTGGATGAGGTCTTGTGAAA | AIMP2 | 13 |
| ILMN_1709348 | GCAGGCAGGAACAGTGTGGGTGAATTGCTATGGCGTGGTAAGTGCCCAGT | ALDH1A1 | 14 |
| ILMN_2365686 | CAATTCTCCCAATGAGCCTTTTGTCTGTGGGAAAAGCAGGAGACGCTTCG | ALG8 | 15 |
| ILMN_1685413 | CTACCTGCTTGGCCTGGGGCCTCTGGAAGTCTGCTGTGAATTTGTATTCC | ALG8 | 16 |
| ILMN_1657283 | CTCTCAGGGGCCAGAACTCCTTTGCCAGCGTGGATTTCTCAAGTCGGGAC | ALKBH5 | 17 |
| ILMN_1723116 | GGTGTGAACCTACCTGCCTTGGAGAGGGCCCAGGTCCCAAATCTCTTCAA | AMFR | 18 |
| ILMN_1666258 | GGTTCCTGTAGCAGCAGCCGAAGGGAGACCTCGCTTAAACCAACACAATC | AMFR | 19 |
| ILMN_2184184 | CCTGGTGGCTCTTTGTGGAGGAAACTAAACATTCCCTTGATGGTCTCAAG | ANXA1 | 20 |
| ILMN_1755937 | CGTGCTTGGGGTTCAGCTGGTGAGGCTGTCCCTGTAGGAAGAAAGCTCTG | ANXA2 | 21 |
| ILMN_2409167 | TGTGGTGGAGATGACTGAAGCCCGACACGGCCTGAGCGTCCAGAAATGGT | ANXA2 | 22 |
| ILMN_1741003 | GTGCCTGCACCACCCCACTGCCTTCCTTCAGCACCTTTAGCTGCATTTGT | ANXA5 | 23 |
| ILMN_1740772 | GCCTTCTCTGCCTGTCCCTCCGATCCTTGTCCACCGTCTATTTATTGCCC | APBB3 | 24 |
| ILMN_2320513 | TTATACGGATGACTGGGAGGCACTGCACCACAACGTAGGACCCTGGCTCC | APBB3 | 25 |
| ILMN_1652505 | CCTAGCTCCTTGTTGGTGAGCTTCTTGTGCCTTAATCCTGTGACCCAGCC | APEX2 | 26 |
| ILMN_1675684 | ACCCCCACAGACCCCGTTCCTCCAGCCTGCGTGCCCCTAACCTGGCTTTT | APOBEC3C | 27 |
| ILMN_1802106 | GCAGAGCTGTGCTTCCTGGACGTGATTCCCTTTTGGAAGCTGGACCTGGA | APOBEC3G | 28 |
| ILMN_2232478 | CATCATGCTGGGGGAGATTCTCAGACACTCGATGGATCCACCCACATTCA | APOBEC3G | 29 |
| ILMN_1756862 | ATGATGGCGGTGGAGGTGGTGGTTGTAGTGTGATGGATCCCCTTTAGGTT | APOL3 | 30 |
| ILMN_2404065 | TCATTGGACTCATGGTGGGCGGTGTTGTCATAGCGACAGTGATCGTCATC | APP | 31 |
| ILMN_2404063 | GCAGAACTAGACCCCCGCCACAGCAGCCTCTGAAGTTGGACAGCAAAACC | APP | 32 |
| ILMN_1715068 | GAGTTACAAGCACCAGGGGATGCTCTACATCAAGGGATGCACCTTCAGTC | AQP9 | 33 |
| ILMN_1699703 | GCTGGTTGAAAAGTACCACTCCCACTCTGAACATCTGGCCGTCCCTGCAA | ARCN1 | 34 |
| ILMN_1682938 | AGGGACCAATCTGGGGCTGGAAATGTTAGGAGGTTGCCTTGGTGCTGCCC | ARF3 | 35 |
| ILMN_2403906 | TGGGACAGTGTGGTGGTACCAGGAAGAAAGAGGATTGGAAAGGCCAGTAC | ARFIP1 | 36 |
| ILMN_2403911 | CCTAACAAGAATTAAGCAGAGTTGGGGGAAGTGGGAGGGGTGACAAGCAT | ARFIP1 | 37 |
| ILMN_ 1732060 | TCTGCTCTCCGCCCTCCTTTGTGTATCAAGTGTCGCTCACAGCCCCATTC | ARHGAP1 | 38 |
| ILMN_1718610 | GTGACTGCTGGCTCTGTCACCTCATCAAACTGGATGTGACCCATGCCGCC | ARHGAP17 | 39 |
| LLMN_2398847 | GGAGCAGGGGCAGGCGAACCTCTTTCTTTGCAGACCGAACAGTGAAAAGC | ARHGAP17 | 40 |
| ILMN_1734742 | CAGGCCTTCCCTGACCCAGCCAGGAACAAACAAGGGACCAAGTGCACACA | ARHGDIA | 41 |
| ILMN_2043816 | ACTGCAGAACTGACATTTTGACGGTCTACCAGCGTGGCGGCTGGTGTTGG | ARPC5L | 42 |
| ILMN_2167426 | TTGCTCCGGGACGATGAGAGTATTTCCCATGCTGGAGTCGGCGAGAAGGG | ASPHD2 | 43 |
| ILMN_1669113 | CAGTGTTTCGTGAAGGTGTTGGAGAGGGGCTGTGTCTGGGTGAGGGATGG | ATF5 | 44 |
| ILMN_2188204 | GAGTCGTGATTGTACCACTGCATTCCTGCTGAGCAACAGAGTGAGACCCC | ATG12 | 45 |
| ILMN_1731783 | CGAAGTCAGAAAACTCATCATCAGGCGACGCCCTGGCGGCTGGGTGGAGA | ATP1A1 | 46 |
| ILMN_2415189 | GGAGTTTGGAACTCTACCCTGGTAGGAAAGCACCGCAGCATGTGGGGAAG | ATP1A1 | 47 |
| I LM N_1775566 | CCCTGGCTTATGAGCAGGCTGAGAGTGACATCATGAAGAGACAGCCCAGA | ATP1A1 | 48 |
| ILMN_1680579 | CCCAACCCTGCTGTTAGGCCTGCTGTTCCCTTTGCTCTTGATTAGGAGAG | ATP2B4 | 49 |
| ILMN_2367753 | GCCTCTTCCTCTGAATAGACCAGACGCCCTTTCACTTAGTTCAGTGCCAG | ATP2B4 | 50 |
| ILMN_1772132 | GCATTCATCGTGAGGGGTCTTTGTCCTCTGTACTGTCTCTCTCCTTGCCC | ATP5B | 51 |
| ILMN_1766185 | CAGCTTGTCTCTTGCCTGCCACTGTGTGAATCGGCGACGGAGCACTGCAC | AXIN1 | 52 |
| ILMN_1768534 | GGTCCAGAGTACTTGTTTTCCCGATGTGTCCAGCCAGCTCCGCAGCAGCT | BHLHE40 | 53 |
| ILMN_2363489 | CCTGCCTCTGTTTTTCCCTCGAGACCAGCCAACTCTCACATTTCAGTCCG | BRE | 54 |
| I LM N_1778684 | TGTCTTCACGGCAGCGTTTTGCTCACACAGCAGCTTTTGCACGCCCCAGG | BRE | 55 |
| ILMN_1728965 | GCCTTAGGCTTCGATGGTTCTTCCAACCCCCTTAATATGGCTTAGGGTGG | C11orf57 | 56 |
| ILMN_1798957 | GGCCTGAAGATGGGAGATTCCTAAGTGGAGGAGAACTGTGCCTTACTGAC | C12orf47 | 57 |
| ILMN_1652722 | GTACTGCTTTCAGTGTGTTCCCCCTCAGCCCCTCCGGCGTGTCAGGCATA | C14orf2 | 58 |
| ILMN_2150000 | CAGAGCTGGCATTTGCACAAACACGGCAACACTGGGTGGCATCCAAGTCT | C15orf24 | 59 |
| ILMN_1773407 | ACGTCATAGCTCCTTAGTTCTGCTCCTGTCGCCCTAACTTGGCATGGGCA | C16orf72 | 60 |
| ILMN_1812441 | CCCTCCTGCCCTCTGTCCATTCTTAGAGCATACCTTCATCCACTCCATGC | C17orf63 | 61 |
| ILMN_1719097 | CCAAACACCAAGTGTTAGCTGCCTTAAGGTTTATCCGGGGCATTGGTGGC | C18orf8 | 62 |
| ILMN_1721225 | ACCTGGTCAGCCTAAATCTTCCCAGTCCCGCTGTGGAGCTGTCAGTCACC | C20orf4 | 63 |
| ILMN_1682812 | AAAGAAGTGGAGCGTGTCCTGAAGGAGTTCCACCAGGCCGGGAAGCCCAT | C21orf33 | 64 |
| ILMN_1737588 | CAGCCACATTTCAGACCTGCTGAGACTGCTGAGTGAGGAATGGCAGTGAG | C21orf33 | 65 |
| ILMN_2386205 | GACCAGAAAAACAAGGTGGTCACGACCCCAGCCTTCATGTGCGAGACGGC | C21orf33 | 66 |
| ILMN_1757347 | GCCCCTTTAGTCCCCGCTCTGGAAGGCCAGGCAGTTTAGGTGTAAATAGG | KIAA0930 | 67 |
| ILMN_2224907 | ACCAAGATCGCACCACTGCATTCCAGCCTAAGCAATAGAGCGAGACTCCC | C4orf34 | 68 |
| ILMN_1713892 | GTGGATGGCAGTTTTCTGTAGTTTTGGGGACTGTGGTAGCTCTTGGATTG | C4orf34 | 69 |
| ILMN_1673478 | GGCAGCACTTATGCTCTGTGACAGTATTGTGTGTCATAGTTGAGCAGTAG | FAM13B | 70 |
| ILMN_1766200 | CTTCTTGGCCAGGGGAAAGGACCACAAGGCAATCTGGGGTGTGGACAGAC | CALHM2 | 71 |
| ILMN_1778242 | TGCTGTCTGTATCCCTTGGAGTAAGAAGGTAGTGGCATGGGTGGAGTGTG | CALM1 | 72 |
| ILMN_1714599 | GGTCTCAGCCAGCCCTAGAGACTGCTTCTTGTGTTTGTGTCATTCTGTCC | CAMLG | 73 |
| ILMN_1655418 | TCCCCTGTACCTGTGCCAAGCCTAGCACTTGTGATGCCTCCATGCCCCGA | CAPNS1 | 74 |
| ILMN_2393254 | AGGAGTGGCTGCAGCTGACTATGTATTCCTGAACTGGAGCCCCAGACCCG | CAPNS1 | 75 |
| ILMN_1768870 | ACTTGAGTGGAATCCTTTCCTCACGTACTCCCACAGACGTCTGGGCCTGG | CAPZA2 | 76 |
| ILMN_1678454 | ACTCCAAGGGCCAAAGCTCAAATGCCCACCATAGAACGACTGTCCATGAC | CASP4 | 77 |
| ILMN_1807540 | AAGGATCTGTGTTAGTCCCTGGGATGGCTCCAAGGCCTGCTCTAGGAAGG | CBARA1 | 78 |
| LLMN_1730347 | TGACTTGAGTCCACAAGGACACAAACACCTGAGTAGCTGGGCAGCCCTTG | CCDC115 | 79 |
| ILMN_1792681 | GGGAGCAGAGCTTTTCCCTAGCACCCACTTTCCCAAACCAGTCTCTGCAG | CCDC86 | 80 |
| ILMN_1731107 | AGGGCGGCACCGATCACCGAGCAGCCGTGCGTGTATCTCAAGGAACTAAA | CCDC92 | 81 |
| ILMN_1801600 | AGATGTCCTCAGACGGGAAGGTTTGAGAAGGGTCAGATGGTAGGCGGGCC | CCDC97 | 82 |
| ILMN_2341793 | AAGCTTTCGTGTGGGAGCCAGCTATGGTGCGGATCAATGCGCTGACAGCA | CCT7 | 83 |
| LLMN_1703718 | GCCCAGGGGGGTACATGGTATGGAGTAGACATCAACAACGAGGACATTGC | CCT7 | 84 |
| ILMN_1662954 | AAAGTGGAGCAGAGGAAGCTGCTGGAAAAGTGTGCCATGACCGCTCTGAG | CCT7 | 85 |
| ILMN_1661589 | AGAGTCCTGGGGAGCTTCTGTCCACCTGTCCTGCAGAGGAGTCGTTTCCA | CD151 | 86 |
| ILMN_2326713 | CACTGCTGTACCCAGATGCCTACAACCATCCCTGCCACATACAGGTGCTC | CD151 | 87 |
| ILMN_1794740 | GTTCCCAGCAGGGGGAGAAACCCTTCACACCCCAGGCCCTTCAGGAACTG | CD151 | 88 |
| ILMN_1736567 | CCCCGTTCCTGACATCACAGCAGCCTCCAACACAAGGCTCCAAGACCTAG | CD74 | 89 |
| ILMN_1761464 | TATGGGAGCATCGGCTACTGCTGGTGTGTCTTCCCCAACGGCACGGAGGT | CD74 | 90 |
| ILMN_2379644 | AAGGCACAGGGAGAAGGGATAACCCTACACCCAGACCCCAGGCTGGACAT | CD74 | 91 |
| ILMN_1676891 | CAGGACGAGCTACTGCTTTGGAGCGAGGGTTTCCTGCTTTTGAGTTGACC | CDK19 | 92 |
| ILMN_1718565 | AAGAGGCTGCGGTGAGCCAATTTAGAGCCCAAAGAGCCCCGAGGGAACCT | CDKN1C | 93 |
| ILMN_1751851 | CACTGTCTCAGAGAGGTTTTCCTGTGCTCGCCCTGTTTCTCTCAGGAAGC | CECR1 | 94 |
| ILMN_1748770 | ACTCTGCTTCTCTCTGTCAGCGTCCTGCTGCTCTAGAAGACTGTCCGTGG | CKAP5 | 95 |
| ILMN_1778203 | GTACAGGCTTTTAGCACCGAAGTGTGGTCCTCAGACCAGTGCCTGCCAAC | CLN5 | 96 |
| ILMN_1752802 | CACGTGTGAACATCTGTCTTGGTCACAGAGCTGGGTGCTGCCGGTCACCT | CLPTM1L | 97 |
| ILMN_2415179 | GACCCTGACTGCTAGTTCTGAGGACACTGGTGGCTGTGCTATGTGTGGCC | CLSTN1 | 98 |
| ILMN_2345837 | GCCCCGCTGGTGCACTGAAGAGCCACCCTGTGGAAACACTACATCTGCAA | CLTA | 99 |
| ILMN_1695420 | TGGGAACGGGTGGCCCGGCTGTGTGACTTTAACCCCAAGTCTAGCAAGCA | CLTA | 100 |
| ILMN_1726769 | GAGGAAAGAAATTAGGGCCTCCTCTGATCTCTCGCTATCTGCGGGTCCTG | CNDP2 | 101 |
| ILMN_2290007 | TGACGCGGATGGTGGGGAAGAACGTGAAGCTGTACGACATGGTGCTGCAG | COBRA1 | 102 |
| ILMN_2399328 | CCAGGGCAGAGCCTCTCCTTGTACTTTGGCAGCCATAGAAAGCGTGCTCA | COBRA1 | 103 |
| ILMN_1742432 | AAAGCGAGGCACACTGCTTACTGCCTTGGGGTTGTGGAGATGGACCCGTG | COBRA1 | 104 |
| ILMN_1730084 | ACGTGCTCCTGCTGACCTTCTGCGGCTCCGGGCTGTGTCCTAAATGCAAA | COMT | 105 |
| ILMN_1684385 | GGAAACCTGCTTCTCCTACTCCGGTTATTGTGGCCTCCCACACAGCCAAC | COPB2 | 106 |
| ILMN_1784785 | TGCCCCTCTGTCTGCTGAAGGACCTGTTGCTGCTTCTGTCTTTTCACCCC | COPS7B | 107 |
| ILMN_1703074 | CTCCCAGTTCTAGAGCAATCTACAGCTGTTTATGTGAGGTGCCCAACACC | CPD | 108 |
| ILMN_1755954 | CACAGAGGTGTTCTGACCTGCAACTTGACTGGGAAGCCCCTGGGTAACAC | CPEB3 | 109 |
| ILMN_2334243 | GCAGAATTGCTTGAACCCAGGAGGCAGAGGGTTGCAGTGAGCCGAGATAG | CREB1 | 110 |
| ILMN_2334242 | TGTTAGGTTATAATTTCTCATTTGGAGCCGGGCGCAGTGGCTCACGCCTG | CREB1 | 111 |
| ILMN_1690122 | GTCCCTACAGGAGGAACAGTGGCCTTGCTTCTTAGACGGTCTTCACTGTG | CRKL | 112 |
| ILMN_1706057 | AAGATACACTCCTGCTGTGCCCCCATCTTTCCTCCAAACTCCTGCCTGTG | CS | 113 |
| ILMN_2396410 | CCTCAGGCAGAGGATGTTCTGGACCTCCCCCTCTTGGTCCCTACTAGAGA | CS | 114 |
| ILMN_1754121 | CGTGTCTCCTCGGTCGCCCCGTGTTTGCGCTTGACCATGTTGCACTGTTT | CSK | 115 |
| ILMN_1653129 | CCTAACCCTTGAATGACTCAAATCAGTGCCAGGTGGAGGACTCCCATCAC | CSTF2 | 116 |
| ILMN_2278235 | GCTCGAGACAATTAAGGACGTGGGATGAGGCTCCGAGACAGGACGCGGTT | CTBP1 | 117 |
| ILMN_2379734 | AAACCGTCAAGCCCGAGGCGGATAGAGACCACGCCAGTGACCAGTTGTAG | CTBP1 | 118 |
| ILMN_1719158 | GAAGACGGCATCACGAAGCAGCTCCAAAAGGAAAAGCTTGGGCGGTGCCC | CTBP1 | 119 |
| ILMN_1786015 | ATGTAGCAGAATGGCACCCAGACCACTGCCCACCAGTGACGGACATGCAC | CTCF | 120 |
| ILMN_1757350 | CTGCAGGGGTCCTCTGTGAACTTGCTCAGGACAAGGAAGCTGCAGAAGCT | CTNNB1 | 121 |
| ILMN_1746396 | AGCTGCAGGGGTCCTCTGTGAACTTGCTCAGGACAAGGAAGCTGCAGAAG | CTNNB1 | 122 |
| ILMN_2063925 | CCTGGATGCGGGGCTCTTTGTTCTCCAGCACATCTGCTACATCATGGCCG | CTNNBL1 | 123 |
| ILMN_1666269 | GACACTTGCACAGCATGGCTCTGCCTCACAATGATGCAGTCAGCCACCTG | CTSZ | 124 |
| ILMN_2385161 | GTTAGGAGGAGACTCTTGATGTCACCTTCAGTATCTTGAAAGCGGGTCCC | CUL4B | 125 |
| ILMN_1651886 | TGTCTGTCATCCAGCTCCTATGTCTGTTATCCAGCTCCAAGTACAGCTTG | CWF19L1 | 126 |
| ILMN_2088437 | ATTGTCATTCCTGTATTCACCCGTCCAGACCTTGTTCACACTCTCACATG | CX3CR1 | 127 |
| ILMN_1745788 | CCATCTGGGAAAATACCCCATCATTCATGCTACTGCCAACCTGGGGAGCC | CX3CR1 | 128 |
| ILMN_2355462 | GGGCACGCGCTGCACTCCGTAACTCAACATGGCATGCCTTTCTCTCCGTA | CYFIP1 | 129 |
| ILMN_1682264 | TAGGTTTTGTGGCATCCACGGTCAGGTGTAGAGGAAGCTGCCCCTTGCAG | DCAF7 | 130 |
| ILMN_2209671 | GTCACCATCCACGAAGCTGCTGTCTCTGGCACATCTCCACAATTAACTCC | DCDC5 | 131 |
| ILMN_1809285 | GGAAAGCCAGCTCCCCTTCTCCTCTAACTATTGGAACGCCAGAAAGTCAG | DCP1A | 132 |
| ILMN_1669905 | CTTCCAAAGACCCACTAGAATGTCAGCTGTACTCTGTACTCTCCACTGAG | DCP2 | 133 |
| ILMN_1740737 | TACCTGCCCTCCTACTACCACCTGCATGTGCACTTCACCGCCCTGGGCTT | DCPS | 134 |
| ILMN_1802456 | GACAGCTTACTGCAGCACTGTTGGTGTTCGGAGCTCTTCTGTGCCCTGGC | DCTD | 135 |
| ILMN_1676984 | ACCAAGGGAGAACCAGGAAACGGAAACAGAGTGGTCATTCCCCAGCCCGG | DDIT3 | 136 |
| ILMN_2381841 | GTTCAGGGCACAGGCCCCGACATCACCCCAAGGACAACGGCACAAGTAGA | DDX19B | 137 |
| ILMN_2168952 | AGTCCAGGTTCAAGACTAGCCTGGGCAACATGGCAAGACCCTGTCCCTAT | DENR | 138 |
| ILMN_1760954 | GTCTGTTGCTGCTGAAAGATGTCTGTGTGCCTGTATCAACATGTGACTTC | DENR | 139 |
| ILMN_1762426 | AAGCAAAAGGATGGCTTGAGCCCAAGAGTTCAGAGCAGCCTGGCCACCAT | DHFRL1 | 140 |
| ILMN_1761450 | CTCCTGGCTTCAGTCCTTACAATGTCAGTAAAACAGCCTTGCTGGGCCTC | DHRS4L2 | 141 |
| ILMN_1744308 | CAGCCCCATTCCAGGGTCCCATCTGTCCGGAGTTGTGTATGTCAAGTCCG | DHX33 | 142 |
| ILMN_2248589 | AGCGCCTCCACCTGGCCTACTCTGTTATTTCCACCTGTTTGGGTAGGGCC | DHX40 | 143 |
| ILMN_2325008 | GCAGCAGAGGACCCAGGACCACAGTGACACACGAAAGGAAACAGGCTAAG | DHX40 | 144 |
| ILMN_1775304 | CATTTCTGTAAGGCAATCTTGGCACACGTGGGGCTTACCAGTGGCCCAGG | DNAJB1 | 145 |
| ILMN_1785177 | GCTACAAGTCTATCTTCTTTCTTGACCCATTTCAGGAGGGAGCCCTCTCC | DNAJC14 | 146 |
| ILMN_1770048 | GCCAGGGTTGGAGCCGACGACCAGAAAATTGCAGCAGGCACTTTAAGGCA | DPH5 | 147 |
| ILMN_2400183 | AATTCCCAGGTGTTGTGGTAGGTAATTGAATCATGGGGGCAGTTTCCCTC | DPH5 | 148 |
| ILMN_2105308 | CTAAAGGCTACGTCTTCCAGATGGAGATGATTGTTCGGGCAAGACAGTTG | DPM1 | 149 |
| ILMN_1658992 | AGCCGTGGGGCCAATTTTTTAACTCAGATCTTGCTGAGACCAGGAGCATC | DPM1 | 150 |
| ILMN_2112301 | GGACGAAGAAGATTACGACTCCTAGCGCCTTCTGCCCCCCAGACCATAGC | DRAP1 | 151 |
| ILMN_1663119 | AATATGACTATTCTAAAGGCTGTGAGGCCATGGGGTATTGGTTAAGTTGC | DSC2 | 152 |
| ILMN_1780302 | GCGCTGGGTCAAGCAGACAAACACCGAGAAGAAGGCCAGTGTGGTAACCT | DYNC1H1 | 153 |
| ILMN_1783448 | AGCCTTAGTCCAGGGGTGTGGCTCTGTCCGGGTGCAGTATGCAGTCATGT | DYNC1LI2 | 154 |
| ILMN_2157435 | CATGGCAGTCGCTTGGAACCCACTCACACCAATCCAGTGACCGTGTGTGG | DYNLRB1 | 155 |
| ILMN_1766762 | AGAAAGGCACAGGACTCGCTAAGTGTTCGCTACGCGGGGCTACCGGATCG | DYNLRB1 | 156 |
| ILMN_1658053 | ATGGCAGAGGTGGAGGAGACACTGAAGCGACTGCAGAGCCAGAAGGGAGT | DYNLRB1 | 157 |
| ILMN_1653115 | CATTCGGTGGCCGAGAGCCTCAACTACGTGGCGTCCTGGAACATGAGCAT | ECH1 | 158 |
| ILMN_1739257 | AGCAGGTCAGAGGCTCCTAACTGGGCAACTCAAGATTCTGGCTTCTACTG | EIF3E | 159 |
| ILMN_1667043 | CAGCAGATCAGTGGGATGAGGGAGACTGTTCACCTGCTGTGTACTCCTGT | EIF4A3 | 160 |
| ILMN_2370772 | AAGTGGCTCCGTGAAGCAGAGGAGGAGTCTGACCACAACTGAGGGCTGGT | EIF4G1 | 161 |
| ILMN_1768470 | CGGTGGCAGTGGGTGCCTGTAGTGTGATGTGTCTGAACTAATAAAGTGGC | EIF4G1 | 162 |
| ILMN_2304624 | GCACCCAGCGGAATGTGCTTAGTATTTGGTCACCAGCCGTCATCCTGGGC | EIF4H | 163 |
| ILMN_1776021 | TGCAGCTGTCTAGGTCTGCGGCCACATCTTGGGGACACACTGGACTGTTC | EIF4H | 164 |
| ILMN_1718044 | AAGAGCACGGTCCCCCAGGAGGCAGCTCAGGATAGGTGGTATGGAGCTGT | ELAC2 | 165 |
| ILMN_1784320 | AGCGTTTGGTGTTACCTTCTCCTGGGAGGTCCTGCTGCAACTCAAGTTCC | ELMO1 | 166 |
| ILMN_1778168 | AGTGATTTTGGTGGCCAGTAAATGCCAGCCATTTCTCAAACCCACCTCGG | ELMO2 | 167 |
| ILMN_2317730 | GCCTCTCTCCCTGGACATACGTTAGCACATTGGCATTCAGTATTGGTGGC | ELMO2 | 168 |
| ILMN_1765446 | GAAGGCGGTGCAGGTCCTCATGGTGCTCTCCCTCATTCTCTGCTGTCTCT | EMP3 | 169 |
| ILMN_1710756 | GAACTTCTACAGAAGCCAAGCTCCCTGGAGCCCTGTTGGCAGCTCTAGCT | ENO1 | 170 |
| ILMN_1744665 | GTGCAAGCGCCACGGACCTGGGACTCAGCACCGATAACTCAGACTTGAAT | EP300 | 171 |
| ILMN_1744963 | TCTTCTGTGTCCCTAAGGCCTGGTACAGTGCCAAGCACATACTTGGTATC | ERO1L | 172 |
| ILMN_2251184 | GTTCGACACCCAGTACCCCTACGGTGAGAAGCAGGATGAGTTCAAGCGTC | ERP29 | 173 |
| ILMN_2323048 | GCTGGTCTGGGGATAGCTGGAGCACTTACTCAGGTGGCTGGTGAAATGAC | ERP29 | 174 |
| ILMN_1733579 | GACCTTGCAATTTAGAATCAAGCAGGTGAGACAGGGAGAAGTATGCCTGC | EVI2A | 175 |
| ILMN_1697735 | CCTGGAAAAATGGATAAAGGCGAGCACCGTCAGGAGCGCAGAGATCGGCC | EWSR1 | 176 |
| ILMN_2413251 | CCTGGACCTTTGATGGAACAGATGGGAGGAAGAAGAGGAGGACGTGGAGG | EWSR1 | 177 |
| ILMN_1727041 | GGGAACCCCTTGTGAGCATGCTCAGTATCATTGTGGAGAACCAAGAGGGC | EWSR1 | 178 |
| ILMN_1651628 | GTGCGAGGGTAGATGGTTCCTGCACACAGAAGTTACCACAGGGGTCAGGT | EXOC6 | 179 |
| ILMN_1709747 | ACTCAAAGCTAAGGAGCAGTCAGGAACCCAGATAAGAAAGCCATCCTAGT | EXOG | 180 |
| ILMN_1670796 | GACAGAGGCTTCAGGTACAACTGGCCACAGAGATAGTCCTGGAAGACACG | EXOSC10 | 181 |
| ILMN_1711189 | GACACCGTGCTCCCGTCTCTCAGGCAGCGAAGTTCGATCCATCAACCAAA | EXOSC10 | 182 |
| ILMN_1758672 | GGGGCCCTGGTAGGCTCCTTTAGAAGGACCATTTCTGTTCCTAGAGCTTA | FAM107B | 183 |
| ILMN_2239772 | GTGCCTGGTGCCAGGTACACGGTCCTCTTCTCGCACGGCAATGCCGTGGA | FAM 108A1 | 184 |
| ILMN_1708143 | CCAGAAACCTCTTGTGTTCTTGCCTAGGCCCAGGTGTTCCTGGCAGCCAA | FAM127A | 185 |
| ILMN_1686254 | CATCCCCAGAGACCTCTTGTGTTCCTGCCACATAGCTGCCAGGGCTTAAG | FAM127B | 186 |
| ILMN_2153466 | GCTGCATTGCTCTGCTGAGCTGTATTGAAACCATGACTGGGCCCACTGTC | FAM50B | 187 |
| ILMN_2143250 | GGCTGACTCCCAGCCCTGACTTGAAACCATTAGCGCTAACTTGCTCTGTT | FAR1 | 188 |
| ILMN_1664614 | GAGGCCCTGACTACCCTGGAAGTAGCAGGCCGCATGCTTGGAGGTAAAGT | FAU | 189 |
| ILMN_1701455 | GACTCCTGCCCCGGTTCAACCCTACCAGCTTGTGGTAACTTACTGTCACA | FBXO6 | 190 |
| ILMN_1810289 | GCCATGTCACCGAGCCCCATTGATTCCCAGAGGGTCTTAGTCCTGGAAAG | MYOF | 191 |
| ILMN_1768743 | TCCCCATCTTCCTGGTTCTGCTCCTTCGTGGCCTAGTCTTGTGGACACCA | FIP1L1 | 192 |
| ILMN_1814998 | CCTGGGCATGGAATCCTGTGGCATCCACAAAACTACCTTCAACTCCATAG | POTEKP | 193 |
| ILMN_1745217 | GTTCTACAACTTGCACAGGGTAACAGAGGAAGTGGCTGAGGCCTAGAGTC | KIAA1310 | 194 |
| ILMN_1764619 | GCTGTGGTCAGTGGCTTAGCTCGGACAAGGAGATGAGAGCCCATGTGTTG | FU45244 | 195 |
| ILMN_1797342 | TGGAGGCATTTTGCTGTGTGAGGCCGATCGCCACTGTAAAGGTCCTAGAG | FNBP1 | 196 |
| ILMN_1745963 | GAATGCTGGTGAGATGCTTCATGGGACTGGGGGTCTCCTGCTCAGTCTGG | FOLR2 | 197 |
| ILMN_1747223 | CTTTTGGGTGTGGGGCAGGCAGAGAGGGATGGTGTCCAGAGATACATCAC | FRYL | 198 |
| ILMN_1686555 | CAGCGGAACCGCCCAGGATCAGATTGCATGTGACTCTGAAGCTGACGAAC | FYN | 199 |
| ILMN_2380801 | GCTGGAAAAAGGACCCTGAAGAACGCCCCACTTTTGAGTACTTGCAGAGC | FYN | 200 |
| ILMN_1781207 | GTTGTGTCTGGAGAAGAAGCTGGGTCAGGGGTGTTTCGCTGAAGTGTGGC | FYN | 201 |
| ILMN_2249920 | GCCGCCTAGTAGTTCCCTGTCACAAAGGGATGCCAAGGCTTACCGATCTG | FYN | 202 |
| ILMN_2412549 | GTTTTGTACAGTGCCTGGCACTCTGTGGGTGCTCAATAAATGGATAGGAG | GAR1 | 203 |
| ILMN_1771026 | CTCTGGGTCAATCGGAAGGCGCTATGCCAGGACTGATGAGATTGGCGTGG | GARS | 204 |
| ILMN_1779558 | GCCATGCTGAGAGCTGGGCTTTCCTCTGTGACCATCCCGGCCTGTAACAT | GAS6 | 205 |
| ILMN_1784749 | GCTGAGAGCTGGGCTTTCCTCTGTGACCATCCCGGCCTGTAACATATCTG | GAS6 | 206 |
| ILMN_1774077 | GCCTGTCCAGCTCCCTCTCCCCAAGAAACAACATGAATGAGCAACTTCAG | GBP2 | 207 |
| ILMN_1737308 | AGTTGGTCTTGGTGTCATATGGATCAGAGGCACAAGTGCAGAGGCTGTGG | GLRX | 208 |
| ILMN_1711617 | TCCTCAGGTGACTGGGGACTTGGAACCCTAGGACCTGAACAACCAAGACT | GMFG | 209 |
| ILMN_1769191 | GCGCATGCACCTTCGTCAGTACGAGCTGCTCTAAGAAGGGAACCCCCAAA | GNAS | 210 |
| ILMN_1784709 | GCTGCCTCCTCCTGGCTGTTTTTGTGCCTGTTTGAAGCTACTGCTGCCTC | GNPDA1 | 211 |
| ILMN_1748018 | GGTGCCACAGTTTTAAACCAGAAGGTGGCACTCTGTGGCTCCTTGTAGTA | GORASP2 | 212 |
| ILMN_1655625 | GCCCCAGGAGCTGCTGAGACGGCTGAAAAGTCTTCCACTAAGAAGGCAGT | GPATCH1 | 213 |
| ILMN_1727709 | CTAAAGGAAGGGCCTCTGCTGACTCCTACCAGAGCATCCGTCCAGCTCAG | GPBAR1 | 214 |
| ILMN_2316386 | CTGGATCAGAGACCCTGCCTCTGTTTGACCCCGCACTGACTGAATAAAGC | GPBAR1 | 215 |
| ILMN_2173451 | AGCAGCGCGAGGCCAGAGTCCAATAAACTCGTGCTCATCTGCAGCCTCCT | GPI | 216 |
| ILMN_2205245 | GGCCCTGCCACCAGAAAGTCGAGCACTGGTCCTAGTCAGGCTGTGATGAA | GPN2 | 217 |
| ILMN_2352097 | GCCAACATTCAGTCTGGTATGTGAGGCGTGCGTGAAGCAAGAACTCCTGG | GPR56 | 218 |
| ILMN_2384122 | GTAGATTGCTGGCCTGTTGTAGGTGGTAGGGACACAGATGACCGACCTGG | GPR56 | 219 |
| ILMN_1671142 | GGCCACACAGCCAAGAAGAGCCCATGCAACTCAGTCCAGGATTTTACTGG | GPR68 | 220 |
| ILMN_1679809 | TTCCTGGCCTCCCCTGAGTACGTGAACCTCCCCATCAATGGCAACGGGAA | GSTP1 | 221 |
| ILMN_1757467 | TCTAGGGCTAGTACTTAGTTTCACACCCGGGAGCTGGGAGAAAAAACCTG | H1F0 | 222 |
| ILMN_1695706 | GAGCACTCAACCCAGAAGGCGAAGATAGCTTTTGGTTGTAGGCGGCTTCC | H3F3B | 223 |
| ILMN_1685415 | AGGTGGCAGCAGCCATCCGTTATTATTTCCAATGGAGACCTAGCCCAGGC | HBP1 | 224 |
| ILMN_2160764 | TCTTTATGGTGGGGGCAGACTTTGCACTTACTGCAGTGCAACACTTGCAC | HBP1 | 225 |
| ILMN_2091123 | AATTAGCCGGGCGTGGTGGCAGGCTCCTCGGGAGGCTGAGGCAGAAAAAT | HCG2P7 | 226 |
| ILMN_1762835 | CCAGGAACTGAGCCTAGGGAGCTCATCTGGCAGCAATGGCTTTTACTCAT | HELZ | 227 |
| ILMN_1813314 | CAGCGAAGTCCGCTCCCGCGCCCAAGAAGGGCTCGAAGAAAGCCGTGACT | HIST1H2BK | 228 |
| ILMN_1796179 | CCCACTGGGGGGTTGGGGTAATATTCTGTGGTCCTCAGCCCTGTACCTTA | HIST1H2BK | 229 |
| ILMN_2382990 | CGTGTGTCCGTGGAACCAGTCCTAGCCGCGTGTGACAGTCTTGCATTCTG | HK1 | 230 |
| ILMN_1778401 | CTGATGTGTCTCTCACAGCTTGAAAAGCCTGAGACAGCTGTCTTGTGAGG | HLA-B | 231 |
| ILMN_1772218 | ACCGTCCTCATCATAAAGTCTCTGCGTTCTGGCCATGACCCCCGGGCCCA | HLA-DPA1 | 232 |
| ILMN_1657395 | CTTGGATGGACCCACGAACGCTCTTAGCTTTCTCAGGGGGTCAGCAGAGT | HMGCR | 233 |
| ILMN_1696485 | TACGACTACTCGCCCTATGGCTATTACGGCTACGGCCCCGGCTACGACTA | HNRNPAB | 234 |
| ILMN_2335718 | GCGGCAGCAGGAGCGACCAACTGATCGCACACATGCTTTGTTTGGATATG | HNRNPAB | 235 |
| ILMN_1651262 | CCAGAGCTCTAGGTGTTTAGGCAGCGTGTGGTGTCTGAGAGGCCATAGCG | HNRNPAB | 236 |
| ILMN_1751368 | GGTGACCAGCAGAGTGGTTATGGGAAGGTATCCAGGCGAGGTGGTCATCA | HNRNPD | 237 |
| ILMN_2321451 | CCCCCAGTATTGTAGAGCAAGTCTTGTGTTAAAAGCCCAGTGTGACAGTG | HNRNPD | 238 |
| ILMN_1745385 | GCCTGCCGGATGATGAATGGCATGAAGCTGAGTGGCCGAGAGATTGACGT | HNRNPM | 239 |
| ILMN_2175894 | ACAACCAACAGAACTGGGGTTCCCAACCCATCGCTCAGCAGCCGCTTCAG | HNRNPR | 240 |
| ILMN_2395728 | CAAGATAAGCATTTCTTTCCTGAGTTCAGGTGACTGAGGAAGAGCCACAA | HNRNPUL1 | 241 |
| ILMN_1690268 | TCCCACTGCCTCCTCTCCAGTGGTCTCCCAGGTGCCAGACCCAAAAGCTT | HNRNPUL1 | 242 |
| ILMN_1741406 | TCCAGAAACTGGGGATGGAATCTAGACTTGTGAGCGGCGGTGGTGCCTGC | HOOK1 | 243 |
| ILMN_1718537 | CCTGGAGGCTTGCTTGGGACTGGAGGCTTGCTTGGACAGTTCCTCTGTGT | HPS6 | 244 |
| ILMN_1880406 | CTGTGCAGCAGTGGCTCTGTGTGTAAATGCTATGCACTGAGGATACACAA | PTEN | 245 |
| ILMN_1841334 | AAGCCTTTGTATGTGTCCTCAGGGGGCAGACCGACTTTAAGAGGGACCAG | CREB1 | 246 |
| ILMN_1873620 | CTGAGGCATCCTGCTGTCATGGGAAGGTCTCCGCCCAAATGTCAGATGCA | TSPAN 14 | 247 |
| ILMN_1667030 | GACCATCCGAAACCTGCGTCCCTGGTGATGTTCTCAAGCCTCGGAAGTGG | HSBP1 | 248 |
| ILMN_1787843 | GGAGATTCACAGCAACTGATCAAAGGGAGTCCAGTCAACGTGAGCAAGCG | HSDL2 | 249 |
| ILMN_1673711 | AATGCTGCAGTTCCTGATGAGATCCCCCCTCTCGAGGGCGATGAGGATGC | HSP90AB1 | 250 |
| ILMN_1797031 | GTCCCAAGTATTTCCAGCAGGAAGTAATGTCTTCCTCAGCCTCAACCAGG | HSPBAP1 | 251 |
| ILMN_1751753 | GAACACCACGGACTTCCTCGACACCATCAAGAGCAACCTGGACAGAGCCC | IDH2 | 252 |
| ILMN_1721833 | GGGATTTAGCCAAGAGCACAGACTTGGATTCCTTCTGTCCCTCCCCACCT | IER5 | 253 |
| ILMN_1807277 | TGGAAGATCAGACCCAGCTCCTTACCCTTGTCTGCCAGTTGTACCAGGGC | IFI30 | 254 |
| ILMN_1717165 | GAACCGACAGAACATGGGCTGATCTTCCCACAACACCACAGGACTGCAGG | IGBP1 | 255 |
| ILMN_1699362 | GGGGCCCTTAGGCCGTTGGGACTTTGATACCCAGGAAGAATACAGCGAGT | IK | 256 |
| ILMN_2334296 | GCAGCTGCTTCGGATCCACACTGTATCTGTGTCATCCCCACATGGGTCCT | IL18BP | 257 |
| ILMN_1684349 | GGGGCTCCACACCTTTGCTGTGTGTTCTGGGGCAACCTACTAATCCTCTC | IL2RB | 258 |
| ILMN_2364376 | CAAGAGGGGCGGGCTCAGAGCTTTGTCACTTGCCACATGGTGTCTCCCAA | ILK | 259 |
| ILMN_1809141 | GGATTCATCCTGGGAGAGGGGGCAAGGTGGAATGCAGATAACTCACATGT | ING4 | 260 |
| ILMN_1803819 | CCCAAAGGCCACATCCAAGACAGGCAATAATGAGCAGAGTTTACAGCTCC | IQGAP1 | 261 |
| ILMN_1708375 | CCTCAACAGGCCCAGGGAGGGAAGTGTGAGCGCCTTGGTATGACTTAAAA | IRF1 | 262 |
| ILMN_2175912 | GGAGGGCTTGAGGTTGGTGAGGTTAGGTGCGTGTTTCCTGTGCAAGTCAG | ITGB2 | 263 |
| ILMN_1654396 | GGAGACTTGAGGAGGGCTTGAGGTTGGTGAGGTTAGGTGCGTGTTTCCTG | ITGB2 | 264 |
| ILMN_1793384 | ATTGCCTCTGACGTCTGGTCTTTTGGAGTCACTCTGCATGAGCTGCTGAC | JAK1 | 265 |
| ILMN_1777584 | AAAACTTCTGTACTGCCCTGGAATATGGGCTGCCCCCCACAGCTGGCTGG | KARS | 266 |
| ILMN_1652065 | CCTCTGTGCCTGAGTTCTCCCTGTTGTCTCAAAGCGGTACCCATCCTCCC | KCNMB1 | 267 |
| ILMN_2410771 | GCCCTGCCGGAAGCAGATTGACCAGCAGAACTGTACCTGTTGAGGCACTT | KEAP1 | 268 |
| ILMN_2410772 | CCTGGACAGTGTGGAGTGTTACGACCCAGATACAGACACCTGGAGCGAGG | KEAP1 | 269 |
| ILMN_1728676 | GCAGATGTTGTGGGTGCCCTTCTGTTCCTGGAGGATTATGTTCGGTACAC | KIAA0196 | 270 |
| ILMN_1752927 | CTGGGCCCTGTAAAATAGTGTTACTGTAATACTCTGTTTTGCCTCCTGCC | FAM160B1 | 271 |
| ILMN_2106227 | TCCTGCGTCTGTCTTCCCTGCTTTTCAGTCGTCGGGCTTAGAGAAGCCTG | KIAA2026 | 272 |
| I LMN_ 1686562 | GTCACCCTGTGCTTCCTGCCCAAGATACTGACCCATTGAACCCCCAAAGC | KIF13B | 273 |
| ILMN_1679929 | TTGCTTGTGTGCATGTGTTGGGTGCATGCTTCCGGGTCTCAGCTGCCCCA | KLF13 | 274 |
| ILMN_1782292 | CGGCACGTCCTTGGCGTCTCTAATGTCTGCAGCTCAAGGGCTGGCACTTT | LAMP1 | 275 |
| ILMN_1683792 | CCAACAAAGATGAAGTTCCCTATCTACGGAAAGGCATGACTGGGAGGCCC | LAP3 | 276 |
| ILMN_1681590 | TCTGGCACCCTCCTGAATGGAACCCCAGAGTACCTCCTGTGTGGAAGGGT | LARP1 | 277 |
| ILMN_1798212 | GTGTGTGCGGGCATGGATGTGACTGGGAGCTCTGCTGGGCACCCACATCT | LLGL1 | 278 |
| ILMN_1708101 | CCTCTTCCAAGCCCTGCGTCCAGCGAGCGTCACAGCACAACCTGCAAAAA | LMNB2 | 279 |
| ILMN_1685538 | CAAGCTGACCATTTCGAAGCTTGCTCCTGGTGGGCACGTGGGACGTTTCT | RPL4 | 280 |
| ILMN_1658950 | ACCCTCTGCGACCGGAGGACTATGCCCCTACTCCCGGAAGCCCTCGGACA | CRKL | 281 |
| ILMN_2051684 | GTGGTAGATCACTTGAGGTCAAGAGTTGTGACACCAGCCTGGCCAACCTG | C4orf3 | 282 |
| ILMN_1738356 | TGATGGTTCTTGCTGGGCAGCTTGGAGAAGGCGTGATACTCTCCAGCTCC | RPL15 | 283 |
| ILMN_1761281 | TAGAACTATTATTGACCACGCCTCCTCCAAGTCCCAGCGAGCCCGTGTAC | MT2A | 284 |
| ILMN_1679867 | GAATGAGCTCAGTGACCACTTGGATGCTATGGACTCCAACCTGGATAACC | HSF1 | 285 |
| ILMN_1770818 | CACTTAACATAGTGACCTCCAGTTCCATCCATGCTGTCGCAAGTGACAGG | FAM13AOS | 286 |
| ILMN_1676503 | AGATAAAGGGGCCACCAAGGAGTCGAGTGAGAAGGATCGCGGCCGGGACA | RNPS1 | 287 |
| ILMN_1679044 | TGCAGCCAGTCACTGCACCTCCGTCCTACCCTCCTACCAGCTATGATCAG | EWSR1 | 288 |
| ILMN_1679042 | CAGAGCTATTGCACCATGAGCGTCCTTCCTCCTTCCTCTCCGGGCTGCCA | ZNF598 | 289 |
| ILMN_1704750 | CCCTTTCCTCTCCCTCAGAATTTGTGTTTGCTGCCTCTATCTTGTTTTTT | TUBB | 290 |
| ILMN_1703692 | TCCTTTCCAACTCTACCTCCCTCACTCGAGCTCCTTTCCCCTGATCAGAG | TUBB | 291 |
| ILMN_1652230 | CCACGCGCGAAAATTCGGCCAGGGTTCTCGCTCTTGTCGCGTCTGCTCAA | RPS29 | 292 |
| ILMN_1667813 | AGTGGCGAGGATGGCAAGAAAAGCTGGCAACTTCTATGTACCTGCAGAAC | RPL7 | 293 |
| ILMN_1687738 | GTAGAAGGTGGAGATGCTGGCAACAGGGAGGACCAGGTCAACAGGCTTAT | RPL7 | 294 |
| ILMN_1814812 | AGCCTTGCATGTGCAGAAAGTAAAAGCCAGGGTAGGCTTGTAACCTGCCC | LOC100510589 | 295 |
| ILMN_1698940 | TTTGCTCGAAGCCTTCAGTCCGTTGCAGAGGAGCGAGTTGGACGCCACTG | RPL15 | 296 |
| ILMN_1716014 | AGGGTCCTGAATTCTTACTGGGTTGGTGAAGATTCCACATACAAATTTTT | RPL15 | 297 |
| ILMN_1780382 | TGCTTCCAACTGCGGGACAGGGAGTGGCCGTAGCGGCTTGTTGGATAAGT | LOC653566 | 298 |
| ILMN_1737586 | CGGAGACGGCAAATGGCGGACTTCGACACCTACGACGATCGGGCCTACAG | EIF4H | 299 |
| ILMN_1785756 | TGGCTTCACGGCTGGCTATGTGGACAGCAAGAGTCGTTTTCGCGGAAGCC | H2AFX | 300 |
| ILMN_1700025 | CCCTCCTGTGAGAGTCTGAAGGATACTATTGCCAGAGCTCTGCCTTCTGG | LOC100290936 | 301 |
| ILMN_1757336 | GTGCGACCTCGATCTGTCCCAAGCATTCATGTTCCCTCACCAGCTGTAGT | LRCH3 | 302 |
| ILMN_2103919 | GGACCCGGACACCCTGTGGGACCTGGCCTCAAACTCACCAAATCGCTCAT | LRFN3 | 303 |
| ILMN_1746148 | GTCATCAAGAGCCGCTGCCACTGGTCCTCCGTTTACTGACCTGGCTGTGT | LRRC33 | 304 |
| ILMN_2214997 | TGAGCAGGGACAGTCATTTTTTAAATGTTTTTGGCCGGGCGTGGTGGCTC | LRRFIP1 | 305 |
| ILMN_1724533 | TGGGAGCCCAGAAGAAATGCTCTTTTGCTTGGAGTTTGTCATCCTACACC | LY96 | 306 |
| ILMN_1775522 | CAGCCAGTGCCAACTTCGCTGCCAACTTTGGTGCCATTGGTTTCTTCTGG | MAGED1 | 307 |
| ILMN_2306189 | GTGTCTGGGCCCTGGAGCTGGGATGACATTGAGTTTGAGCTGCTGACCTG | MAGED1 | 308 |
| ILMN_1723020 | TGCTGTGTGACTATGATTCCTAAGATTTCCAGGGCTTAAGGGCTAACTTC | MAP3K1 | 309 |
| ILMN_2360229 | GGCCCTGGTTGAAAAGTACTCATCTCCTGGTCTGACATCCAAAGAGTCAC | MAPKAP1 | 310 |
| ILMN_1777721 | GCAGGATTCTGCAAAATGTGTCTCACCCACTACTGAGATTGTTCAGCCCC | MAPRE1 | 311 |
| ILMN_1717337 | CCTGTTTGGATCCTGGTCCTTTTTAACTGTTCCTTGGTAATTCTGAGCAT | MARCH7 | 312 |
| ILMN_1714433 | CCTGAGCCAGAAGTGGGGTGCTTATACTCCCAAACCTTGAGTGTCCAGCC | MARCKSL1 | 313 |
| ILMN_1806818 | CAGGATGACAATCAGGTCATGGTGTCTGAGGGCATCATCTTCCTCATCTG | MCM3 | 314 |
| ILMN_2224143 | GGCTTTGGGTGGTTCCAATTGGTGGAGAGAAGCTCTGAGGCACGTCATGC | MCM3 | 315 |
| ILMN_2388272 | CCATCTGCTGGCACCTGAGGAGAGTGAGCAGCCTGGACCACAAGCCCAGT | MED24 | 316 |
| ILMN_1711853 | CCTCTAGCGGCTTCCAGTTCCCCGCTCCTGACTCCTGACCTCCAGGATGT | MED24 | 317 |
| ILMN_1755643 | TGCAGAAGGAATGGTGGATCCAAGTCTCAATCCCATTTCAGCCTTTCGAC | MGAT4A | 318 |
| ILMN_2147251 | CTTCTGCCATGATTGTGAGGCCTCCCCAGCCATGTGGAACTGTGAATCCA | HSPC072 | 319 |
| ILMN_1718734 | GTAGGGGTTTCCAGCTTCCCCAGGCTCCGGCCTTGTCAGTCTCTTTGCAT | MLLT6 | 320 |
| ILMN_1750689 | CCCTCTGTGGTTCTGACTGGAGACCCCAGTGTGGGGGAGGTCTTACCATT | MPP5 | 321 |
| ILMN_2189424 | CAAGATGGCAGCGGCGCTGCGCGTGCGTTGTTGAGTGTTCGGGACGCCGG | MRPL20 | 322 |
| ILMN_1693352 | GGCCTGCAGGCGCCATGGTCTTCCTCACCGCGCAGCTCTGGCTGCGGAAT | MRPL20 | 323 |
| ILMN_1691156 | TGCTGCTCCTGCTGCCCCATGAGCTGTGCCAAGTGTGCCCAGGGCTGCAT | MT1A | 324 |
| ILMN_1686664 | GAACCCGCGTGCAACCTGTCCCGACTCTAGCCGCCTCTTCAGCACGCCAT | MT2A | 325 |
| ILMN_1770035 | AGAAGGAGGGTTTCTGGCTGTGGTTCTAAATGGAGCCCCAGGAAGCTGCC | NCOA5 | 326 |
| ILMN_2119937 | CTATCCAGAAGAAGCTGGCTGCAAAAGGGCTAAGGGATCCATGGGGCCGC | NDUFB3 | 327 |
| ILMN_2119945 | CCAGAAGAAGCTGGCTGCAAAAGGGCTAAGGGATCCATGGGGCCGCAATG | NDUFB3 | 328 |
| ILMN_1682993 | CTGAGCCTGGGTGCTCACTGTGGCGGTCCCCGTCCTGGCTATGAAACCTT | NKG7 | 329 |
| ILMN_1749752 | CCCATATAAGCTGCTGCCACTGCAGAGGTTTTTACCTCGACCTCCAGGTG | GAR1 | 330 |
| ILMN_1716678 | AGCTCCGGGTGGCTGGTTCTCAGTGGTTGTCTCATGTCTCTTTTTCTGTC | NPC2 | 331 |
| ILMN_2116127 | CCCGGTTCATTTTATGCGTGCGAGAAGTCAGTGGTAACTGCTGCAGGGCT | NPEPPS | 332 |
| ILMN_1716596 | TATCACAATTGCCACCCATCGGGTTTTGGGTGTGTGTTTTCATAGCGTGG | NSMAF | 333 |
| ILMN_1689342 | GCCAGTCTTTTTTCATTGACGCCCCAGATTCCCCAGCCACGTTAGCCTAC | NUBP1 | 334 |
| ILMN_2097546 | CATCAGGAGAAAGGCTGGGTCTTGGGACCTTGTCCTCCCCAGTTGGCCTA | NUDC | 335 |
| ILMN_1738681 | CAGCTTCCAGTGGTGGCCGTAGACTTGGCTCGGAACTTAGTGGCACCAGA | NUP62 | 336 |
| ILMN_2196569 | CGAGGACCGCGACTCTCAACTCCGAAGTCAAGCCCGCACTCTGATTACCT | NUP93 | 337 |
| ILMN_2410826 | AGAGAGACTTCCTGAAGCAGCGCCCCACCAAGCTCAAGAGCCTCATCCGC | OAS1 | 338 |
| ILMN_1658247 | CTCCTGCTTCCTCCCTGCCATTCATCCCTGCCCCTCTCCATGAAGCTTGA | OAS1 | 339 |
| ILMN_1675640 | AAAGGGTTGGAGGCAGCTGGCACAAGAGGCTGAGGCCTGGCTGAATTACC | OAS1 | 340 |
| ILM N_2405078 | GGCTATTCCTCCCCAGAACCTGACATTCAAGACTCCTCTGGAAGTGAAGC | OSBPL8 | 341 |
| ILMN_2209180 | GTTGCCAACTGTTGTTCCAGCCATCCACACAGGAGTCTGTTCTGAGGTGG | RPRD1A | 342 |
| ILMN_1798354 | CCTGGAGATCAGACTGTTGCTTTCGCATGATGTATGTAGTGTCTCATGAC | PAPOLA | 343 |
| ILMN_1712950 | GGGGCCCCCACCTTCAATGTCACTGTCACCAAGACTGACAAGACGCTAGT | PFN1 | 344 |
| ILMN_2151817 | GGCTGGATGGACAGACACCTCCCCCTACCCATATCCCTCCCGTGTGTGGT | PFN1 | 345 |
| ILMN_1661366 | GCTAATCCCAGTCGGTGCCGCATCCCCAGCCCGCCGCCATGGCCGCCTAC | PGAM1 | 346 |
| ILMN_2112417 | ATTGTCAAGCATCTGGAGGGTCTCTCTGAAGAGGCTATCATGGAGCTGAA | PGAM1 | 347 |
| ILMN_1813657 | CCATGTTGAGGGGCTCCATTCCCAATTCCTGGGTCAAGGTGAATTAACCC | PHF20 | 348 |
| ILM N_2112402 | CTTGCAGCAGCTCTGGTGGCAGCTGTCCTTGAGGAACCTTTGGTGTGTGG | PHF5A | 349 |
| ILMN_1691291 | TAGTTGGCTTTGTCTGTCAGGTGCAGTCTGGCGGGAGTCCAGGAGGCAGC | PIGS | 350 |
| ILMN_1698072 | TCCTCGGACCCGAGAACCCGAAAATTGCCAAGGACCCATCCTGGATCATC | PITRM1 | 351 |
| ILMN_1688702 | AGCCCAGGTCTAAATGTAATGGTTGGTTTATTGTTCTATAACCCCAGCCC | PJA2 | 352 |
| ILMN_1786601 | CCACTTGCACCTCTCCACCTTTGGCACTAGAACTCCTGAGACACCACTTC | PLAGL2 | 353 |
| ILMN_1756669 | GAGTAACGGCTCTGCTGCCAGGGTTTCTCTGGGCTCATTCTTCCACTGAC | POGK | 354 |
| ILMN_2337058 | TGGCTCGGATCCTCAGTGCCTGTGTCTTGTCAAAGCGGTGCCCGCCAGAC | PORCN | 355 |
| ILMN_1716862 | CGTTCAAACTGTCCACTCTGATCCAACCCTGTACTGATAGTACTTCCCAG | PPM1B | 356 |
| ILMN_1734991 | GTAATCCTAGCACTTTTGTCGCCTGGGCGACACACCAAGGCTCTGTCTCA | PPM1B | 357 |
| ILMN_1806867 | CTCCATGGCCCTCGGCCGCTTGCACCCGCTCTCTGTTGTACACTTTCAAT | PPM1G | 358 |
| ILMN_1695827 | TGGCCAAGTTCCTCCACAAGCACGACTTGGACCTCATCTGCCGAGCACAC | PPP1CA | 359 |
| ILMN_2377980 | GCCAAGAAATAGCCCCCGCACACCACCCTGTGCCCCAGATGATGGATTGA | PPP1CA | 360 |
| ILMN_2405018 | GCTGCCCCTGAGAAGAGACTTAATCCAAGCCTGATTGTACTAGTGGCATC | PPP1CB | 361 |
| ILMN_1688865 | CGAGTTCCTGAAAAAGGAGACTGCACAGCGTCGGGTTCTGGAGGAGTCGG | PPP1R9B | 362 |
| ILMN_1738784 | TCCAGTCCTCACAACCTGTCCTTCACCTAGTCCCTCCTGACCCAGGGATG | PPP2R5A | 363 |
| ILMN_1670970 | CTTCTTCCTAGGGGCCTCGTGATCTGAGGGGTGGTGCCTACTTCCACTGT | PPP3CA | 364 |
| ILMN_1724544 | TGATCAGCTCTGAGGTGCAACTTCTTCACATACTGTACATACCTGTGACC | PPP4R1 | 365 |
| ILMN_2345512 | CAGCCCTGTGTGTGAATCGTTTGTGACGTGTGCAAATGGGAAAGGAGGGG | PPP4R1 | 366 |
| ILMN_1796210 | CCCAGAAGAACCTCAGGAGGTAACCTTGGGCCCTTCCCTGCTATCCTTTT | PPRC1 | 367 |
| ILMN_1740633 | GATTCACCCTGTCCAAACTGCCTAAGCCCTCCGCCATTCTCAAGCCCTGC | PRF1 | 368 |
| ILMN_1769545 | GGGAACGGATGTGGAAGGAAGAACTGTCACCCTCTTAAGGCCCAGGGTCG | PRPF19 | 369 |
| ILMN_1759952 | GGAGAAGCTGAATGCAACAAACATTGAGCTAGCCACAGTGCAGCCTGGCC | PSMA5 | 370 |
| ILMN_1683026 | ACCCTGGAGCTAGTGGAGGAAACTGTGCAGGCTATGGAGGTGGAGTAAGC | PSMB10 | 371 |
| ILMN_1764794 | TGCAGCCCACGGCTATGGTGCCTTCCTGACTCTCAGTATCCTCGACCGAT | PSMB2 | 372 |
| ILMN_1702837 | ACCAGTGAAGACATTGAGGAGCTGGTGGAACCTGTGGCAGCACATGGCCC | PSMD1 | 373 |
| ILMN_1806017 | ACCGGGACATCCGGCTGATGGTCATGGAGATCCGCAATGCTTATGCTGTG | PSME1 | 374 |
| ILMN_1726698 | GCTCAGCTTCTCCACAAGGCTAGAAATGGGGCACAGAGCCACTGGAGGCC | PSME1 | 375 |
| ILMN_1786612 | TGATTTTGGGGTAGCAATCCAGGAGAAGGTGCTGGAGAGGGTGAATGCCG | PSME2 | 376 |
| ILMN_2088410 | CTGCAGCTTCGTAGTACTCCCTTCCGGTACCTACTTACACCTTCCATGCA | PSMG2 | 377 |
| ILMN_1701134 | TAATCTGGACATTCGAGGAATTGGCCGCTGTCACTGCTTGTTGTTTGCGC | PTEN | 378 |
| ILMN_1681591 | ACCCTGCAGAGCTATGGTGAGGTGTGGATAAGGCTTAGGTGCCAGGCTGT | PTPN1 | 379 |
| ILMN_1738675 | GGAGAAGAGCAAGGGTTCCCTCAAGAGGAAGTGAGCGGTGCTGTCCTCAG | PTPN6 | 380 |
| ILMN_1728305 | CCTAGTCTTCCTTCATCCTTGCCCTCTGTTGGCACAGGCATTATCTCTGC | PUM2 | 381 |
| ILMN_1808568 | CCTGTCCCATGTTGGAAGTTGCTCTGAAGGGGTGGTAGATGCTGGAAGCC | PYCR2 | 382 |
| ILMN_1672443 | GGTGTCATGTTGGATCGCTTTGTGACTGTTCATCTGTCCTTGACAGTGGC | QDPR | 383 |
| ILMN_1676002 | AATCTCCCCAGCCGACTTCCACTGGGCTGACAGACTTTGCTGACCACAGG | QRICH1 | 384 |
| ILMN_1660691 | CCCTGTAGTCCAGTGGTGCTGCCCTGTTGTGCAAACTGCTCCTTTTTCTC | RAB31 | 385 |
| ILMN_1755364 | GCTTTGAGACCTTTCCTCTCCTGGGTACTGAGGTGCTATGAAGCCAACTG | RALA | 386 |
| ILMN_1758214 | AGAGTCGCGGGGACACAGGAGTCTTCCTACAGTACACACACGCCCGCCTC | RARS2 | 387 |
| ILMN_1654586 | CATCCTAAAAATGGGGTCCAGGCAGACCCCTCCAGACCTCACACTGCCGA | RASA3 | 388 |
| ILMN_2362902 | GCTCCTGCTGCAACCGCTGTGAATGCTGCTGAGAACCTCCCTCTATGGGG | RASSF5 | 389 |
| ILMN_1666739 | GGCTCACACAGCTTAAGAGTAGCTGTCTCTCAAACGTGCGCTCACAGTTG | RBM15 | 390 |
| ILMN_1670456 | AGCTTCGCAAGAGCATGTGGAAGGACCGGAATCTGGACGTGGTCCGCAAG | RBM42 | 391 |
| ILMN_1716465 | GTGCAAACAGACATTCCAGAGAGCCTGATCCACATCCAGCAGCAGAGCCC | RBP7 | 392 |
| ILMN_1720124 | GTTGGGAATGTGCTTGGCGCTGACCCTGCGGGCATCTGACTGGTCTTCCA | RCC2 | 393 |
| ILMN_1705266 | ATACCTGTGGCCCCCCACACAACTGAGCCCATGCTGATGGAGTACCCTGA | RELA | 394 |
| ILMN_1800787 | CTGGCGAACCTTGGAGAGGGAATGCTGATTGTCTTGACCAAACCCACAGC | RFTN1 | 395 |
| ILMN_1661002 | CCCTCCTTTATGACCTTTGGGACATTGGGAATACCCAGCCAACTCTCCAC | RFWD2 | 396 |
| ILMN_2408001 | CCATGGACATTGCTGCTCTTGGTGGTGTTATCTAATTTTTGTGATAGGGA | RFWD2 | 397 |
| ILMN_2124386 | CAGAAATTCAGAAAGGGAGCCAGCCACCCTGGGGCAGTGAAGTGCCACTG | RGL2 | 398 |
| ILMN_2373062 | CCCAGGGAGTGCTCGAGGCGCATCAGGCCCGTTTTTTACCAGTTTATATC | RHBDF2 | 399 |
| ILMN_1735792 | CTCAGTTCCTAATATCCCGCTCCTTGCTGAGACCATCTCCTGGGGCAGGG | RHBDF2 | 400 |
| ILMN_1691717 | TGCTCGAGGCGCATCAGGCCCGTTTTTTACCAGTTTATATCACGGTCTTC | RHBDF2 | 401 |
| ILMN_1810559 | ATACCGGCTTCCAGAGACCCCTTTTCTCCAGCCATATTACATCAGGCTAG | RHOQ | 402 |
| ILMN_1669310 | AATTCTCAGGGCTCTACCCCCCTTTCCTGGTCCTAGGTGGCCAGTGGGTA | RHOT2 | 403 |
| ILMN_2142695 | CCGCAGCTCTCATCATTGTGATGTGTAGCATGTCTGCCCTCTGACTGGAC | RNF4 | 404 |
| ILMN_1687941 | CACTGTCGTCCTTCCTCAGAGGGCCTCACGCCAAACAAACGGCCTTTTCG | RNF4 | 405 |
| ILMN_2375599 | AGCCTGGCTCTGTGCTGCGGGTGCTCTGGTTGGCCGACTGCGATGTGAGT | RNH1 | 406 |
| ILMN_1660880 | GGTCCTGTACGACATTTACTGGTCTGAGGAGATGGAGGACCGGCTGCAGG | RNH1 | 407 |
| ILMN_1691843 | GCACCGATGCACACACCGCACCCCACCACTGTACTCTGAAATTGGCGAGT | RNPS1 | 408 |
| ILMN_1672446 | GCATTGGGGCCAAACACAGAATCAGCAAAGAGGAGGCCATGCGCTGGTTC | RPL11 | 409 |
| ILMN_2114876 | TTGGGGCCAAACACAGAATCAGCAAAGAGGAGGCCATGCGCTGGTTCCAG | RPL11 | 410 |
| ILMN_2413278 | CAGGGGATTTGGGGCTTTCTTGAAAGACAGTCCAAGCCCTGGATAATGCT | RPL13 | 411 |
| ILMN_1762747 | GGTTACCCACTCTGTCCACTCCCATAGGCTACAGAAAAAGTCACAAGCGC | RPL15 | 412 |
| ILMN_2290808 | TCCTTTCGGCCGGAACCGCCATCTTCCAGTAATTCGCCAAAATGACGAAC | RPL21 | 413 |
| ILMN_1752285 | TCCGGGTGGATAAGGCAGCTGCTGCAGCAGCGGCACTACAAGCCAAATCA | RPL4 | 414 |
| ILMN_2331890 | AGGCAGAGGTCCAAGTAAACCGCTAGCTTGTTGCACCGTGGAGGCCACAG | RPL41 | 415 |
| ILMN_2087080 | GGAGCGGGCTGCTGAGAGCTAAACCCAGCAATTTTCTATGATTTTTTCAG | RPL5 | 416 |
| ILMN_1764207 | GCCAACCTCCCTGTCCAGATGCAGCTATTTTGGTATCTCCTATCACATGC | RPRD1A | 417 |
| ILMN_1753534 | CTGGACAACAAGCTCCGTGAAGACCTGGAGCGACTGAAGAAGATTCGGGC | RPS18 | 418 |
| ILMN_2415722 | CCTCGTTGCACTGCTGAGAGCAAGATGGGTCACCAGCAGCTGTACTGGAG | RPS29 | 419 |
| ILMN_2298818 | AGAGCAGCCCAGGTGGTCATCAGAGTCACCAATGCCAATGCCAGCCTGCA | RPS29 | 420 |
| ILMN_1694742 | GCGAAAATTCGGCCAGGGTTCTCGCTCTTGTCGTGTCTGTTCAAACCGGC | RPS29 | 421 |
| ILMN_1738243 | TGGACTAAATGCTCTTCCTTCAGAGGATTATCCGGGGCATCTACTCAATG | RPS29 | 422 |
| ILMN_1673638 | CTGGGGACGAGACAGGTGCTAAAGTTGAACGAGCTGATGGATATGAACCA | RPS3A | 423 |
| ILMN_2139943 | CCAACAGGTCCGCCAAATCCGGAAGAAGATGATGGAAATCATGACCCGAG | RPS3A | 424 |
| ILMN_1657722 | GGGACGAGACAGGTGCTAAAGTTGAACGAGCTGATGGATATGAACCACCA | RPS3A | 425 |
| ILMN_1657515 | GGGGCATGTTGTGTCATGTAGTCAGCCACTTATGCACCAATGTGAGGAAA | RPS6KA5 | 426 |
| ILMN_1812973 | GTCCTATAAATGCACCTCCTGTCAAAACCATGCCTGAGAGGTCCCGGCTG | RRP1B | 427 |
| ILMN_1767658 | CCTGGCCGCTGCCTTCATTGAGTTTAAAGGGACAGGATTGCCCTTCCGTC | RRS1 | 428 |
| ILMN_2175465 | CCAGCATTGATCTAGAAGCAGAGGAATCCCAGCGCCTTTTAAAAGTTGTT | RSL24D1 | 429 |
| ILMN_1756928 | CGTGGCTAGGCCTTTCCTGCCGAGTGCTCTGATGCAATAGTGGAAATCGC | RTN1 | 430 |
| ILMN_1787461 | AACCATCCCAGAGCTGGCGAGAGGATGGAGCTGGGTGGAAACTGCTTTGC | RUNX3 | 431 |
| ILMN_2046730 | GAGCAGTTCGCTCCTCCCTGATAAGAGTTGTCCCAAAGGGTCGCTTAAGG | S100A10 | 432 |
| ILMN_1796712 | GGCAGAAATGAGCAGTTCGCTCCTCCCTGATAAGAGTTGTCCAAAGGGTC | S100A10 | 433 |
| ILMN_1729801 | TAACTTCCAGGAGTTCCTCATTCTGGTGATAAAGATGGGCGTGGCAGCCC | S100A8 | 434 |
| ILMN_1697554 | GGGCCTCCCTTGACCCCAGTACGAAGTCTATGCCCTGAATCCCCAGAGTA | SASH3 | 435 |
| ILMN_1728298 | CAGAGCCCCAGCCCCTCATGTCTTGCCGCCCTTCCTCCATGTGTTTGTAA | SBK1 | 436 |
| ILMN_1744210 | GCCAACCGCCTCGGGGCAAACTCGCTCTTGGACCTGGTTGTCTTTGGTCG | SDHA | 437 |
| ILMN_2051232 | CTGTGCCACCATCCCGCCAGCCATTCGCTCCTACTGATGAGACAAGATGT | SDHA | 438 |
| ILMN_1657483 | GCTGTCTCCAGTGCCTGTTAAGCTGAGGATACAACCAGGAAATGCAACGG | SEC23B | 439 |
| ILMN_2366246 | GCCACGTTACATCAACACGGAGCATGGAGGCAGTCAGGCTCGATTCCTTT | SEC23B | 440 |
| ILMN_1724959 | GCGCTGCCTTTCTTCAGCAACAGACCCTCAAACCAAGAGGAAGCTAGATG | SEC31A | 441 |
| ILMN_2399896 | CCCAGGCCAATAAGCTGGGTGTCTAAAAGGACAGCTTCTCTTCCACTCAA | SEC31A | 442 |
| ILMN_1769118 | GTTCGTTTCATCAGGCTCTGTTCCTCAATGGCCTTTTGCTACGTGCCTCC | SEPT9 | 443 |
| ILMN_1775939 | ATGGCCATGACCCAGAAGTATGAGGAGCATGTGCGGGAGCAGCAGGCTCA | SF3B2 | 444 |
| ILMN_1722648 | GCTGTAGCTCCTTGGGGCAAAGGTACTAATCCCTTTCAGCACCCCCACTC | SF3B4 | 445 |
| ILMN_1659874 | GCCTGAGGTGACAGACAGGGCAGGTGGTAACAAAACCGTTGAACCTCCCA | SCAF4 | 446 |
| ILMN_2069593 | CTGCTCCGACAGCAGCCCCAGGAAATACGGGAATGGTTCAGGGACCAAGT | SCAF11 | 447 |
| ILMN_2175075 | TGGCCTTTCCTACAGGGAGCTCAGTAACCTGGACGGCTCTAAGGCTGGAA | SRSF4 | 448 |
| ILMN_1752046 | AAGGCCTTGGACTCTTCCCTGAGGGTTGCCTGAAATTCCTTCATGCTTTC | SH2B3 | 449 |
| ILMN_1737163 | ATCACCACACTCCCCCCAGCCTTCACCTGGCCATGAAGGACCTTTTGACC | SH3BGRL3 | 450 |
| ILMN_1712887 | GGTCATTGGCCACTTCATCTACAGCAGCCTGTTCCCAGTTCCCTGAGGCC | SLC10A3 | 451 |
| ILMN_1814173 | CCGTCCACTCCTCCTACTGTATTTTATTGGACAGGTCAGACTCGCCGGGG | SMARCA4 | 452 |
| ILMN_1752111 | ATGGAGCTCCTGGAAGCAGCAGAGTCCTTTGACCCAGGAAGTGCTTCAGG | SMARCAL1 | 453 |
| ILMN_1774661 | CCACCAAGGCCCTAGACTCATCTTGGCCCTCCTCAGCTCCCTGCCTGTTT | SNRPB | 454 |
| ILMN_1799103 | GCGTGGGCTCGATTCCTCAGGGCCACGTTACCACAGACCTGTTTGTTTCT | SNRPB | 455 |
| ILMN_1793410 | CCCCATTTCATGGTTTTCAGTGGCAACTTACTGACCCTTGTTTTTGCCTG | SNTB1 | 456 |
| ILMN_1707077 | GGTCCCCATGTGCCTGTTGTTCAGCCCTCTCTCTTGTTCCCTTTCTGAGC | SORT1 | 457 |
| ILMN_1666482 | CACCTCCCTGATGCCTGCTTTCAGTTGAGGGTTGGGGGCAATGATGAGCA | SP2 | 458 |
| ILMN_1684446 | CTGCTATTAGAGCCCATCCTGGAGCCCCACCTCTGAACCACCTCCTACCA | SPAG7 | 459 |
| ILMN_1682864 | TGGAGCCCGCGTTGCTGTTCCCACAGGGCCTCGGTTTTTCCTAACTTGCT | SPSB3 | 460 |
| ILMN_2249473 | GATACCTGTAATCCCAGCTACGTGGGAGGCTGAGGTGGGAGAATTGCTTC | SPTLC1 | 461 |
| ILMN_1813491 | GGTGGGTTTGCCTAGGGACGTGTAACTACAGGCTTTTACTAAGCCAAGGA | SPTLC1 | 462 |
| ILMN_1662618 | GGAGGCCAGTGTTGTGGGCTTCCTGCTGGGACTGAGAAGGCTCACGAAGG | SQSTM1 | 463 |
| ILMN_1729987 | ACCCAAGTCTTCTCCCGTCCATTCCAGTCAAATCTGGGCTCACTCACCCC | SRC | 464 |
| ILMN_1803398 | TATGTGCAGCGACCCTTGGTGTTTCCCTTCCTCGGTGGCTCTGGGGTATG | SRF | 465 |
| ILMN_1703524 | GCCTTTGGTCAGTAATGCGTTCAGGAGTCCACACCAGGCACAGATGGGGC | SRP68 | 466 |
| ILMN_1798804 | GGCAGCTGTAGATCTTGATCTTCCAGGTACCCCATGTACCTTTATTGAGC | SRPK1 | 467 |
| ILMN_1785660 | TCTTGGCCTCCCAACTCTTCCCACTCCCAGAATCCAGAAGTAAGCTCTGC | SRPR | 468 |
| ILMN_1756501 | CGACAGCAGGACATACATGTTGGTGTGAAGACTGGGACGACACTGGGTAG | ST6GAL1 | 469 |
| ILMN_1690761 | TCAACCTCACCAGGGCTGTCTCTTGGTCCACACCTCGCTCCCTGTTAGTG | SURF4 | 470 |
| ILMN_1778032 | GGAGCAGTACCTTCCCGGAGTCCACGCATGTGAGTTGGGTCAAGTGCATT | SURF6 | 471 |
| ILMN_1797813 | CTCTGATTTTTGCCTCTGGATAGTAGATCTCGAGCGTTTATCTCGGGCTT | SUZ12 | 472 |
| ILMN_1678707 | TTGCCAGAGTTTTGCCTGCTGCTTTCCTCGTGGCCTCTTCTTGGGTAGTG | TAF15 | 473 |
| ILMN_2402131 | ATGGAGGAGACCGAGGAGGTGGCTATGGAGGAGATCGAGGTGGCTATGGA | TAF15 | 474 |
| ILMN_1751079 | GTAACGGAGTTTAGAGCCAGGGCTGATGCTTTGGTGTGGCCAGCACTCTG | TAP1 | 475 |
| ILMN_1723944 | AAAGAAGAGCAACACGATTCTGGGATCCCAGGAGGGGAACACCATGAAGA | TARP | 476 |
| ILMN_2374770 | GTCTGCAGCCAGCCTGCTCGAAACTTTAGTCGGCCTGATGGCTTAGAGGA | TAX1BP1 | 477 |
| ILMN_1793118 | GAGCGGATGCTTTCATGCACCCTTTACTGCACTTTCTGACCAGGAGCTAC | TAX1BP1 | 478 |
| ILMN_1803941 | CACTCTCTGCCAGTGGAGCCAGAAATGACAGCCCAACACAGATACCAGTG | TBC1D15 | 479 |
| ILMN_1672486 | CGCTCGTCACCAAGTCTTTAGAATAGCTTTAGCGTCGTGAACCCCGCTGC | TCF7L2 | 480 |
| ILMN_2285713 | CTGTATTAAAAAGGCAAATCGAAGGCCGGGCGCGGTGACTCACGCCTGTC | TDP1 | 481 |
| ILMN_2391789 | CAGCCCCCTGCAGCTAAGAATTGTATTGACTGTCCTCACAGCGGCTTTTC | TDP1 | 482 |
| ILMN_1726520 | AGGGCTGCAGGGCCTCCCACCTTCCAACAGACAGGCTCTGCTGTATCTGT | TDP1 | 483 |
| ILMN_1768488 | GGGCTATGTAGGCAGGTTAATCCTCCACTTCTCATGTGGTTGAACCAGTG | TERF2 | 484 |
| ILMN_1663954 | CTTCAGTGAGAAACTGCCCTTACAAACAGTCCCTTCTCTGCTGTCAATCC | TH1L | 485 |
| ILMN_1711450 | CATCGCAGGTACCATCAAAACGGAAGGCGAGCATGACCCTGTGACGGAGT | TH1L | 486 |
| ILMN_1750518 | TCGCTCTGGTCGCAGCTTAGGAACAGCAGACGTGCACTTTGAGCGGAAGG | THOC4 | 487 |
| ILM N_1815079 | GCTGCAACCCCTCATTATCCACCACGCACAGATGGTACAGCTGGGGCTGA | TICAM 1 | 488 |
| ILMN_1724863 | GACAAAACGGGCGCGATGATGCCCTGGCTTTCAGGGTGGTCAGAACTGGA | TICAM1 | 489 |
| ILMN_1674421 | GCAGCGCCAAGCGGCATCCACCAAGCATCAAGTTGGAGAAAAGGGAACCC | TM9SF4 | 490 |
| ILMN_2092756 | CTTCTCTCCCCATCGCTCCACAACCTGAAACCGAGAAGGAGTTGCTGACC | TMEM109 | 491 |
| ILMN_1753608 | CTGAGAACCTTTCCCGTTACTGCGTTTTCACCACCTGTCTTCCCCATGCT | TMEM131 | 492 |
| ILMN_2212714 | GCTGGAGGGCGCTGTCATTGTCTATCAGCTGTACTCCCTAATGTCCTCTG | TMEM39B | 493 |
| ILMN_1692754 | ATATTCCATCCTGCCCAACCCTTCCTCTCCCATCCTCAAAAAAGGGCCAT | VMP1 | 494 |
| ILMN_1674402 | TGACTCAAGGGCTGTAGATGTTCCCTTTCCACCCCCCACACTTGGTGCGT | TMEM71 | 495 |
| ILMN_1801307 | GTAGTGTATCACAGTAGTAGCCTCCAGGTTTCCTTAAGGGACAACATCCT | TNFSF10 | 496 |
| ILMN_2214197 | GGCATGAGTTAGGGAGACTGAAGAGTATTGTAGACTGTACATGTGCCTTC | TP53INP1 | 497 |
| ILMN_1714108 | AGTACCTGGGGAGGTTAGATGTGTGTTTCAGGCTTGGAGTGTATGAGTGG | TP53INP1 | 498 |
| ILMN_1729234 | CAGTGCTTGGCACCTGGAAGTAGGTGGCAGATGTTAACGCCCTTCCTCCC | TPP1 | 499 |
| ILMN_1695432 | GGTGAACCAGAACAGCACCTCCTCCCACTTAGGAAGCTCGTGATTTCCAG | TPST2 | 500 |
| ILMN_2329679 | CCTTAATTTGCAGGACTGCCTTGGTGGCTTTGTTTGCTGGGACAAGGCCC | TPST2 | 501 |
| ILMN_2392143 | TATTAGTGCGCTGTGAGGTCTCCACCCGCTTTGACATGGGTAGCCTTCGG | TRAF6 | 502 |
| ILMN_1758250 | CAGTAACGAGGCTTTTGATGTGTTGAGCTGGAGGTGAGTGGACCGGGGGC | TRAFD1 | 503 |
| ILMN_1738704 | TGATCTCCCAGTCTTGTCCTTACCCATTCCAAGTGCTCTGCCAGCCCCTG | TRIM26 | 504 |
| ILMN_1704972 | CACCAATGAATGAGTCCTTAGCCCTGTGTCAGTTTACCCTCGATGCCCTT | TRIM5 | 505 |
| ILMN_2049536 | TGTCCTGGCTTCCCCTCCCAAGGAGGATGAGGATGGTGCCTCTGAGGAAA | TRPV2 | 506 |
| ILMN_1785060 | CCCTGGGGATAGCTGGGGCATTTGTCTAGCTGGGCTACCTTCTAACACTT | TSPAN14 | 507 |
| ILMN_1665583 | TTTGCCCCTCTCACCAGCCGTGGAAGCCAGCAGTATCGAGCTCTCACAGT | TUBB | 508 |
| ILMN_2101885 | CTCTGGAGTGGTGTATACTGCCACATCAGTGTTTGAGTCAGTCCCCAGAG | TUBB | 509 |
| ILMN_1783753 | GCATGCGTGTGCAGGACTGGCTGTGTGCTTGGACTCGGCTCCAGGTGGAA | TXNDC12 | 510 |
| ILMN_1713993 | AGAGGGAAGGCAGGGGTGGACCGCCATGAGCATGAAAAGACCCGAAGCAA | UBAC2 | 511 |
| ILMN_1769520 | CCTCCAGCATTCAGTCCAGGGGGAGCCACGGAAACCATGTTCTTGCTTAA | UBE2L6 | 512 |
| ILMN_1703108 | TCGGTCCTGCTGGAGGCCACGGGTGCCACACACTCGGTCCCGACATGATG | UBE2L6 | 513 |
| ILMN_1810729 | GCCTGAATGATGAGCCTATGTCCCTGCCTAACACTGGTGTCTCACTCATC | UBL3 | 514 |
| ILMN_1685625 | AGGCTCCCTTCTGAGCCTCTCCTGCTGCTGACCTGATCACCTCTGGCTTT | UCP2 | 515 |
| ILMN_1683120 | TTCTCCTAGGGTTATGTCCAGTTGGGGTTTTTAAGGCAGCACAGACTGCC | UNG | 516 |
| ILMN_1656165 | CCCAGTGACGTGGAAGTCATCAGAACCCCACGGTACTTGGAGTACCTCTC | USP9X | 517 |
| ILMN_2175601 | CCTTGGTTCCCTAACCCTAATTGATGAGAGGCTCGCTGCTTGATGGTGTG | VDAC1 | 518 |
| ILMN_1662316 | CTGATATTTTTCCTTGGGGGCGTAACCTTCGCTGAAATTGCTGCCCTGCG | VPS33A | 519 |
| ILMN_2383107 | CACTTGACCCCAGGAGACGTAGGTTGTGGTGAGCTGAGATCGCGCCATTG | VPS41 | 520 |
| ILMN_1703379 | GTTCTGTGTGCTGTGACGACTGTCAAAGAGTATCTGGCCATGGCGGACAC | VPS41 | 521 |
| ILMN_1666632 | TCCAGTCTGTCACCCTCCTTTCCTGCTCCCATACACCCAAGGCTTGTTTC | VPS52 | 522 |
| ILMN_1766435 | GCTAACATCCATTCCCTTTCATACCACCATTTTCACCCTGTTTCTTCCCC | WBP11 | 523 |
| ILMN_1788604 | GCGCTAACATTCACTCTTGTTTGTCCCTGGACTGGCCATGAAGTGAGGAG | WBP2 | 524 |
| ILMN_1675844 | CATACCGGCTGGCCACGGGAAGCGATGATAACTGCGCGGCATTCTTTGAG | WDR1 | 525 |
| ILMN_1780036 | GGCACCGTGTCCAAGTTTTTAGAACCCTTGTTAGCCAGACCGAGGTGTCC | WDR1 | 526 |
| ILMN_1706706 | CTCAGCCCGACTGGATCGCCATCTGCTACAACAACTGCCTGGAGATACTC | DCAF7 | 527 |
| ILMN_1669015 | GGAAGATGCCCCGACTTCTTTGGCCAGTGATGGGGAATCAGTGAGTGCTC | XPNPEP1 | 528 |
| ILMN_2054928 | AAAAAATGATTTCTGGCCGGGCGTGGTGGCTCAAGCCTGTAATCCCAGCA | XPNPEP3 | 529 |
| ILMN_2166506 | TGGAAGCCCTCACCAAGCACTTCCAGGACTGACCAGAGGCCGCGCGTCCA | XRCC6 | 530 |
| ILMN_1766010 | GGGTGGGGTACTTCTCCATAAGGCATCTCAGTCAAATCCCCATCACTGTC | YARS | 531 |
| ILMN_1694385 | CTGCCTAACGTTTGCTTCTGTGATGGTTATATTGCCTAGCAAGCACACCC | YWHAB | 532 |
| ILMN_2277099 | TTGCCCATTCGGCTGTGGATAGAGAAGCAGGAAGAGCACTGGACTTGGAG | YWHAB | 533 |
| ILMN_1795905 | CTGAGGCAAACAGGCATGGGAAAATGGAAGGGTTGAGGATGGACCGGAGA | ZBTB4 | 534 |
| ILMN_2097793 | CCAGAATGTGGTGGTTCTGGGCAACAAATGAGATTGTGGCGACGTGGAGA | ZBTB4 | 535 |
| ILMN_1730568 | TGTGTCACAGCCAGAGGGACAAAGTGTGGGTGATCCTGGAGACGCCAGTT | ZDHHC7 | 536 |
| ILMN_1656413 | GGGATCAACTGTACGCCTTTGGTATCTGACCATAAAGTCTTTTGCTCCGC | ZMPSTE24 | 537 |
| ILMN_1753782 | AGCCAAGGACAGAGACCTGGAACAGATGCTTTCATTATGGCCTCCAGAGG | ZNF266 | 538 |
| ILMN_1711361 | GGTGCTGGCTCTCTGTCACAATGCCTCAAAAGACATGGAACCCAGGCCTA | ZNF319 | 539 |
| ILMN_1710873 | CTCACTATGAGGAACAAGAGAACTAGGGGAGCTGCTCTGGTGGCCGTGTG | ZNF330 | 540 |
| ILMN_1786722 | AGTCTGTAGCCTCCCCGATCCAAGTTCCTAGACCTCATGGCTGTCCCCTC | ZNF385A | 541 |
| ILMN_2372200 | AGGCCTTGTTGCTTAAGACACCTTCAGTCTTTGCAGGAGGGCATGGAAGC | ZNF586 | 542 |
| ILMN_1666727 | TTCCAGCCTGCCAGTCATGAATCTCAGACAGCCTGCCACCTATTGCCCTG | ZNF586 | 543 |
| ILMN_2075818 | GAGCTGAAAGCTGCGGCGCCACTGGTGCCAGAGTCAGATGTCACAGATGT | ZNF598 | 544 |
| ILMN_1727183 | AGGCGTGGTGGCCTGCTTCTGTAATCCTAGCTAGTTGGGAGGCTGGCACA | ZNF763 | 545 |

**Table 2**

| illumina_Probe | 50-base_Sequence | Gene_Symbol | Gene_Name |
|---|---|---|---|
| ILMN_1770031 | CCAGGCAGAGACTACCTTTGTGACCAGCTCCCAAAAACCCCAGGCAC | ABHD10 | abhydrolase domain containing 10 |
| ILMN_1673252 | CAGTGTGACAGTGCCAGCCAATGTGCAGAGGTGGATGAGGTCTTGTGAAA | AIMP2 | aminoacyl tRNA synthetase complex-interacting multifunctional protein 2 |
| ILMN_1732060 | TCTGCTCTCCGCCCTCCTTTGTGTATCAAGTGTCGCTCACAGCCCCATTC | ARHGAP1 | Rho GTPase activating protein 1 |
| ILMN_1718610 | GTGACTGCTGGCTCTGTCACCTCATCAAACTGGATGTGACCCATGCCGCC | ARHGAP17 | Rho GTPase activating protein 17 |
| ILMN_2398847 | GGAGCAGGGGCAGGCGAACCTCTTTCTTTGCAGACCGAACAGACCGAACAGTGAAAAGC | ARHGAP17 | Rho GTPase activating protein 17 |
| ILMN_1734742 | CAGGCCTTCCCTGACCCAGCCAGGAACAAACAAGGGACCAAGTGCACACA | ARHGDIA | Rho GDP dissociation inhibitor (GDI) alpha |
| ILMN_1652722 | GTACTGCTTTCAGTGTGTTCCCCCTCAGCCCCTCCGGCGTGTCAGGCATA | C14orf2 | chromosome 14 open reading frame 2 |
| ILMN_1773407 | ACGTCATAGCTCCTTAGTTCTGCTCCTGTCGCCCTAACTTGGCATGGGCA | C16orf72 | chromosome 15 open reading frame 72 |
| ILMN_1719097 | CCAAACACCAAGTGTTAGCTGCCTTAAGGTTTATCCGGGGCATTGGTGGC | C18orf8 | chromosome 18 open reading frame 8 |
| ILMN_1757347 | GCCCCTTTAGTCCCCGCTCTGGAAGGCCAGGCAGTTTAGGTGTAAATAGG | KIAA0930 | KIAA0930 |
| ILMN_1714599 | GGTCTCAGCCAGCCCTAGAGACTGCTTCTTGTGTTTGTGTCATTCTGTCC | CA M LG | calcium modulating ligand |
| ILMN_1736567 | CCCCGTTCCTGACATCACAGCAGCCTCCAACACAAGGCTCCAAGACCTAG | CD74 | CD74 molecule, major histocompatibility complex, class II invariant chain |
| ILMN_1761464 | TATGGGAGCATCGGCTACTGCTGGTGTGTCTTCCCCAACGGCACGGAGGT | CD74 | CD74 molecule, major histocompatibility complex, class II invariant chain |
| ILMN_2379644 | AAGGCACAGGGAGAAGGGATAACCCTACACCCAGACCCCAGGCTGGACAT | CD74 | CD74 molecule, major histocompatibility complex, class II invariant chain |
| ILMN_1751851 | CACTGTCTCAGAGAGGTTTTCCTGTGCTCGCCCTGTTTCTCTCAGGAAGC | CECR1 | cat eye syndrome chromosome region, candidate 1 |
| ILMN_1809285 | GGAAAGCCAGCTCCCCTTCCTCTAACTATTGGAACGCCAGAAAGTCAG | DCP1A | DCP1 decapping enzyme homolog A (S. cerevisiae) |
| ILMN_2168952 | AGTCCAGGTTCAAGACTAGCCTGGGCAACATGGCAAGACCCTGTCCCTAT | DENR | density-regulated protein |
| ILMN_1760954 | GTCTGTTGCTGCTGAAAGATGTCGTGTGCCTGTATCAACATGTGACTTC | DENR | density-regulated protein |
| ILMN_2248589 | AGCGCCTCCACCTGGCCTACTCTGTTATTTCCACCTGTTTGGGTAGGGCC | DHX40 | DEAH (Asp-Glu-Ala-His) box polypeptide 40 |
| ILMN_2325008 | GCAGCAGAGGACCCAGGACCACAGTGACACACGAAAGGAAACAGGCTAAG | DHX40 | DEAH (Asp-Glu-Ala-His) box polypeptide 40 |
| ILMN_2157435 | CATGGCAGTCGCTTGGAACCCACTCACACCAATCCAGTGACCGTGTGTGG | DYNLRB1 | dynein, light chain, roadblock-type 1 |
| ILMN_1766762 | AGAAAGGCACAGGACTCGCTAAGTGTTCGCTACGCGGGGCTACCGGATCG | DYNLRB1 | dynein, light chain, roadblock-type 1 |
| ILMN_1658053 | ATGGCAGAGGTGGAGGAGACACTGAAGCGACTGCAGAGCCAGAAGGGAGT | DYNLRB1 | dynein, light chain, roadblock-type 1 |
| ILMN_1744665 | GTGCAAGCGCCACGGACCTGGGACTCAGCACCGATAACTCAGACTTGAAT | EP300 | E1A binding protein p300 |
| ILMN_1768743 | TCCCCATCTTCCTGGTTCTGCTCCTTCGTGGCCTAGTCTTGTGGACACCA | FIP1L1 | FIP1 like 1 (S. cerevisiae) |
| ILMN_1745217 | GTTCTACAACTTGCACAGGGTAACAGAGGAAGTGGCTGAGGCCTAGAGTC | KIAA1310 | KAT8 regulatory NSL complex subunit 3 |
| ILMN_1737308 | AGTTGGTCTTGGTGTCATATGGATCAGAGGCACAAGTGCAGAGGCTGTGG | GLRX | glutaredoxin (thioltransferase) |
| ILMN_1711617 | TCCTCAGGTGACTGGGGACTTGGAACCCTAGGACCTGAACAACCAAGACT | GMFG | glia maturation factor, gamma |
| ILMN_1679809 | TTCCTGGCCTCCCCTGAGTACGTGAACCTCCCCATCAATGGCAACGGGAA | GSTP1 | glutathione S-transferase pi 1 |
| ILMN_1696485 | TACGACTACTCGCCCTATGGCTATTACGGCTACGGCCCCGGCTACGACTA | HNRNPAB | heterogeneous nuclear ribonucleoprotein A/B |
| ILMN_2335718 | GCGGCAGCAGGAGCGACCAACTGATCGCACACATGCTTTGTTTGGATATG | HNRNPAB | heterogeneous nuclear ribonucleoprotein A/B |
| ILMN_1651262 | CCAGAGCTCTAGGTGTTTAGGCAGCGTGTGGTGTCTGAGGCCATAGCG | HNRNPAB | heterogeneous nuclear ribonucleoprotein A/B |
| ILMN_1718537 | CCTGGAGGCTTGCTTGGGACTGGAGGCTTGCTTGGACAGTTCCTCTGTGT | HPS6 | Hermansky-Pudlak syndrome 6 |
| ILMN_1699362 | GGGGCCCTTAGGCCGTTGGGACTTTGATACCCAGGAAGAATACAGCGAGT | IK | IK cytokine, down-regulator of HLA II |
| ILMN_1793384 | ATTGCCTCTGACGTCTGGTCTTTTGGAGTCACTCTGCATGAGCTGCTGAC | JAK1 | Janus kinase 1 |
| ILMN_1777584 | AAAACTTCTGTACTGCCCTGGAATATGGGCTGCCCCCCACAGCTGGCTGG | KARS | lysyl-tRNA synthetase |
| ILMN_1704750 | CCCTTTCCTCTCCCTCAGAATTTGTGTTTGCTGCCTCTATCTTGTTTTTT | TUBB | tubulin, beta class I |
| ILMN_1703692 | TCCTTTCCAACTCTACCTCCCTCACTCGAGCTCCTTTCCCCTGATCACAGAG | TUBB | tubulin, beta class I |
| ILMN_1700025 | CCCTCCTGTGAGAGTCTGAAGGATACTATTGCCAGAGCTCTGCCTTCTGG | LOC100290936 | phosphoglycerate mutase 1 (brain) |
| ILMN_2103919 | GGACCCGGACACCCTGTGGGACCTGGCCTCAAACTCACCAAATCGCTCAT | LRFN3 | leucine rich repeat and fibronectin type III domain containing 3 |
| ILMN_1806818 | CAGGATGACAATCAGGTCATGGTGTCTGAGGGCATCATCTTCCTCATCTG | MCM3 | minichromosome maintenance complex component 3 |
| ILMN_2224143 | GGCTTTGGGTGGTTCCAATTGGTGGAGAGAAGCTCTGAGGCACGTCATGC | MCM3 | minichromosome maintenance complex component 3 |
| ILMN_1798354 | CCTGGAGATCAGACTGTTGCTTTCGCATGATGTATGTAGTGTCTCATGAC | PAPOLA | poly(A) polymerase alpha |
| ILMN_2112402 | CTTGCAGCAGCTCTGGTGGCAGCTGTCCTTGAGGAACCTTTGGTGTGTGG | PHFSA | PHD finger protein SA |
| ILMN_1738784 | TCCAGTCCTCACAACCTGTCCTTCACCTAGTCCCTCCTGACCCAGGGATG | PPP2R5A | protein phosphatase 2, regulatory subunit B', alpha |
| ILMN_2362902 | GCTCCTGCTGCAACCGCTGTGAATGCTGCTGAGAACCTCCCTCTATGGGG | RASSF5 | Ras association (RaIGDS/AF-6) domain family member 5 |
| ILMN_2290808 | TCCTTTCGGCCGGAACCGCCATCTTCCAGTAATTCGCCAAAATGACGAAC | RPL21 | ribosomal protein L21 |
| ILMN_2087080 | GGAGCGGGCTGCTGAGAGCTAAACCCAGCAATTTTCTATGATTTTTTCAG | RPL5 | ribosomal protein L5 |
| ILMN_1673638 | CTGGGGACGAGACAGGTGCTAAAGTTGAACGAGCTGATGGATATGAACCA | RPS3A | ribosomal protein 53A |
| ILMN_2139943 | CCAACAGGTCCGCCAAATCCGGAAGAAGATGATGGAAATCATGACCCGAG | RPS3A | ribosomal protein S3A |
| ILMN_1657722 | GGGACGAGACAGGTGCTAAAGTTGAACGAGCTGATGGATATGAACCACCA | RPS3A | ribosomal protein S3A |
| ILMN_1729801 | TAACTTCCAGGAGTTCCTCATTCTGGTGATAAAGATGGGCGTGGCAGCCC | S100A8 | 5100 calcium binding protein A8 |
| ILMN_1744210 | GCCAACCGCCTCGGGGCAAACTCGCTCTTGGACCTGGTTGTCTTTGGTCG | SDHA | succinate dehydrogenase complex, subunit A, flavoprotein (Fp) |
| ILMN_2051232 | CTGTGCCACCATCCCGCCAGCCATTCGCTCCTACTGATGAGACAAGATGT | SDHA | succinate dehydrogenase complex, subunit A, flavoprotein (Fp) |
| ILMN_1724959 | GCGCTGCCTTTCTTCAGCAACAGACCCTCAAACCAAGAGGAAGCTAGATG | SEC31A | SEC31 homolog A (S. cerevisiae) |
| ILMN_2399896 | CCCAGGCCAATAAGCTGGGTGTCTAAAAGGACAGCTTCTTCCACTCAA | SEC31A | SEC31 homolog A (S. cerevisiae) |
| ILMN_1752111 | ATGGAGCTCCTGGAAGCAGCAGTCCTTTGACCCAGGAAGTGCTTCAGG | SMARCAL1 | SWI/SNF related, matrix associated, actin dependent regulator of chromatin, subfamily a-like 1 |
| ILMN_1666482 | CACCTCCCTGATGCCTGCTTTCAGTTGAGGGTTGGGGGCAATGATGAGCA | SP2 | Sp2 transcription factor |
| ILMN_2212714 | GCTGGAGGGCGCTGTCATTGTCTATCAGCTGTACTCCCTAATGTCCTCTG | TMEM39B | transmembrane protein 398 |
| ILMN_2049536 | TGTCCTGGCTTCCCCTCCCAAGGAGGATGAGGATGGTGCCTCTGAGGAAA | TRPV2 | transient receptor potential cation channel, subfamily V, member 2 |
| ILMN_1665583 | TTTGCCCCTCTCACCAGCCGTGGAAGCCAGCAGTATCGAGCTCTCACAGT | TUBB | tubulin, beta class I |
| ILMN_2101885 | CTCTGGAGTGGTGTATACTGCCACATCAGTGTTTGAGTCAGTCCCCAGAG | TUBB | tubulin, beta class I |
| ILMN_1713993 | AGAGGGAAGGCAGGGGTGGACCGCCATGAGCATGAAAAGACCCGAAGCAA | UBAC2 | UBA domain containing 2 |
| ILMN_1766435 | GCTAACATCCATTCCCTTTCATACCACCATTTTCACCCTGTTTCTTCCCC | WBP11 | WW domain binding protein 11. |
| ILMN_1694385 | CTGCCTAACGTTTGCTTCTGTGATGGTTATATTGCCTAGCAAGCACACCC | YWHAB | tyrosine 3-monooxygenase/tryptophan 5-monooxygenase activation protein, beta polypeptide |
| ILMN_2277099 | TTGCCCATTCGGCTGTGGATAGAGAAGCAGGAAGAGCACTGGACTTGGAG | YWHAB | tyrosine 3-monooxygenase/tryptophan 5-monooxygenase activation protein, beta polypeptide |
| ILMN_1753782 | AGCCAAGGACAGAGACCTGGAACAGATGCTTTCATTATGGCCTCCAGAGG | ZNF266 | zinc finger protein 266 |
| ILMN_1711361 | GGTGCTGGCTCTCTGTCACAATGCCTCAAAAGACATGGAACCCAGGCCTA | ZNF319 | zinc finger protein 319 |

**Table 3. (A) Patient demographics by cancer status within the first (CC1,n = 110), the second (CC2, n = 100) and third dataset (CC3, n = 118). HT = hormone therapy. (B) Cell type and staging information for cancer history in primary (CC1) and secondary (CC2) dataset.**

| **A.** | | **CC1** | | **CC2** | | **CC3** | |
|---|---|---|---|---|---|---|---|
| | | Cases | Controls | Cases | Controls | Cases | Controls |
| Samples (n) | | 55 | 55 | 50 | 50 | 59 | 59 |
| Age in years (mean ± sd) | | 58.8 (4.1) | | 58.4 (4.1) | | 58.4 (5.0) | |
| Current HT user (%) | | 5.7 | 16.7 | 10.6 | 18.4 | | 20.7 |
| Current medication users (%) | | 67.4 | 61.1 | 67.7 | 65.9 | | 75.9 |

| **B.** | | **CC1** (55 pairs) | | **CC2** (49 pairs) | | **CC3** (59 pairs) | |
|---|---|---|---|---|---|---|---|
| | | Cases | Controls | Cases | Controls | Cases | Controls |
| **Cancer history at time of blood sampling** | | | | | | | |
| | Primary breast cancer | 53 | 4 | 49 | 1¥ | 59 | 3¥ |
| | Recurrent breast cancer ¥¥ | 2* | 0 | 0 | 0 | 4 | 0 |
| | History of cervix cancer | 4** | 4 | 5 | 5 | 0 | 0 |
| | History of melanoma | 0 | 0 | 0 | 1 | 3 | 2§ |
| | Others | 0 | 0 | 1 | 0 | 3 | 0 |

| **Breast cancer receptor status** | | | | | | | |
|---|---|---|---|---|---|---|---|
| | Estrogen receptor status | | | | | | |
| | Positive | 33 | 2 | 33 | 0 | 42 | 2 |
| | Negative | 6 | 0 | 7 | 0 | 9 | 0 |
| | Progestagen receptor | | | | | | |
| | Positive | 26 | 1 | 29 | 0 | 33 | 1 |
| | Negative | 13 | 0 | 12 | 0 | 17 | 0 |

| **Histology of breast cancer diagnosed at the time of blood sampling** | | | | | | | |
|---|---|---|---|---|---|---|---|
| | Invasive ductal carcinoma | 29 | 2 | 34 | 1 | 38 | 2 |
| | Ductal carcinoma in situ | 1 | 0 | 4 | 0 | 2 | 1 |
| | Invasive lobular carcinoma | 4 | 0 | 2 | 0 | 10 | 0 |
| | Invasive tubular carcinoma | 3 | 0 | 0 | 0 | 2 | 0 |
| | Others | 5 | 2 | 1 | 0 | 0 | 0 |
| | Missing value | 13 | 0 | 8 | 0 | 9 | 0 |

| **Stage of breast cancer diagnosed at the time of blood sampling** | | | | | | | |
|---|---|---|---|---|---|---|---|
| | In situ | 1 | 1 | 4 | 0 | 4 | 1 |
| | Stage I | 21 | 2 | 20 | 1 | 27 | 1 |
| | Stage II | 12 | 1 | 14 | 0 | 18 | 1 |
| | Stage III | 2 | 0 | 1 | 0 | 1 | 0 |
| | Stage IV | 0 | 0 | 0 | 0 | 1 | 0 |
| | Missing value | 19 | 0 | 10 | 0 | 8 | 0 |

| | | | | | | | |
|---|---|---|---|---|---|---|---|
| ^{¥} Previously diagnosed In situ duct carcinoma or invasive duct carcinoma diagnosed years after blood sampling ^{¥¥} Previously diagnosed In situ duct carcinoma or invasive duct carcinoma in the other breast diagnosed few months after blood sampling * One patient being her third diagnosis of breast cancer ** One patient had also a history of thyroid cancer ^{§} One was metastatic and the same patient was diagnosed with endometrial cancer 31 years later | | | | | | | |

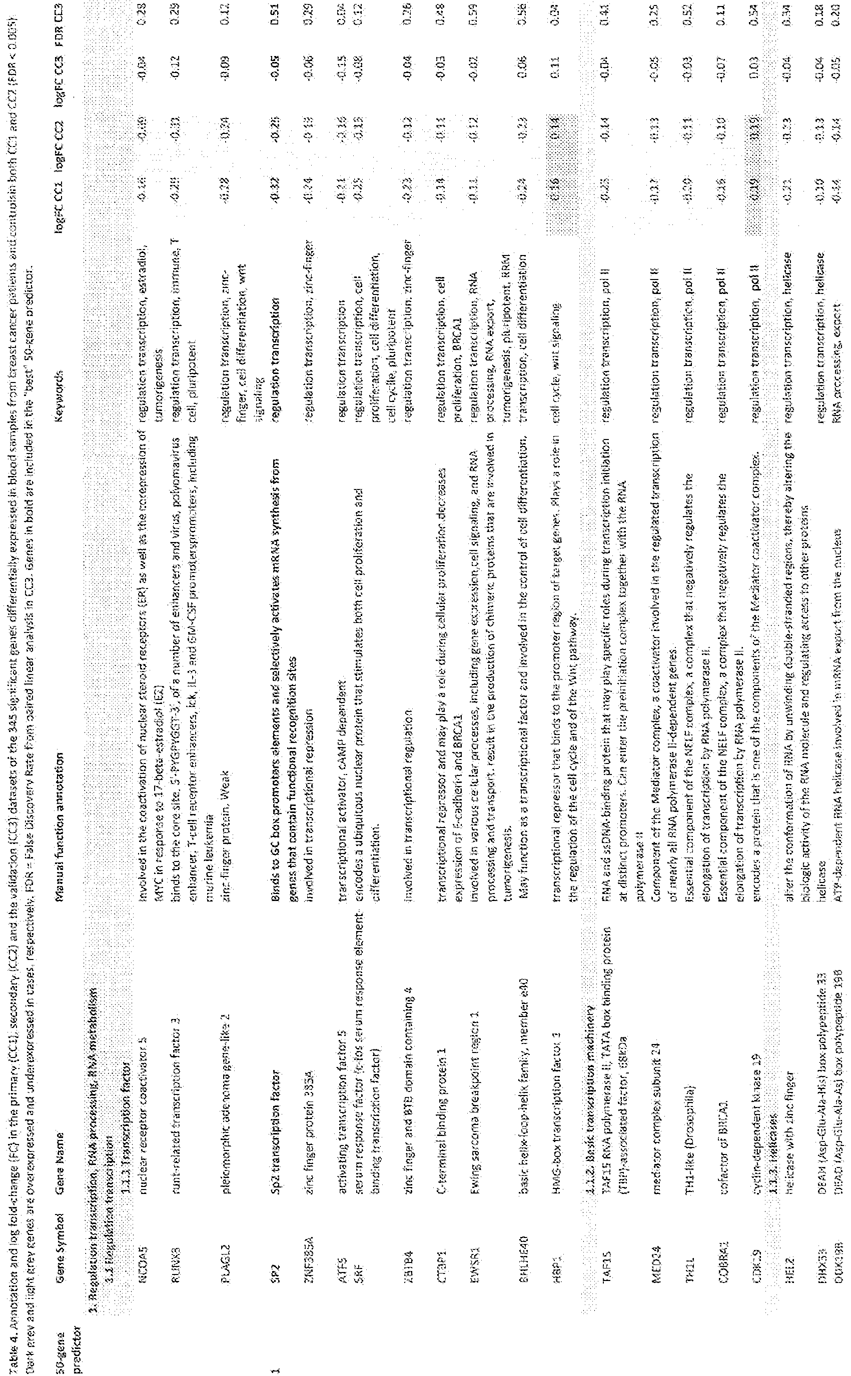

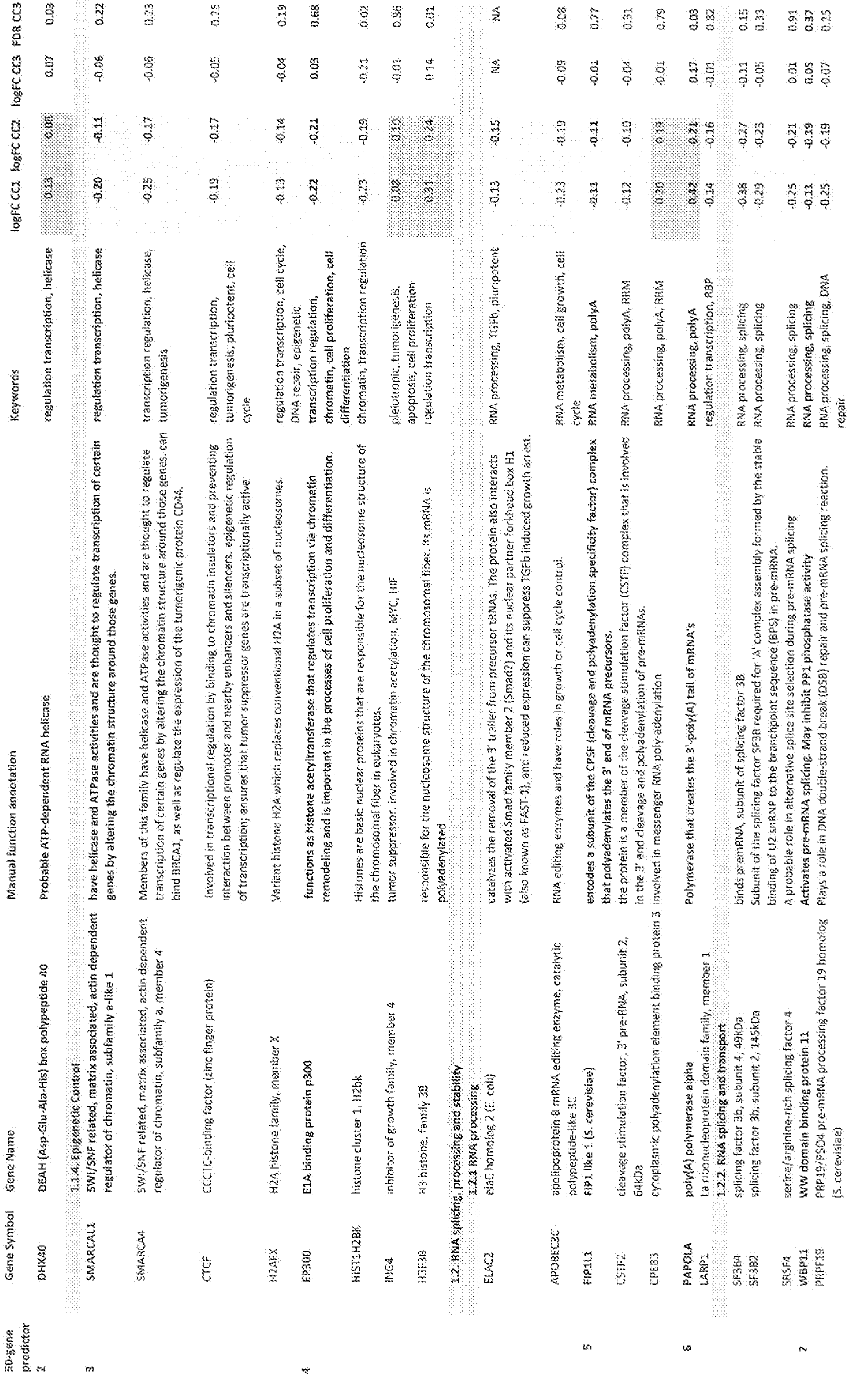

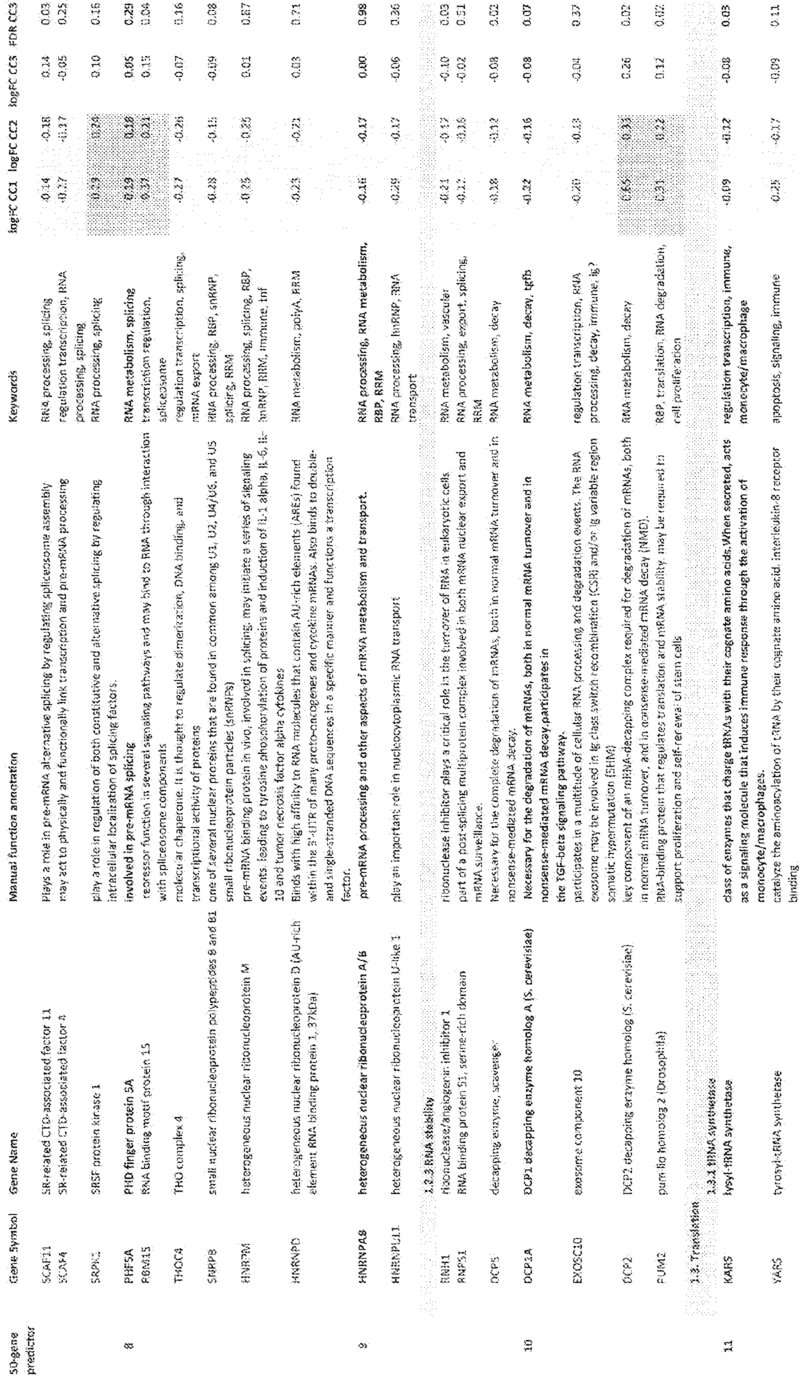

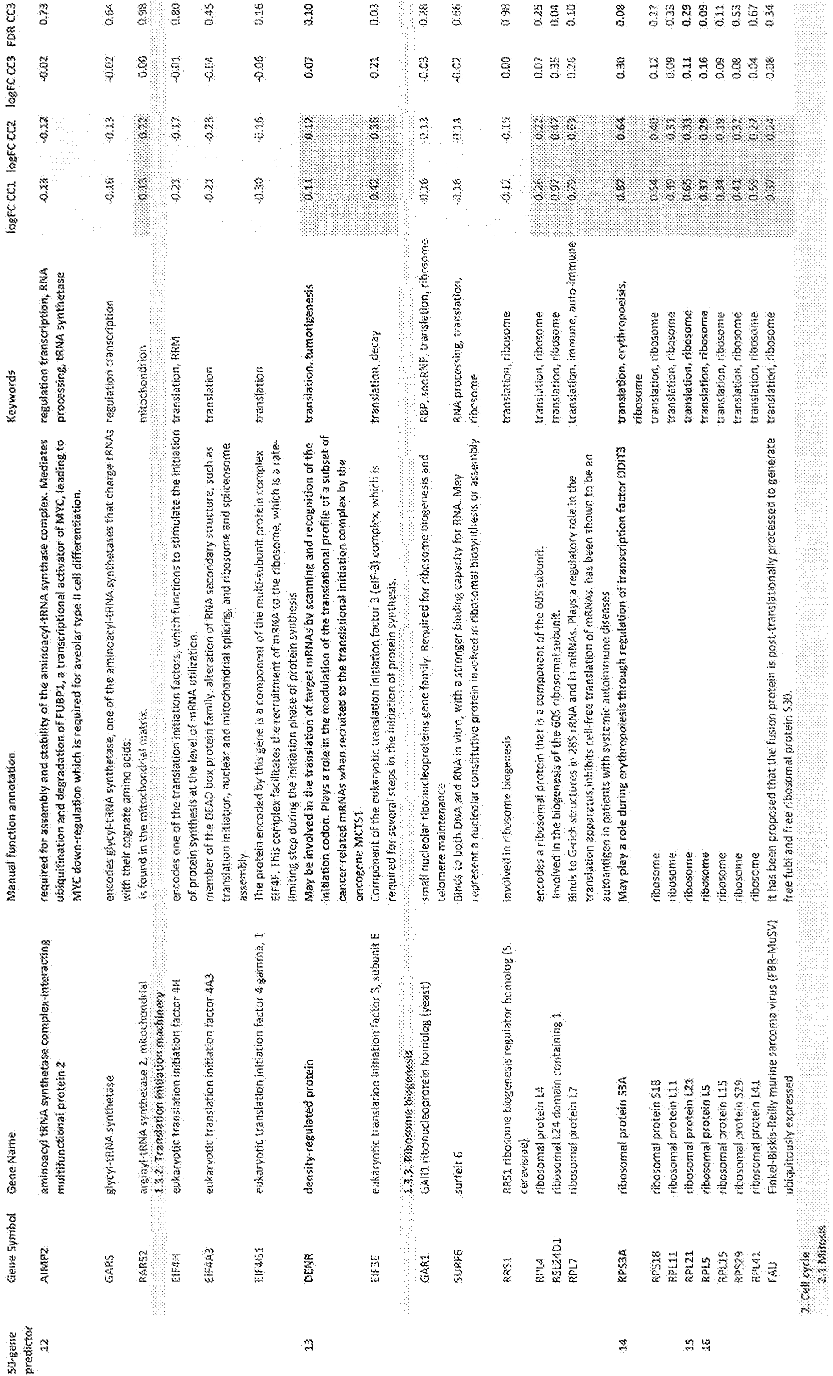

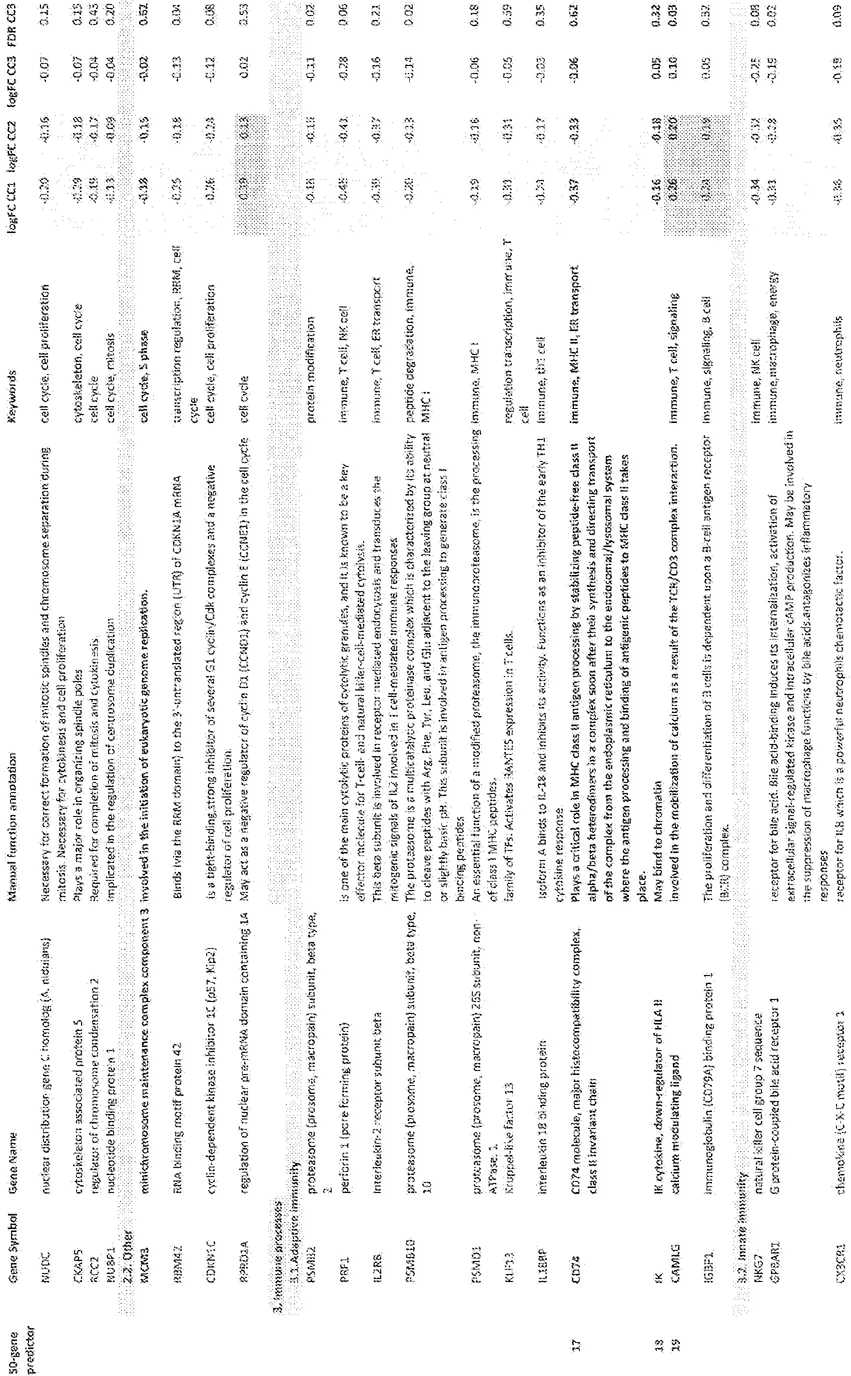

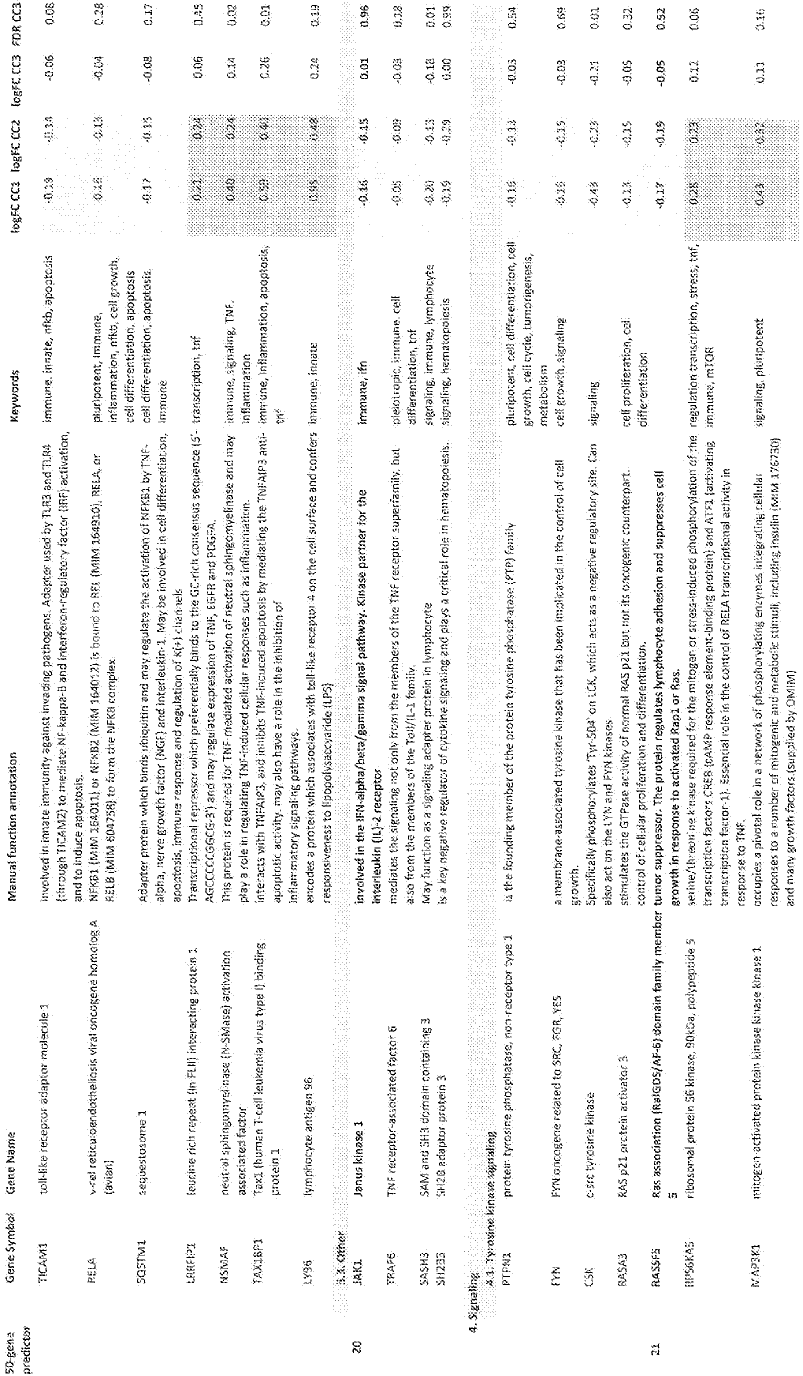

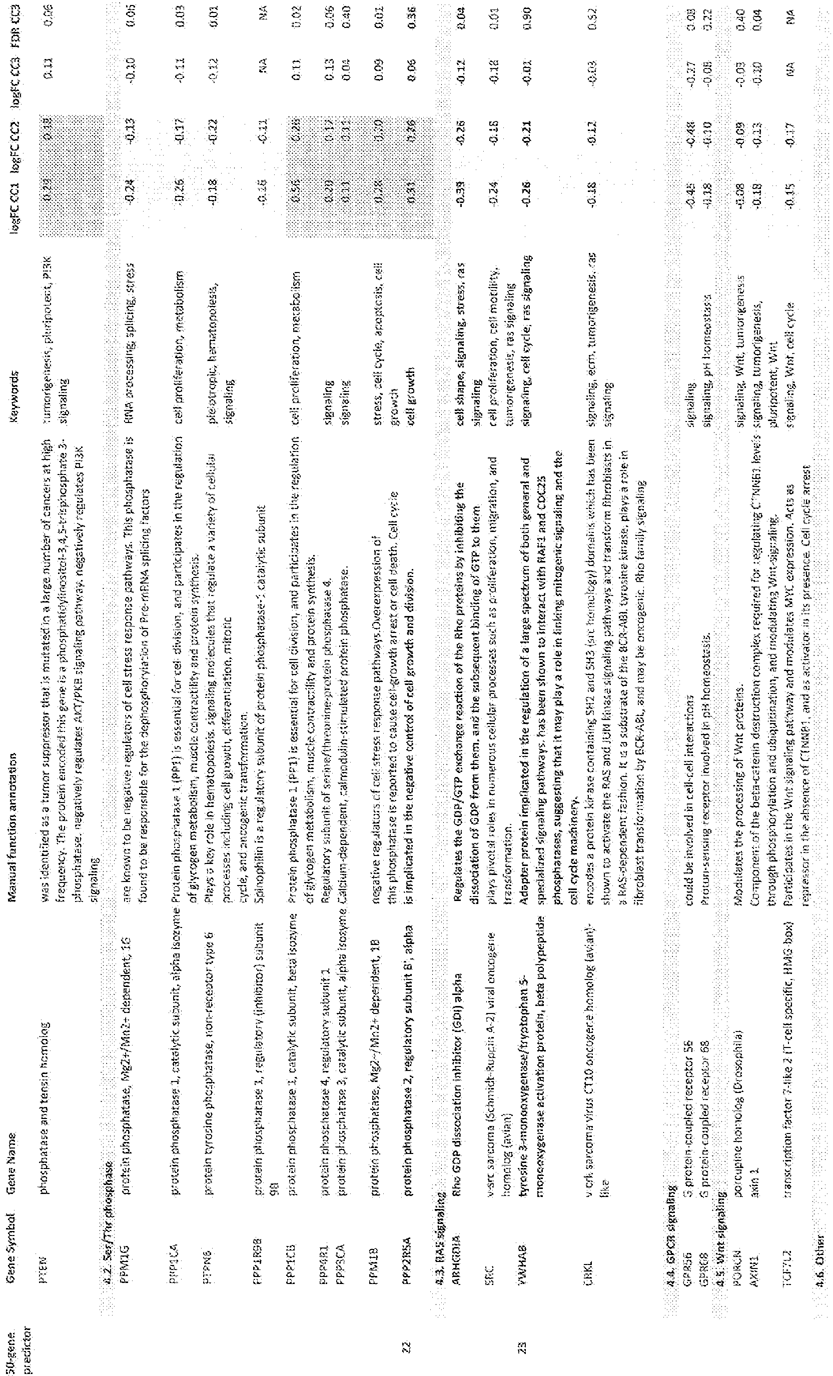

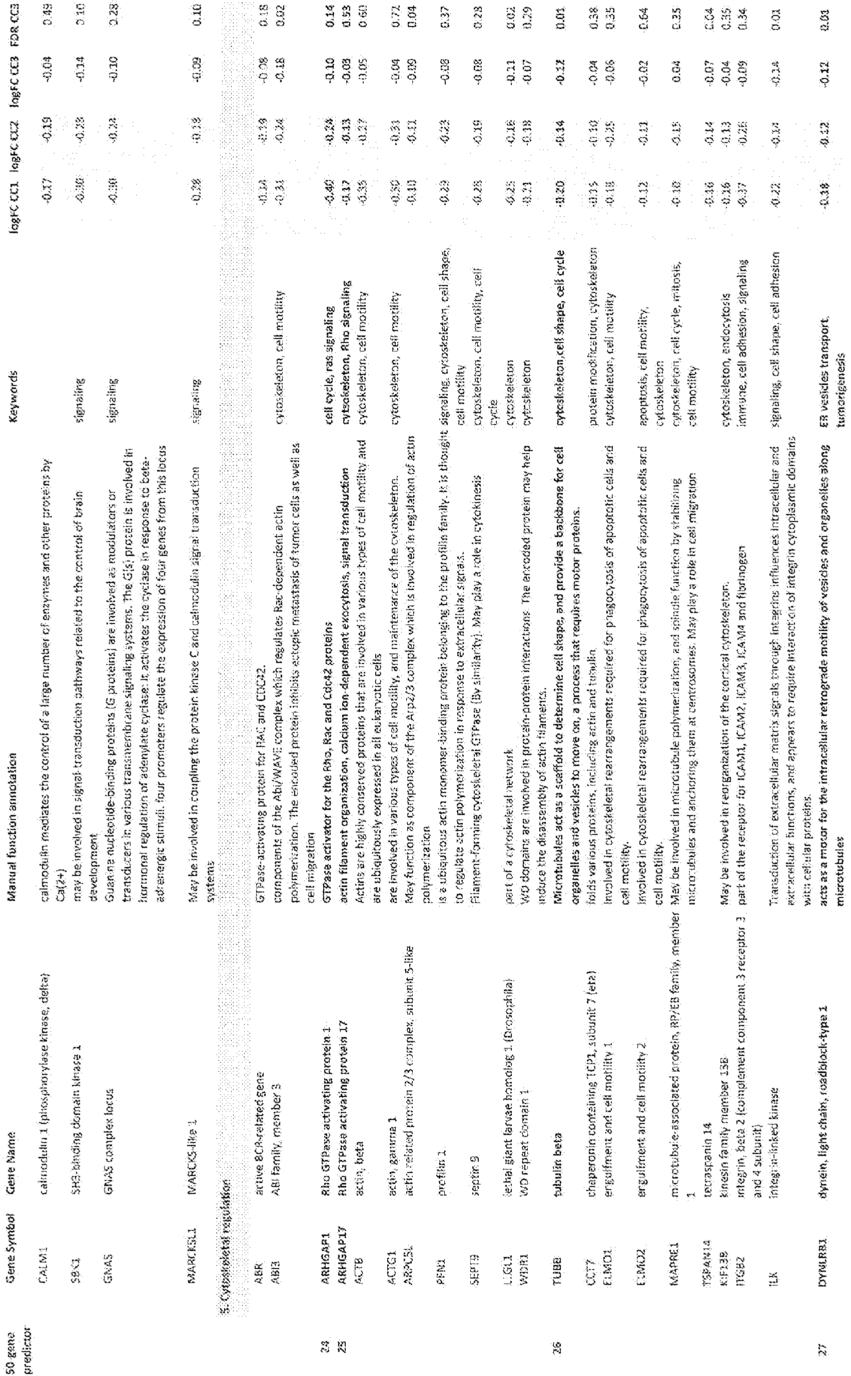

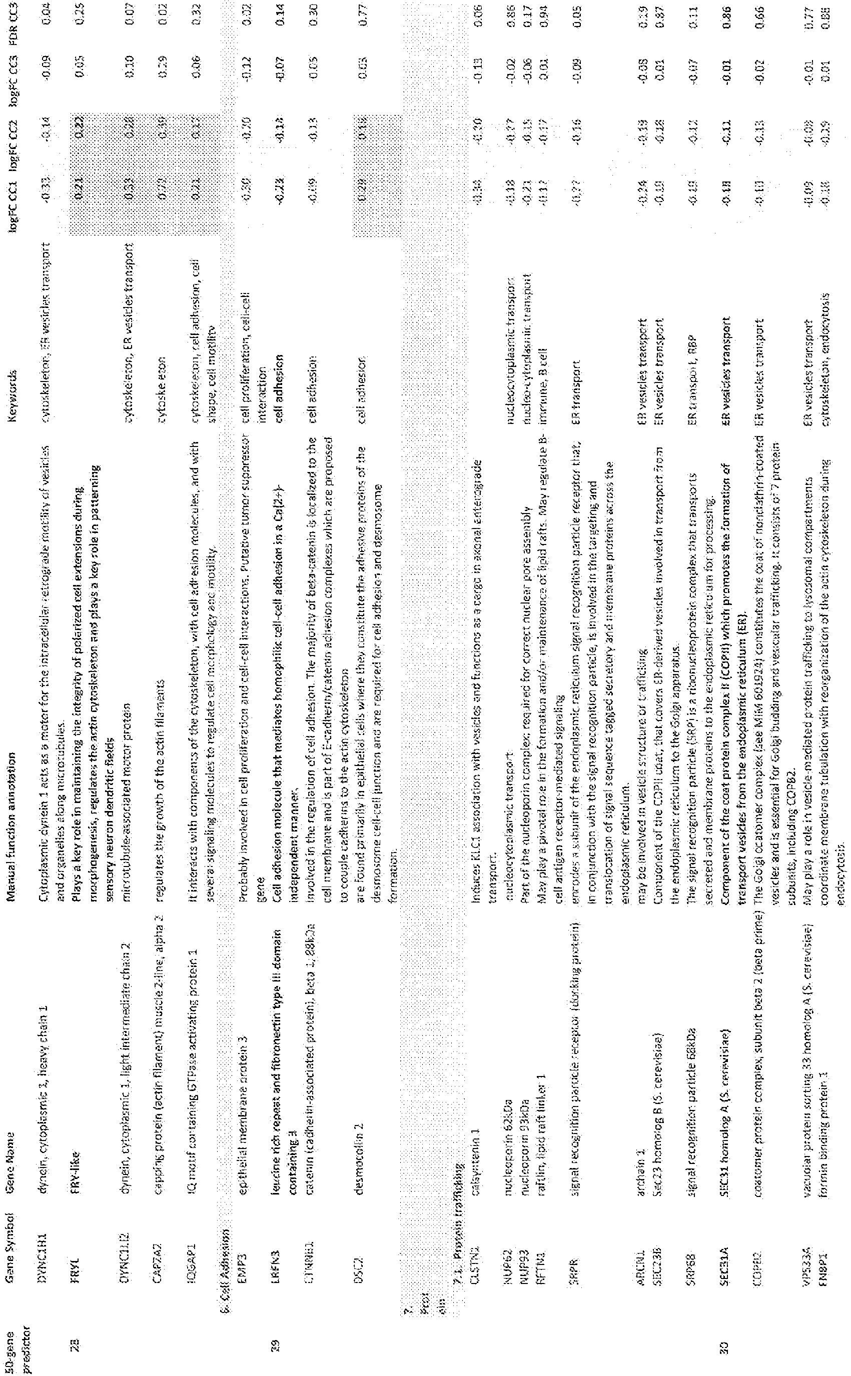

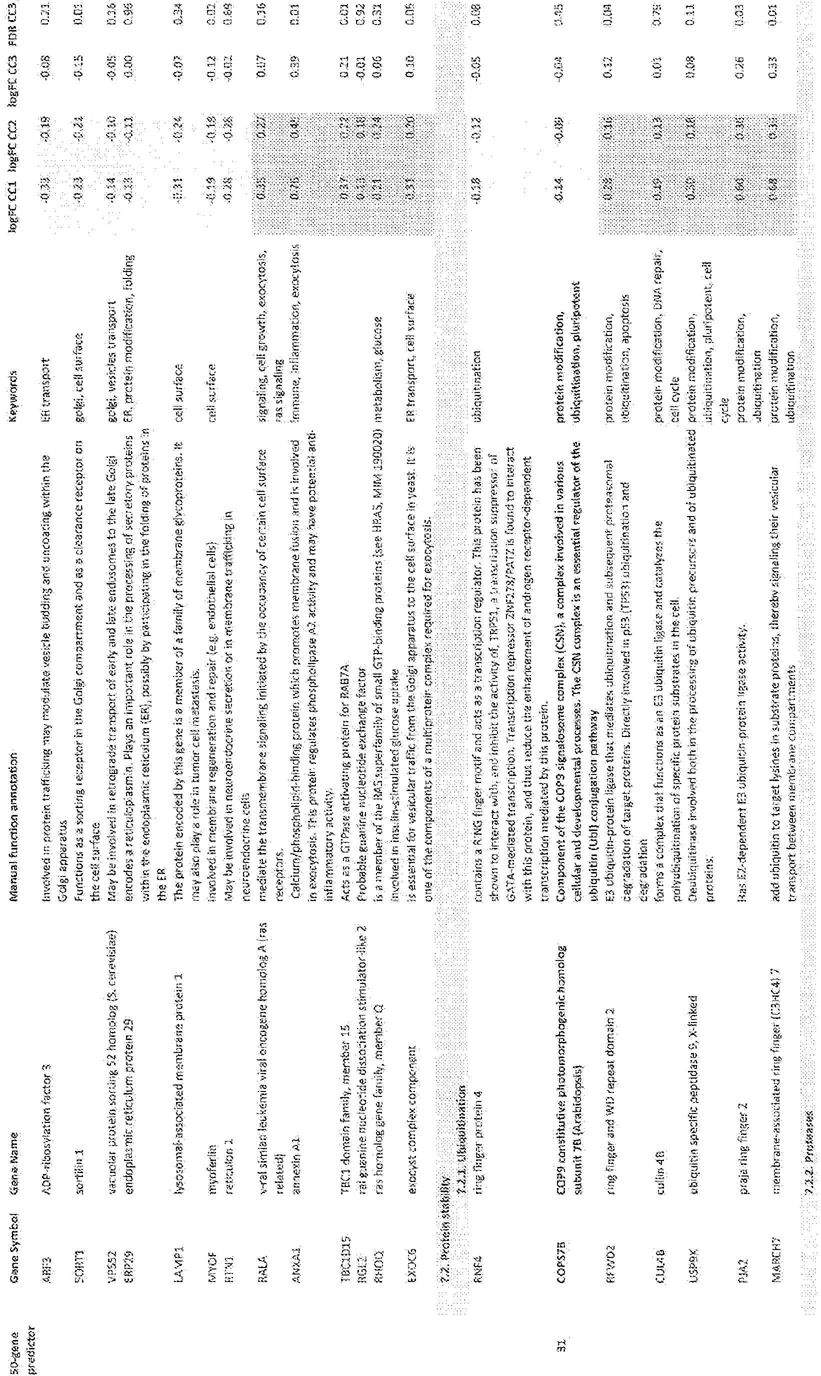

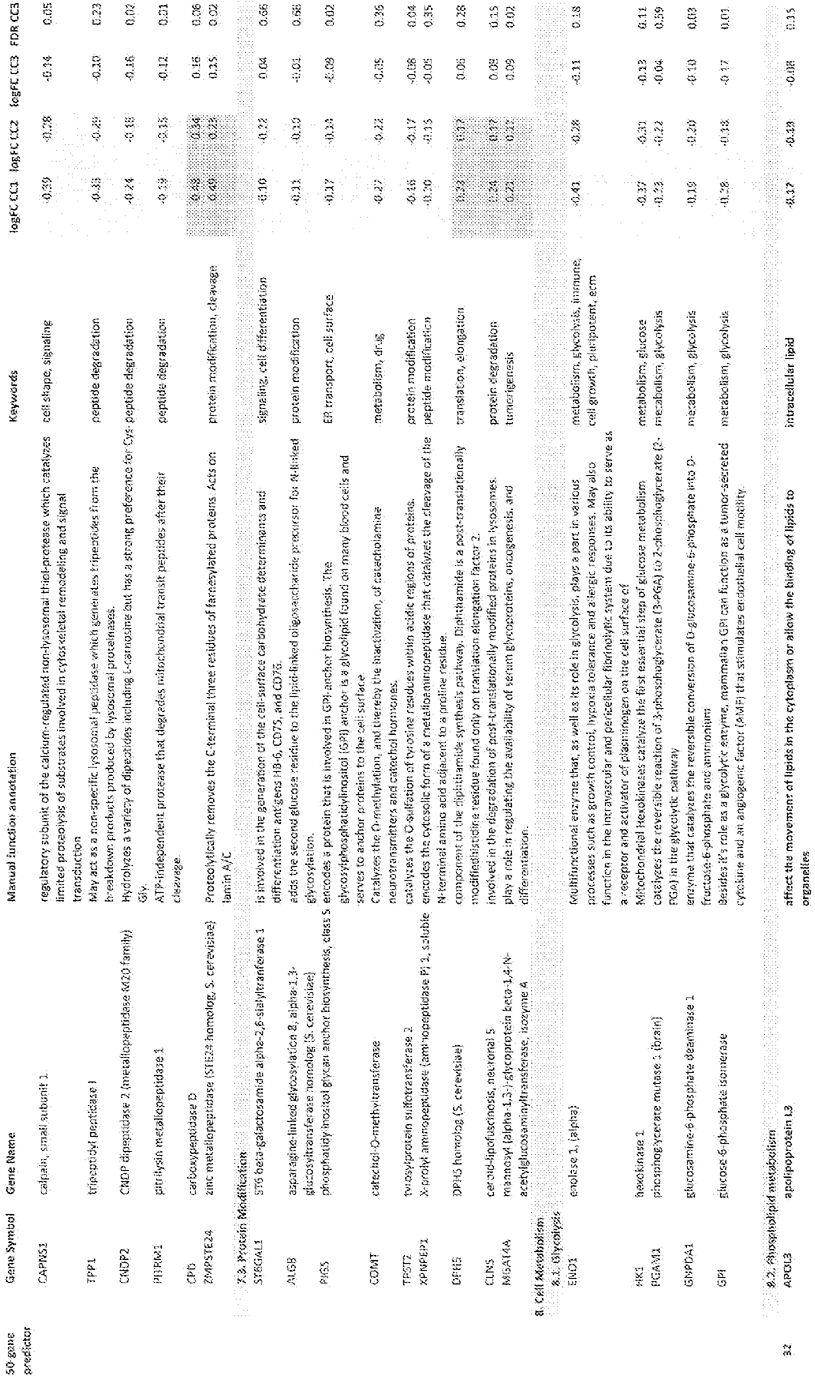

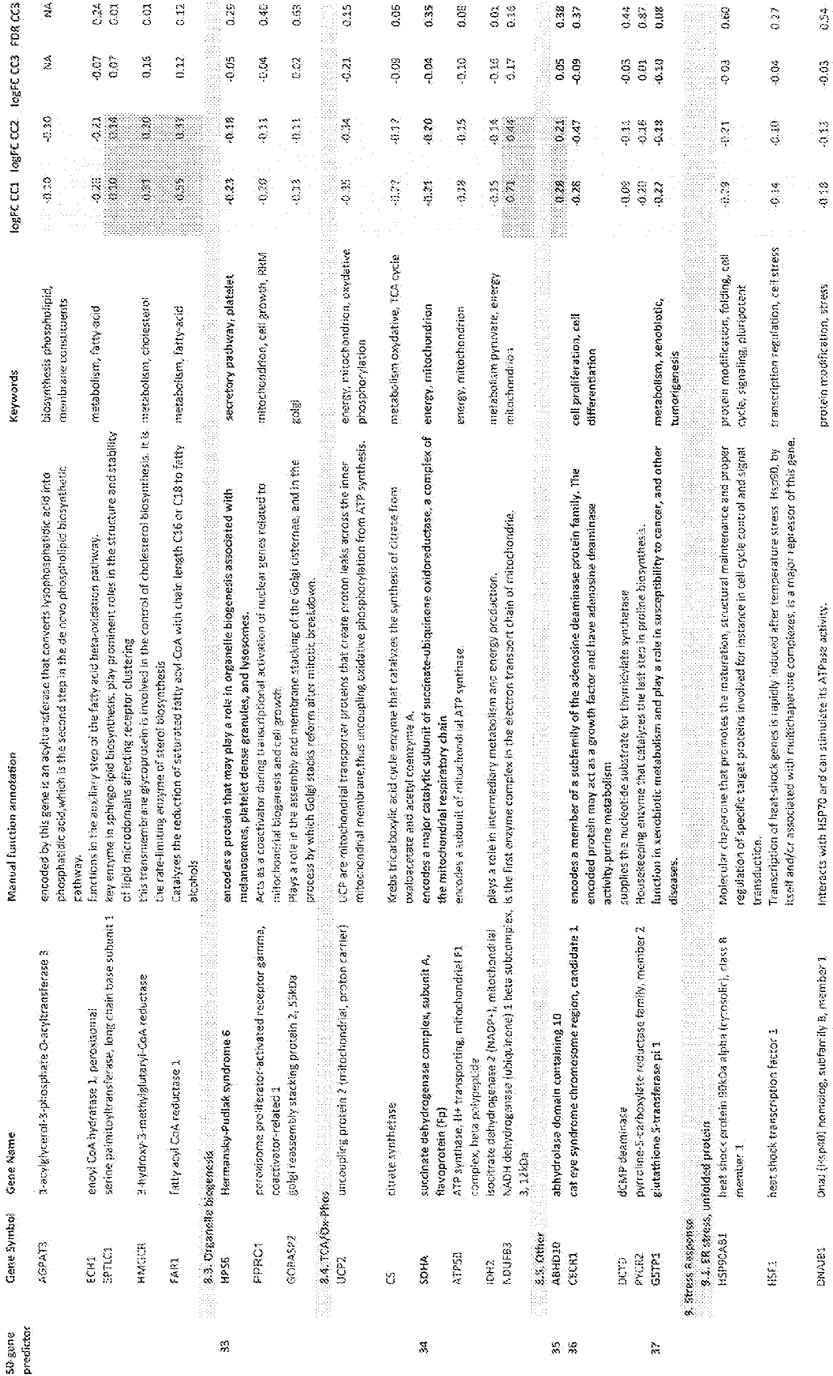

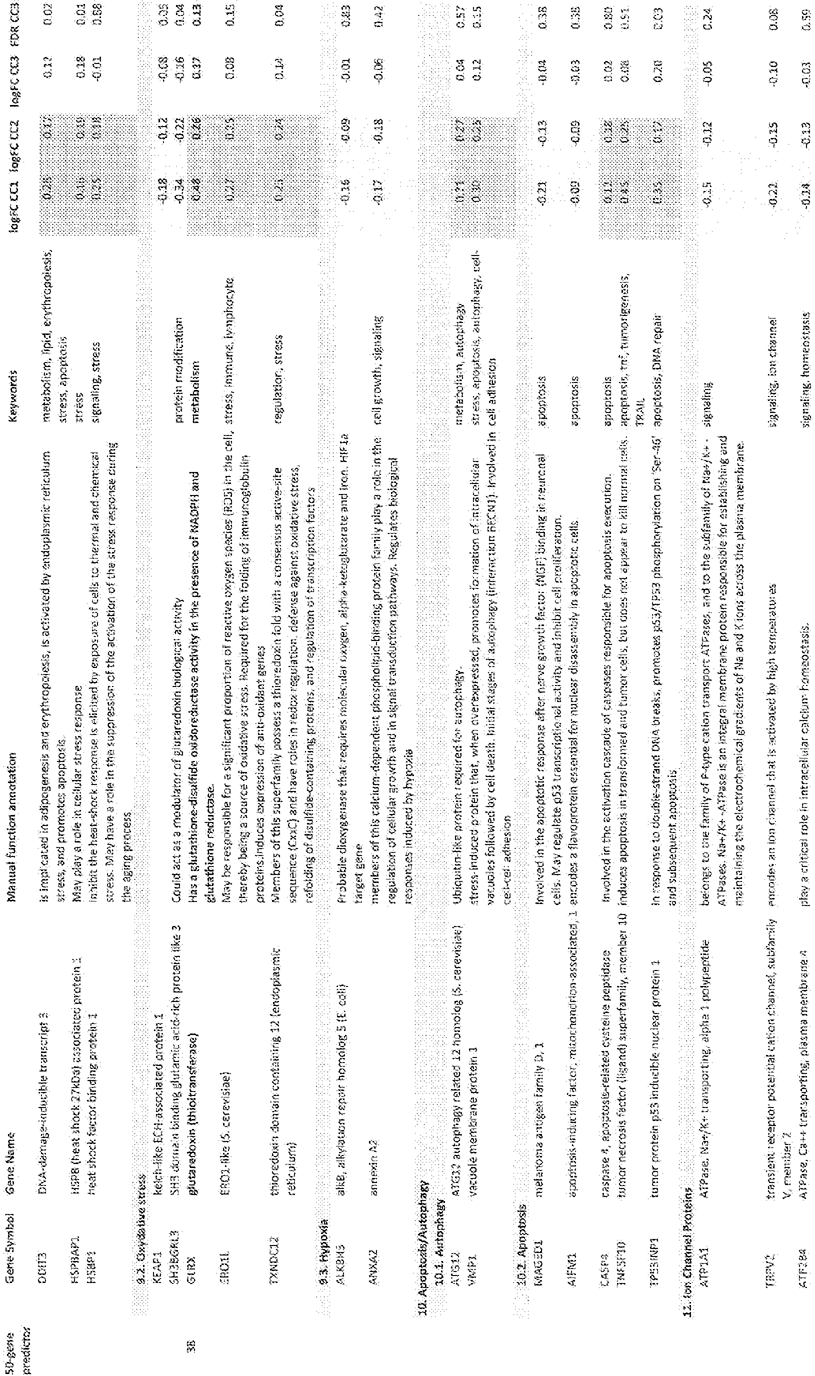

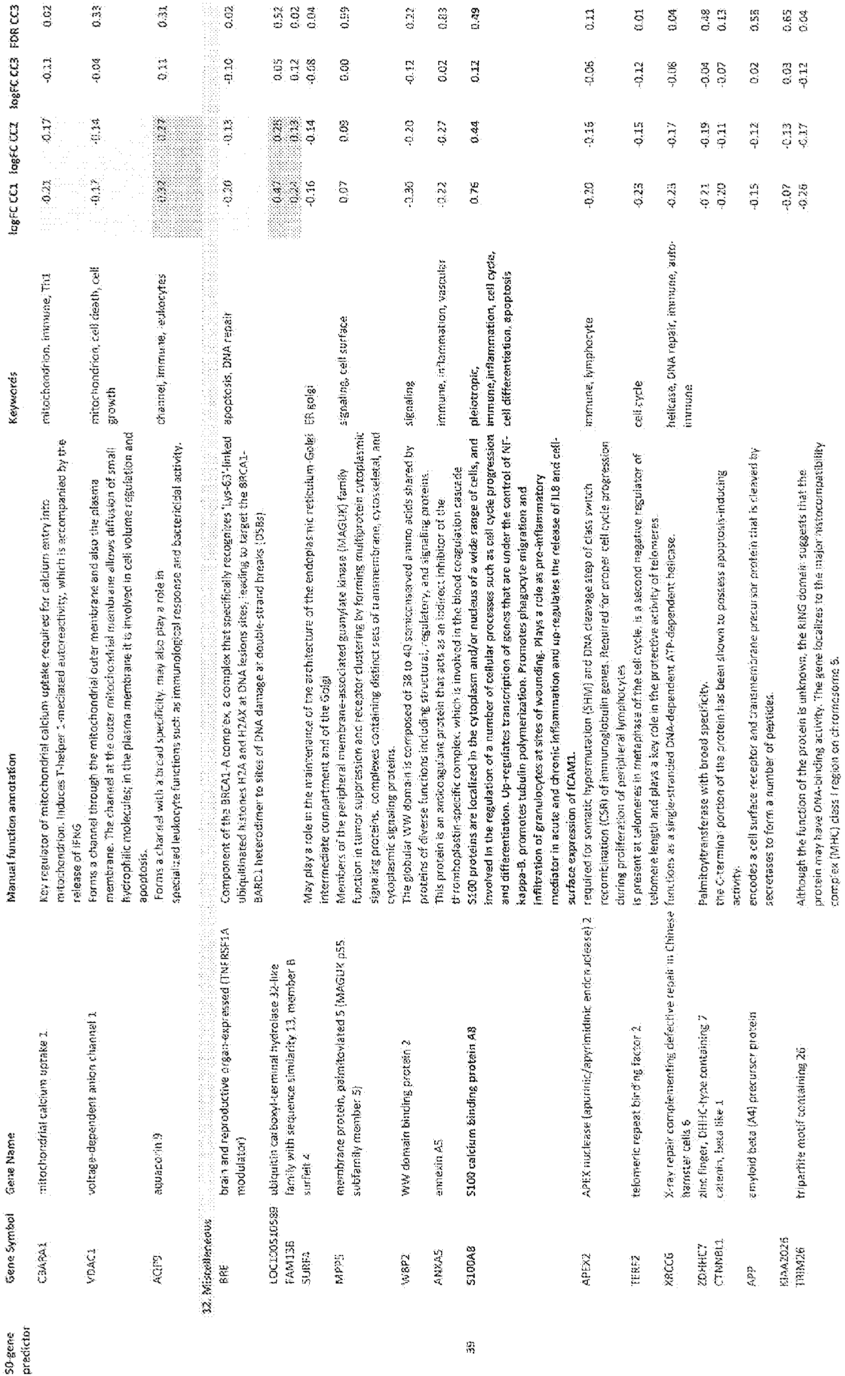

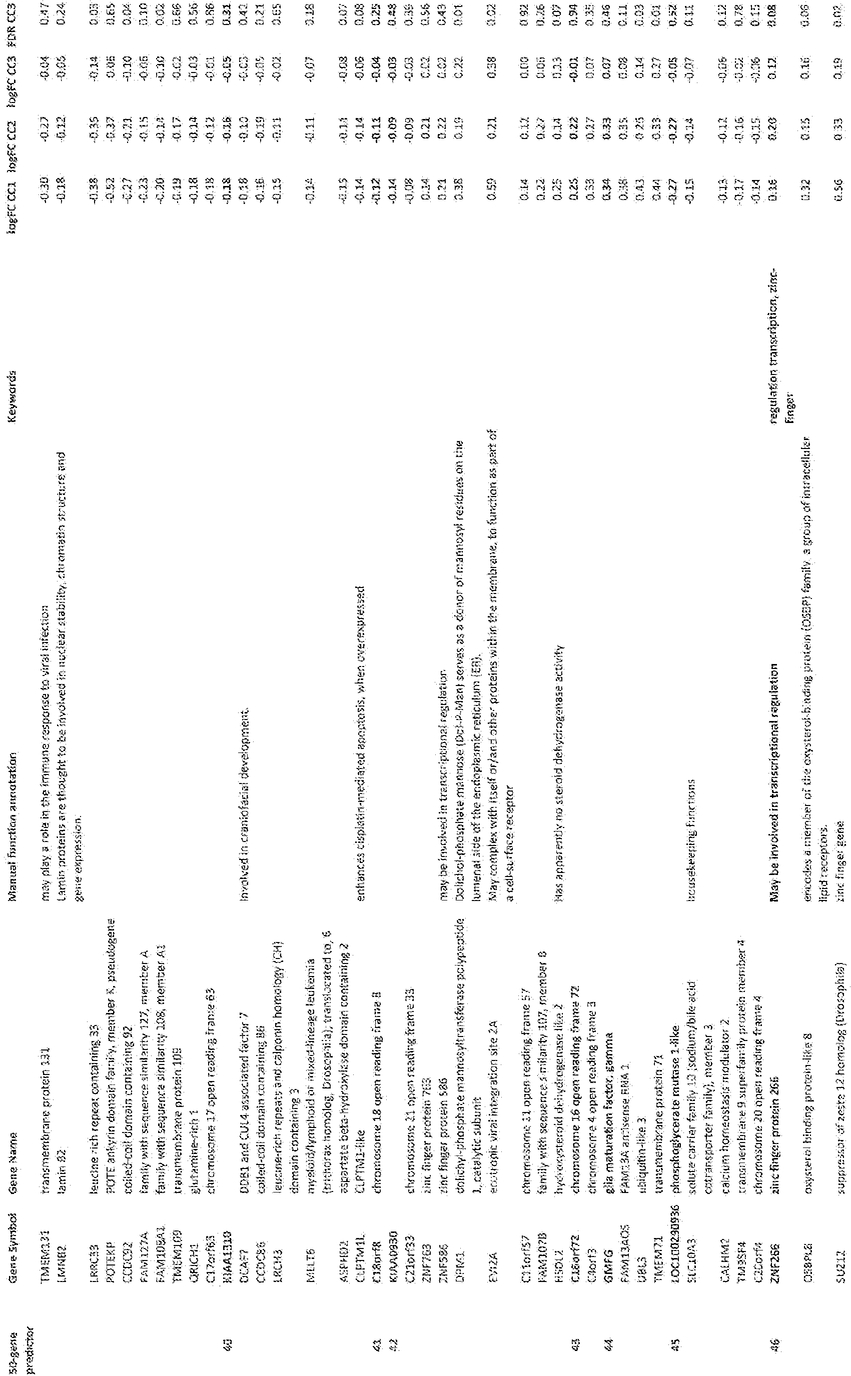

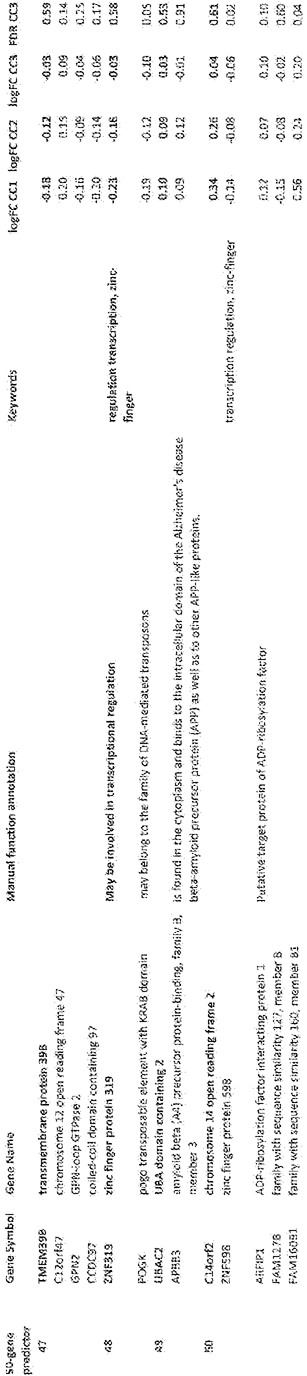

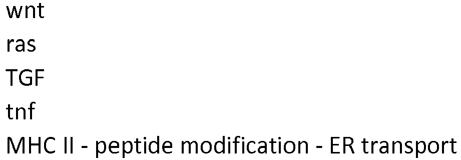

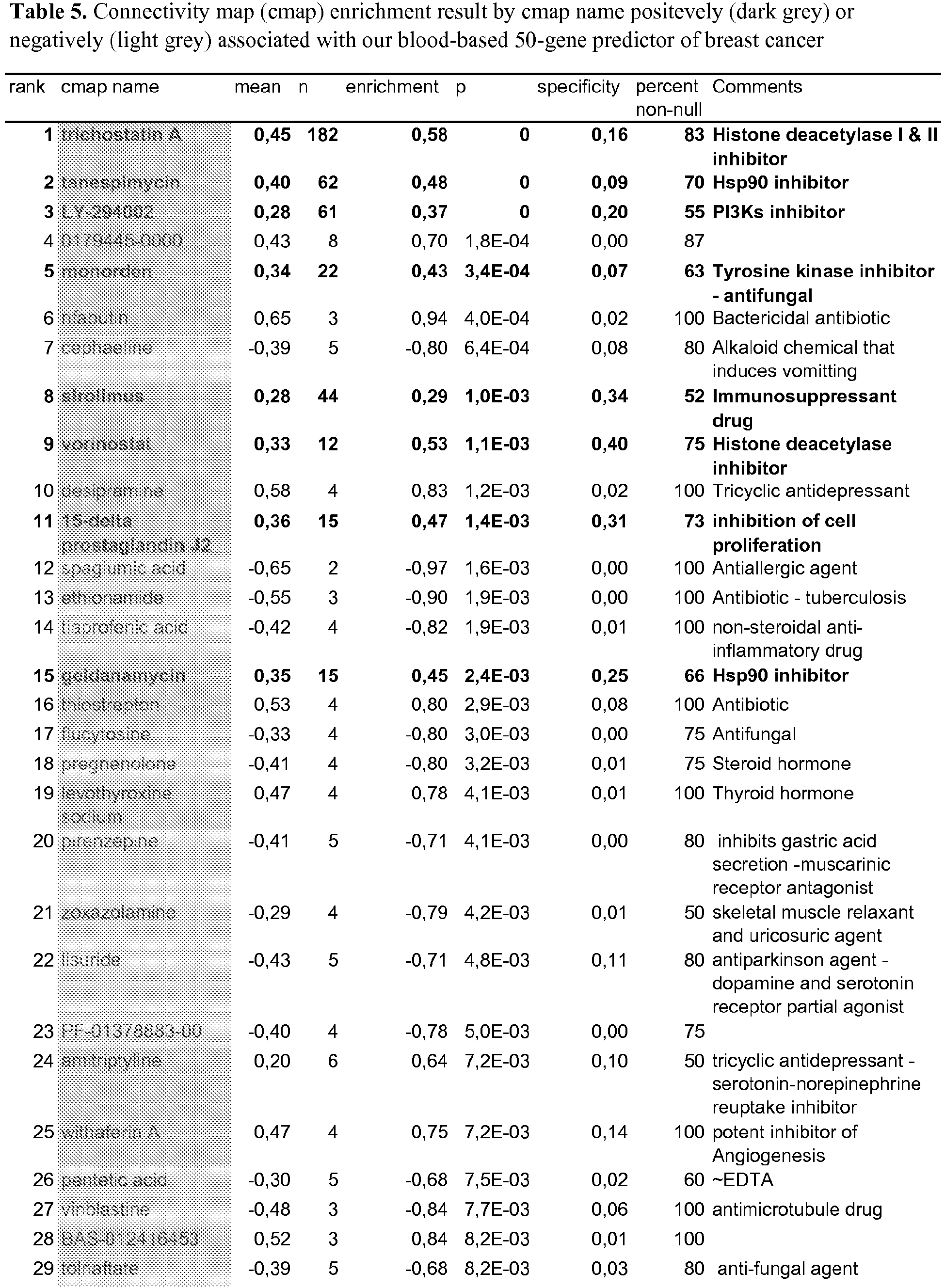

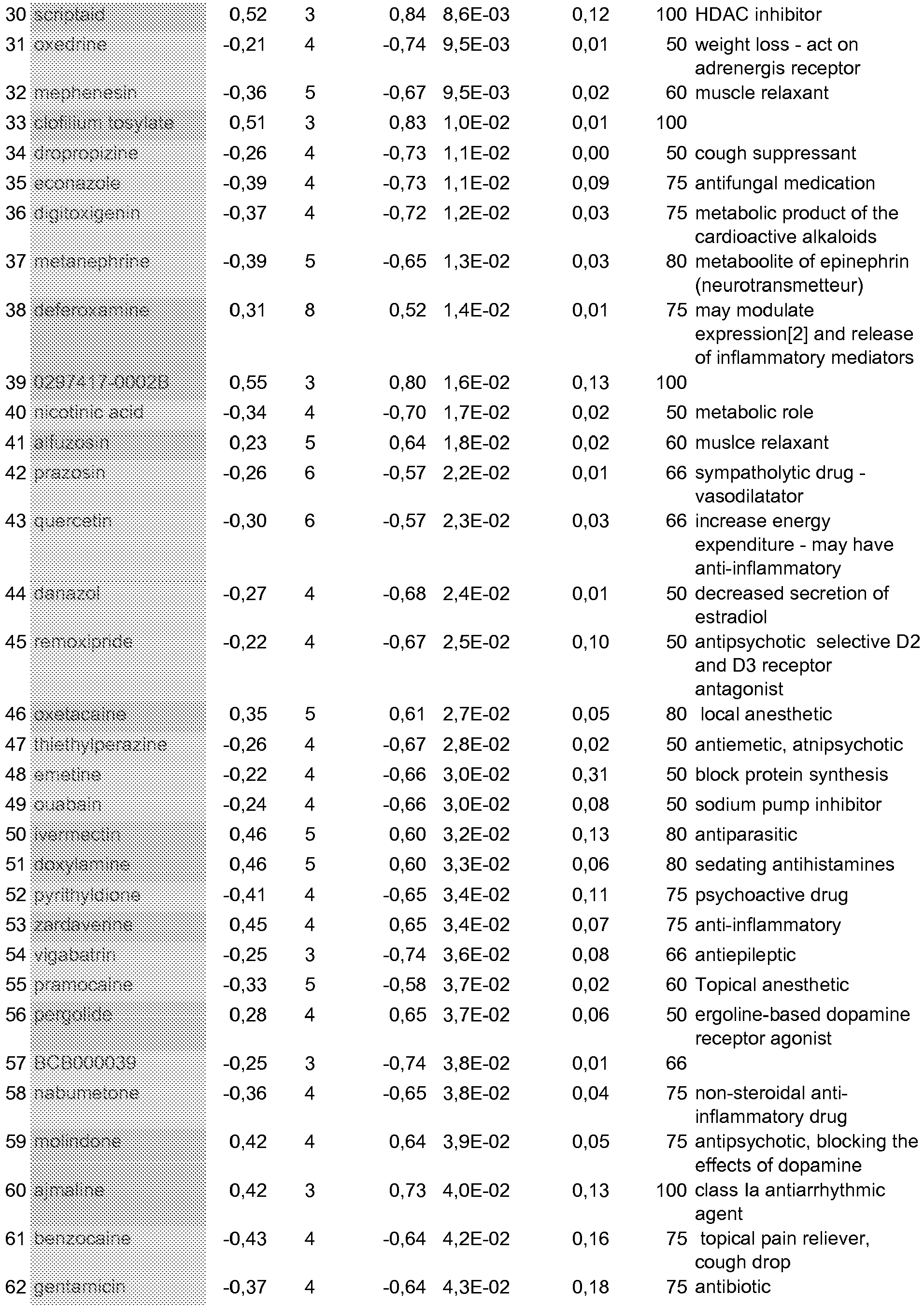

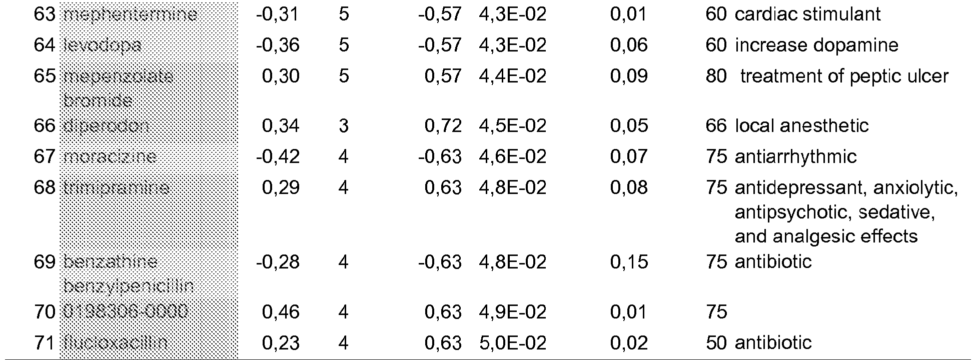

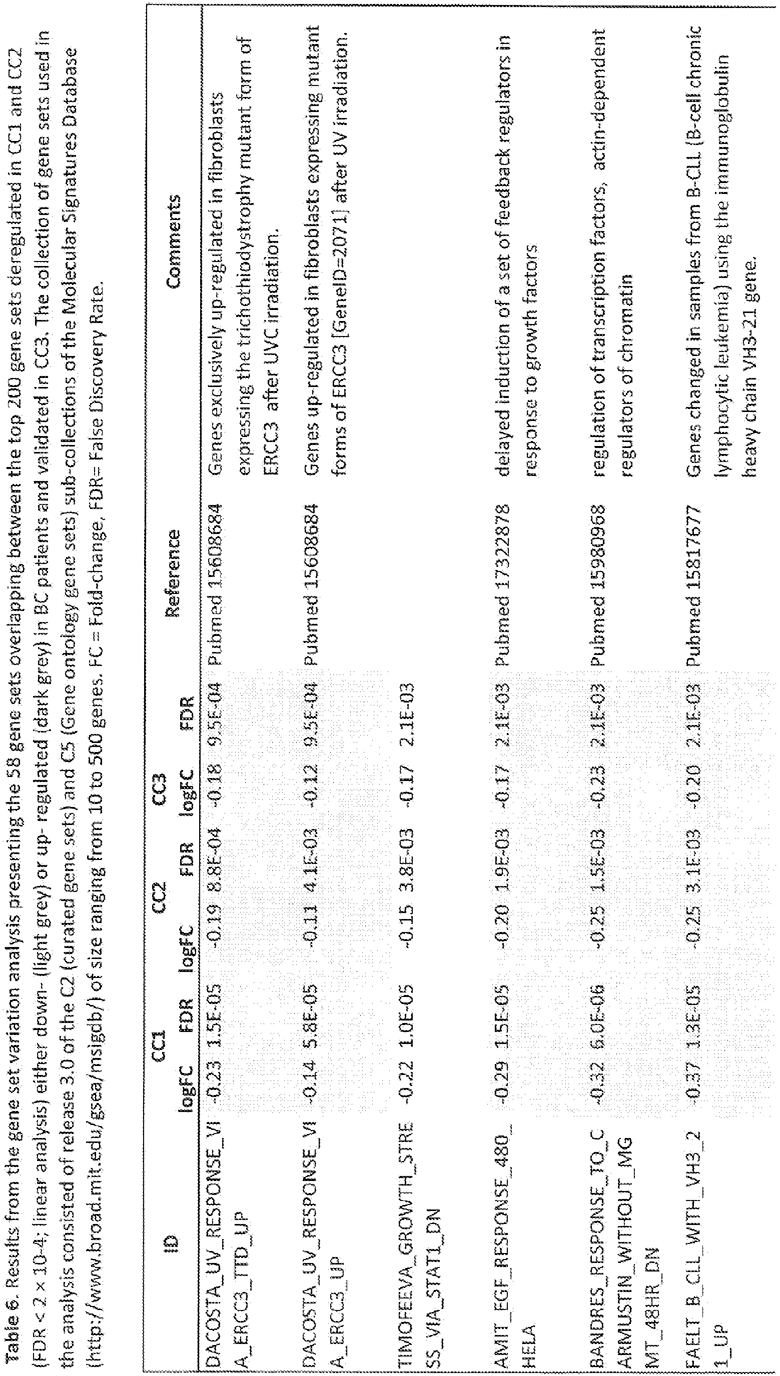

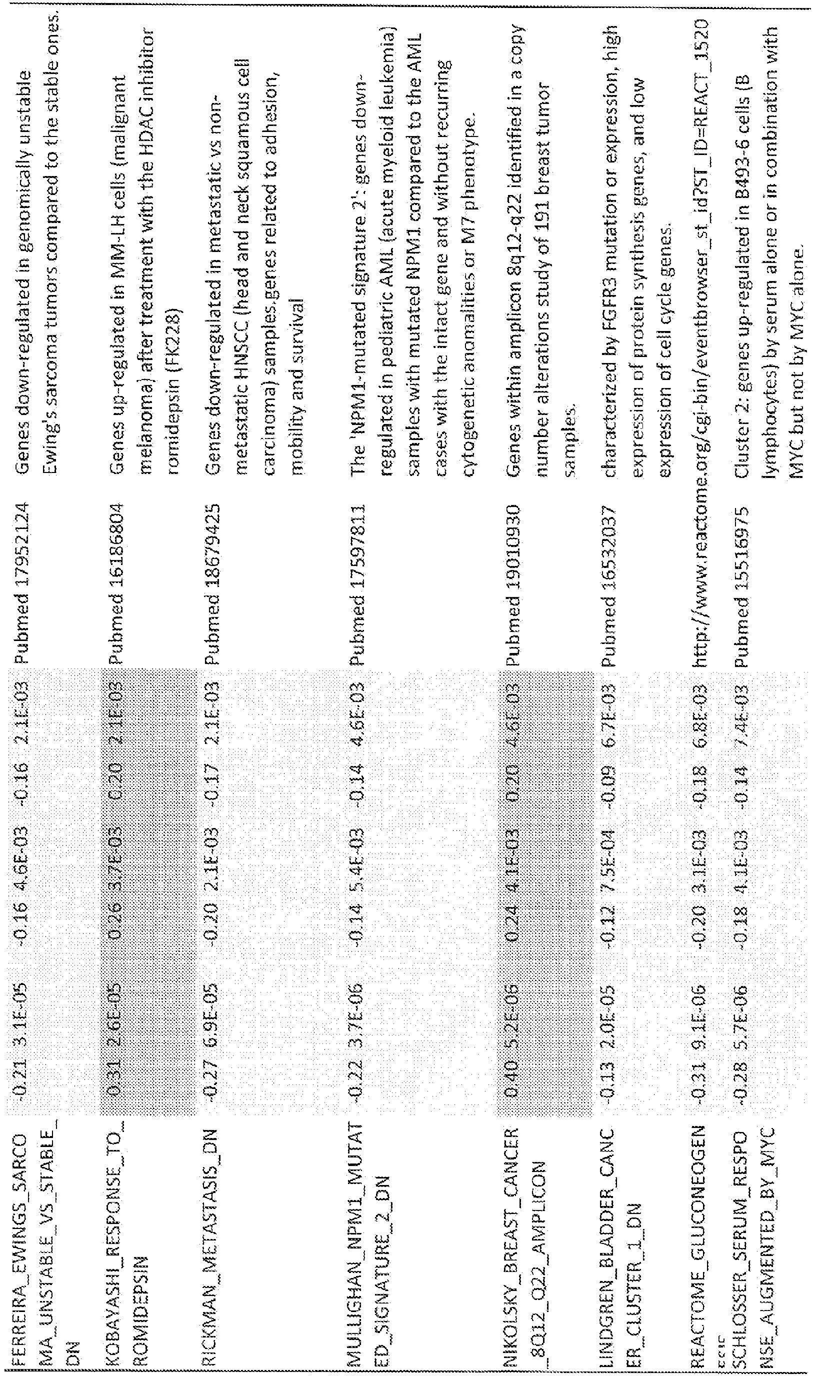

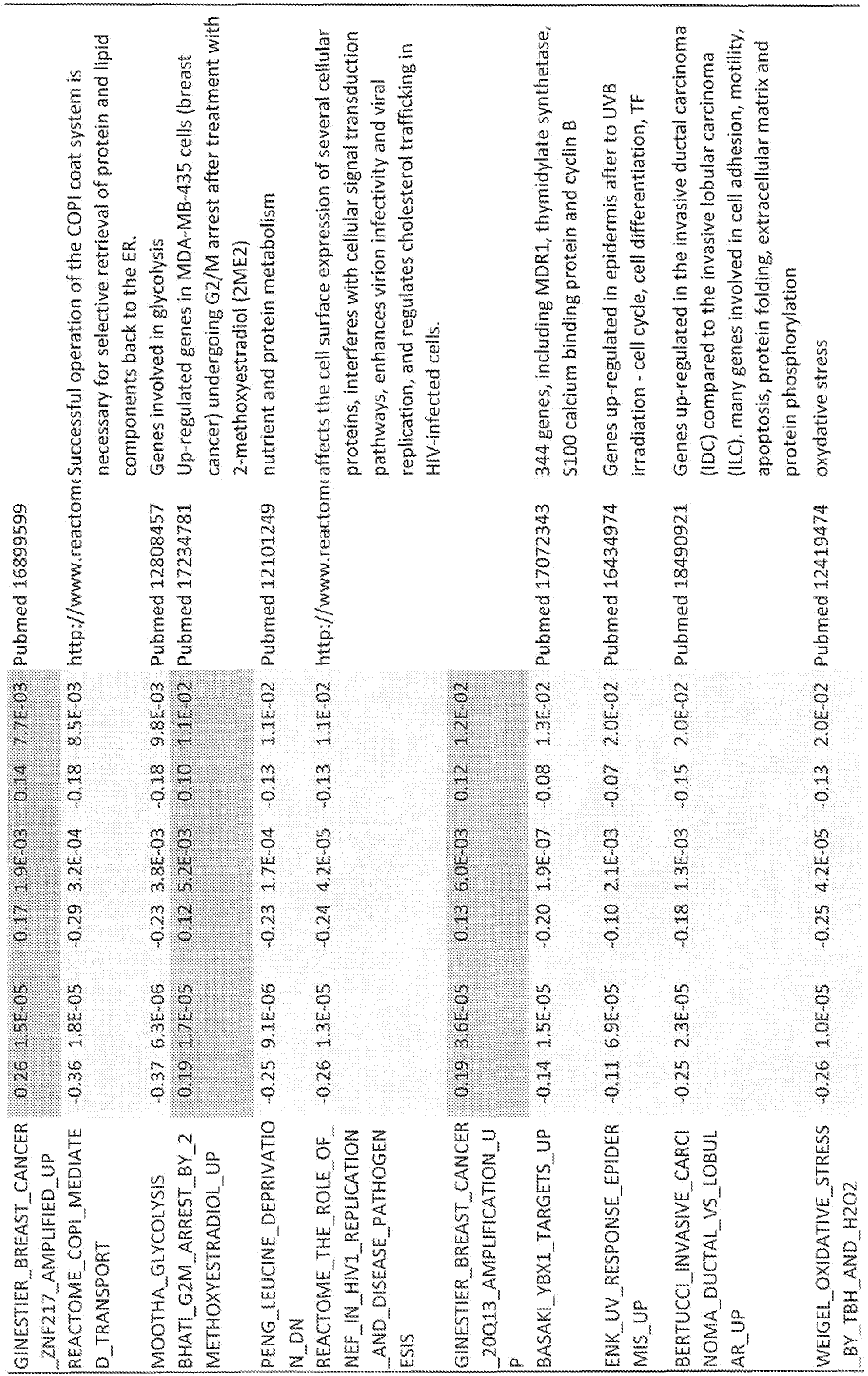

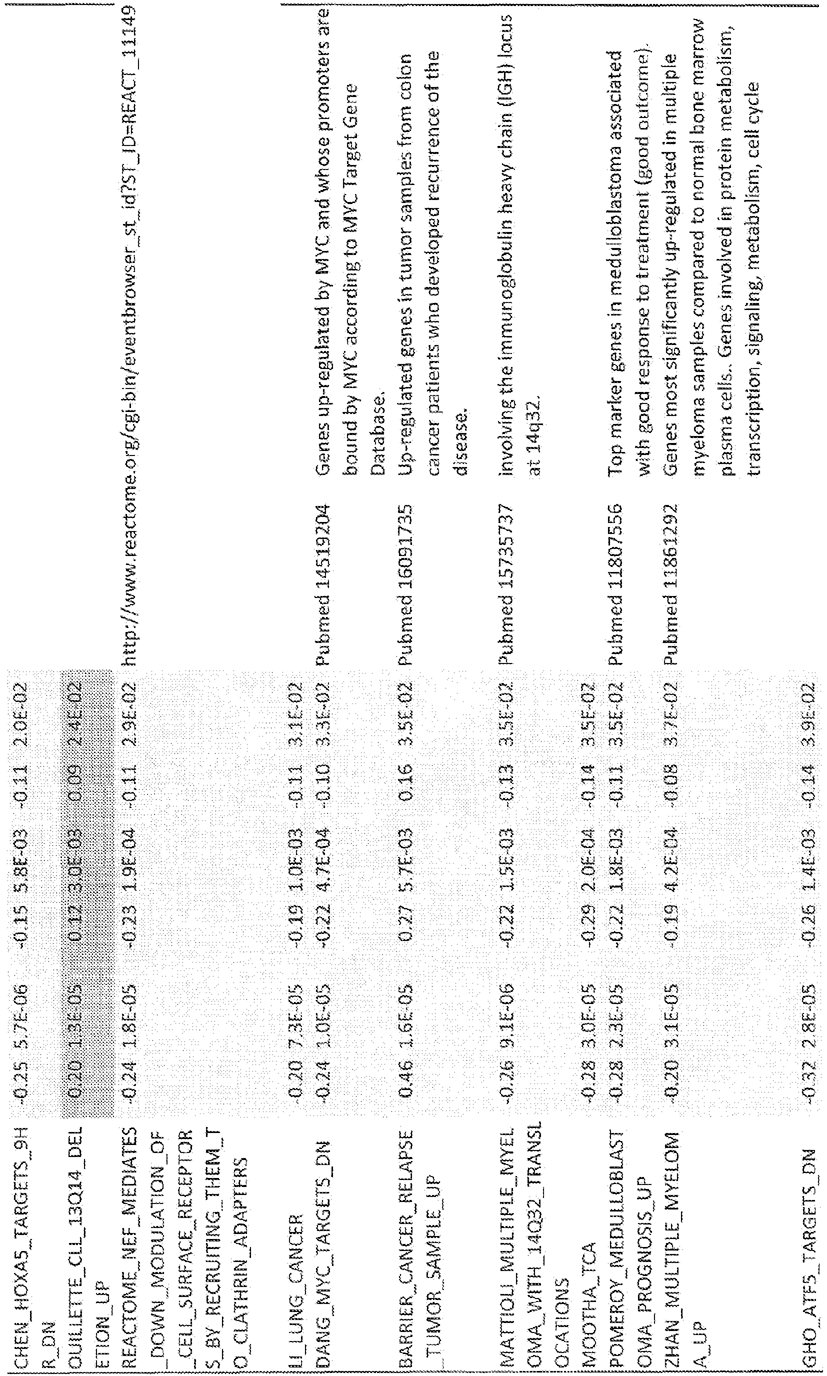

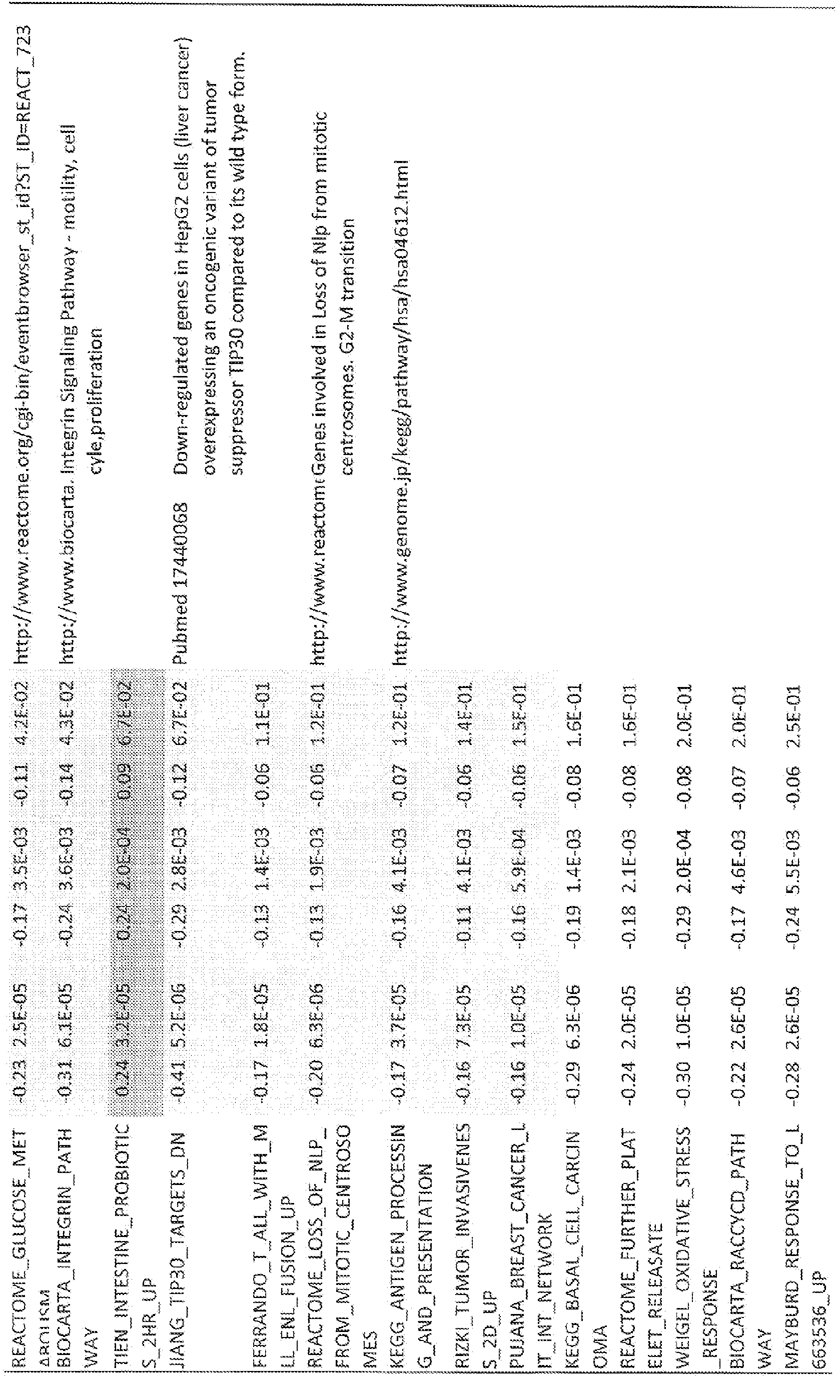

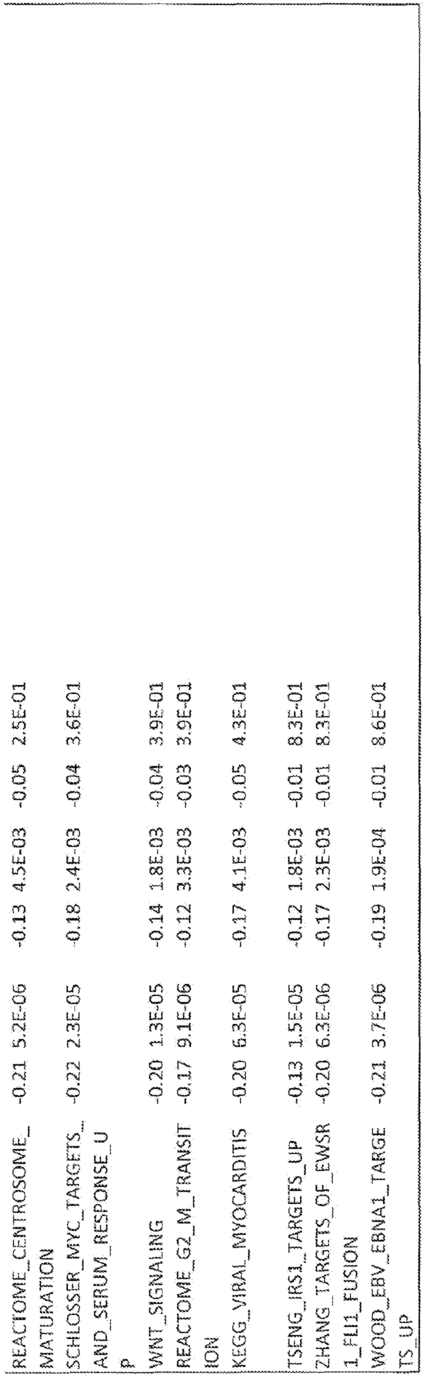

**Table 7.** Gene symbols and names included in set of cognate mRNAs modulated by both T cell receptor loci in tumor-infiltrating lymphocytes published in Curtis *et al*. (2012)

| **Gene Symbol** | **Gene Name** |
|---|---|
| AMICA1 | Adhesion molecule interacting with CXADR antigen |
| APOBEC3G | apolipoprotein B mRNA editing enzyme, catalytic polypeptide-like 3G |
| B2M | beta-2-microglobulin |
| BCAR1 | breast cancer anti-estrogen resistance 1 |
| BLK | B lymphoid tyrosine kinase |
| BTK | Bruton agammaglobulinemia tyrosine kinase |
| CCL17 | chemokine (C-C motif) ligand 17 |
| CCL19 | chemokine (C-C motif) ligand 19 |
| CCL21 | chemokine (C-C motif) ligand 21 |
| CCL22 | chemokine (C-C motif) ligand 22 |
| CCL5 | chemokine (C-C motif) ligand 5 |
| CCR4 | chemokine (C-C motif) receptor 4 |
| CCR6 | chemokine (C-C motif) receptor 6 |
| CCR7 | chemokine (C-C motif) receptor 7 |
| CD19 | CD19 molecule |
| CD226 | CD226 molecule |
| CD247 | CD247 molecule |
| CD27 | CD27 molecule |
| CD3D | CD3d molecule, delta (CD3-TCR complex) |
| CD3E | CD3e molecule, epsilon (CD3-TCR complex) |
| CD3G | CD3g molecule, gamma (CD3-TCR complex) |
| CD4 | CD4 molecule |
| CD74 | CD74 molecule, major histocompatibility complex, class II invariant chain |
| CD80 | CD80 molecule |
| CD86 | CD86 molecule |
| CD8A | CD8a molecule |
| CD8B | CD8b molecule |
| CXCL10 | chemokine (C-X-C motif) ligand 10 |
| CXCL13 | chemokine (C-X-C motif) ligand 13 |
| CXCL9 | chemokine (C-X-C motif) ligand 9 |
| CXCR3 | In multiple Geneids |
| CXCR5 | chemokine (C-X-C motif) receptor 5 |
| CXCR6 | chemokine (C-X-C motif) receptor 6 |
| DGKA | diacylglycerol kinase, alpha 80kDa |
| FCER1G | Fc fragment of IgE, high affinity I, receptor for; gamma polypeptide |
| FGR | Gardner-Rasheed feline sarcoma viral (v-fgr) oncogene homolog |
| FYB | FYN binding protein |
| FYN | FYN oncogene related to SRC, FGR, YES |
| GAB3 | GRB2-associated binding protein 3 |
| GNG2 | guanine nucleotide binding protein (G protein), gamma 2 |
| GNGT2 | guanine nucleotide binding protein (G protein), gamma transducing activity polypeptide 2 |
| GRAP | GRB2-related adaptor protein |
| GRAP2 | GRB2-related adaptor protein 2 |
| GZMA | granzyme A (granzyme 1, cytotoxic T-lymphocyte-associated serine esterase 3) |
| GZMB | granzyme B (granzyme 2, cytotoxic T-lymphocyte-associated serine esterase 1) |
| HCST | hematopoietic cell signal transducer |
| HLA-A | major histocompatibility complex, class I, A |
| HLA-DMA | major histocompatibility complex, class II, DM alpha |
| HLA-DMB | major histocompatibility complex, class II, DM beta |
| HLA-DOB | major histocompatibility complex, class II, DO beta |
| HLA-DPA1 | major histocompatibility complex, class II, DP alpha 1 |
| HLA-DPB1 | major histocompatibility complex, class II, DP beta 1 |
| HLA-DRA | major histocompatibility complex, class II, DR alpha |
| HLA-DRB3 | major histocompatibility complex, class II, DR beta 3 |
| HLA-F | major histocompatibility complex, class I, F |
| HLA-G | major histocompatibility complex, class I, G |
| IFNG | interferon, gamma |
| IL12RB1 | interleukin 12 receptor, beta 1 |
| IL15 | interleukin 15 |
| IL18R1 | interleukin 18 receptor 1 |
| IL3RA | interleukin 3 receptor, alpha (low affinity) |
| IL7R | interleukin 7 receptor |
| IRF9 | interferon regulatory factor 9 |
| ITGA4 | integrin, alpha 4 (antigen CD49D, alpha 4 subunit of VLA-4 receptor) |
| ITGAX | integrin, alpha X (complement component 3 receptor 4 subunit) |
| ITGB2 | integrin, beta 2 (complement component 3 receptor 3 and 4 subunit) |
| ITK | IL2-inducible T-cell kinase |
| KIR2DL4 | killer cell immunoglobulin-like receptor, two domains, long cytoplasmic tail, 4 |
| KIR3DL1 | killer cell immunoglobulin-like receptor, three domains, long cytoplasmic tail, 1 |
| KLRC1 | killer cell lectin-like receptor subfamily C, member 1 |
| KLRC3 | killer cell lectin-like receptor subfamily C, member 3 |
| KLRD1 | killer cell lectin-like receptor subfamily D, member 1 |
| LAT | linker for activation of T cells |
| LAT2 | linker for activation of T cells family, member 2 |
| LCK | lymphocyte-specific protein tyrosine kinase |
| LCP2 | lymphocyte cytosolic protein 2 (SH2 domain containing leukocyte protein of 76kDa) |
| LTA | lymphotoxin alpha (TNF superfamily, member 1) |
| LYN | v-yes-1 Yamaguchi sarcoma viral related oncogene homolog |
| MAPK1 | mitogen-activated protein kinase 1 |
| NCK1 | NCK adaptor protein 1 |
| NCR3 | natural cytotoxicity triggering receptor 3 |
| NFATC3 | nuclear factor of activated T-cells, cytoplasmic, calcineurin-dependent 3 |
| PAG1 | phosphoprotein associated with glycosphingolipid microdomains 1 |
| PLCB2 | phospholipase C, beta 2 |
| PNOC | prepronociceptin |
| PRF1 | perforin 1 (pore forming protein) |
| PSMB10 | proteasome (prosome, macropain) subunit, beta type, 10 |
| PSMB8 | proteasome (prosome, macropain) subunit, beta type, 8 (large multifunctional peptidase 7) |
| PSMB9 | proteasome (prosome, macropain) subunit, beta type, 9 (large multifunctional peptidase 2) |
| PSME1 | proteasome (prosome, macropain) activator subunit 1 (PA28 alpha) |
| PTK2B | In multiple Geneids |
| PTPN2 | protein tyrosine phosphatase, non-receptor type 2 |
| PTPN7 | protein tyrosine phosphatase, non-receptor type 7 |
| PTPRC | protein tyrosine phosphatase, receptor type, C |
| SH2D2A | SH2 domain containing 2A |
| SLA2 | Src-like-adaptor 2 |
| SOCS1 | suppressor of cytokine signaling 1 |
| STAT1 | signal transducer and activator of transcription 1, 91kDa |
| STAT4 | signal transducer and activator of transcription 4 |
| SYK | spleen tyrosine kinase |
| TAP1 | transporter 1, ATP-binding cassette, sub-family B (MDR/TAP) |
| TAPBP | TAP binding protein (tapasin) |
| TNFRSF13B | tumor necrosis factor receptor superfamily, member 13B |
| TNFRSF17 | tumor necrosis factor receptor superfamily, member 17 |
| TNFRSF4 | tumor necrosis factor receptor superfamily, member 4 |
| TNFRSF9 | tumor necrosis factor receptor superfamily, member 9 |
| TNFSF13B | tumor necrosis factor (ligand) superfamily, member 13b |
| TNFSF14 | tumor necrosis factor (ligand) superfamily, member 14 |
| TRAT1 | T cell receptor associated transmembrane adaptor 1 |
| TYROBP | TYRO protein tyrosine kinase binding protein |
| VAV1 | vav 1 guanine nucleotide exchange factor |
| VCAM1 | vascular cell adhesion molecule 1 |
| WIPF1 | WAS/WASL interacting protein family, member 1 |
| ZAP70 | zeta-chain (TCR) associated protein kinase 70kDa |

**Table 8. Gene symbols and names included in the natural killer cell specific set.**

| **Gene Symbol** | **Gene Name** |
|---|---|
| AKR1C3 | aldo-keto reductase family 1, member C3 (3-alpha hydroxysteroid dehydrogenase, type II) |
| ASCL2 | achaete-scute complex homolog 2 (Drosophila) |
| BZRAP1 | benzodiazapine receptor (peripheral) associated protein 1 |
| CCL3 | chemokine (C-C motif) ligand 3 |
| CCL3L3 | chemokine (C-C motif) ligand 3-like 3 |
| CD160 | CD160 molecule |
| CD247 | CD247 molecule |
| CD7 | CD7 molecule |
| CD96 | CD96 molecule |
| CHST12 | carbohydrate (chondroitin 4) sulfotransferase 12 |
| CLIC3 | chloride intracellular channel 3 |
| CMKLR1 | chemokine-like receptor 1 |
| COLQ | collagen-like tail subunit (single strand of homotrimer) of asymmetric acetylcholinesterase |
| CX3CR1 | chemokine (C-X3-C motif) receptor 1 |
| FASLG | Fas ligand (TNF superfamily, member 6) |
| FEZ1 | fasciculation and elongation protein zeta 1 (zygin I) |
| FGFBP2 | fibroblast growth factor binding protein 2 |
| GK5 | glycerol kinase 5 (putative) |
| GNLY | granulysin |
| GPR56 | G protein-coupled receptor 56 |
| GZMB | granzyme B (granzyme 2, cytotoxic T-lymphocyte-associated serine esterase 1) |
| IGFBP7 | insulin-like growth factor binding protein 7 |
| IL2RB | interleukin 2 receptor, beta |
| KIR2DL1 | killer cell immunoglobulin-like receptor, two domains, long cytoplasmic tail, 1 |
| KIR3DL1 | killer cell immunoglobulin-like receptor, three domains, long cytoplasmic tail, 1 |
| KIR3DL2 | killer cell immunoglobulin-like receptor, three domains, long cytoplasmic tail, 2 |
| KLRC3 | killer cell lectin-like receptor subfamily C, member 3 |
| KLRD1 | killer cell lectin-like receptor subfamily D, member 1 |
| KLRF1 | killer cell lectin-like receptor subfamily F, member 1 |
| LAIR2 | leukocyte-associated immunoglobulin-like receptor 2 |
| LAIR2 | leukocyte-associated immunoglobulin-like receptor 2 |
| LDB2 | LIM domain binding 2 |
| LOC401321 | uncharacterized LOC401321 |
| MYOM2 | myomesin (M-protein) 2, 165kDa |
| NCAM1 | neural cell adhesion molecule 1 |
| NCR1 | natural cytotoxicity triggering receptor 1 |
| NMUR1 | neuromedin U receptor 1 |
| OSBPL5 | oxysterol binding protein-like 5 |
| PDGFD | platelet derived growth factor D |
| PDGFRB | platelet-derived growth factor receptor, beta polypeptide |
| PDZD4 | PDZ domain containing 4 |
| PLEKHF1 | pleckstrin homology domain containing, family F (with FYVE domain) member 1 |
| PRF1 | perforin 1 (pore forming protein) |
| PRSS23 | protease, serine, 23 |
| PTGDR | prostaglandin D2 receptor (DP) |
| PTGDS | prostaglandin D2 synthase 21kDa (brain) |
| SH2D1B | SH2 domain containing 1B |
| SIGLECP3 | sialic acid binding Ig-like lectin, pseudogene 3 |
| TKTL1 | transketolase-like 1 |
| ZBTB16 | zinc finger and BTB domain containing 16 |

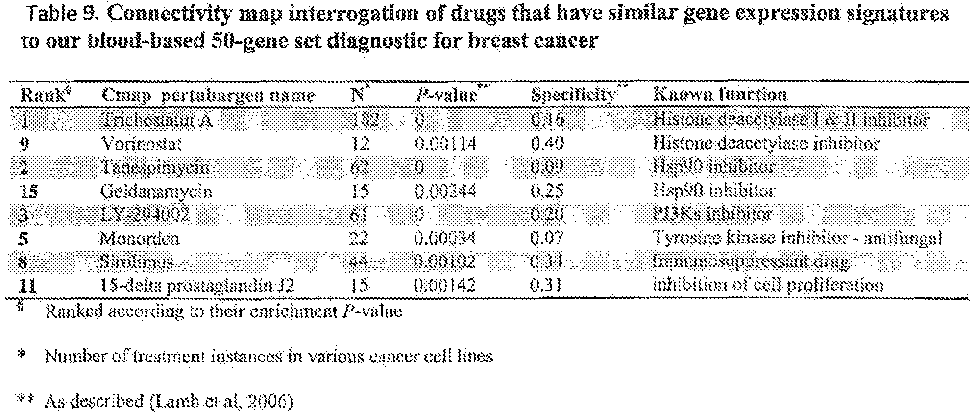

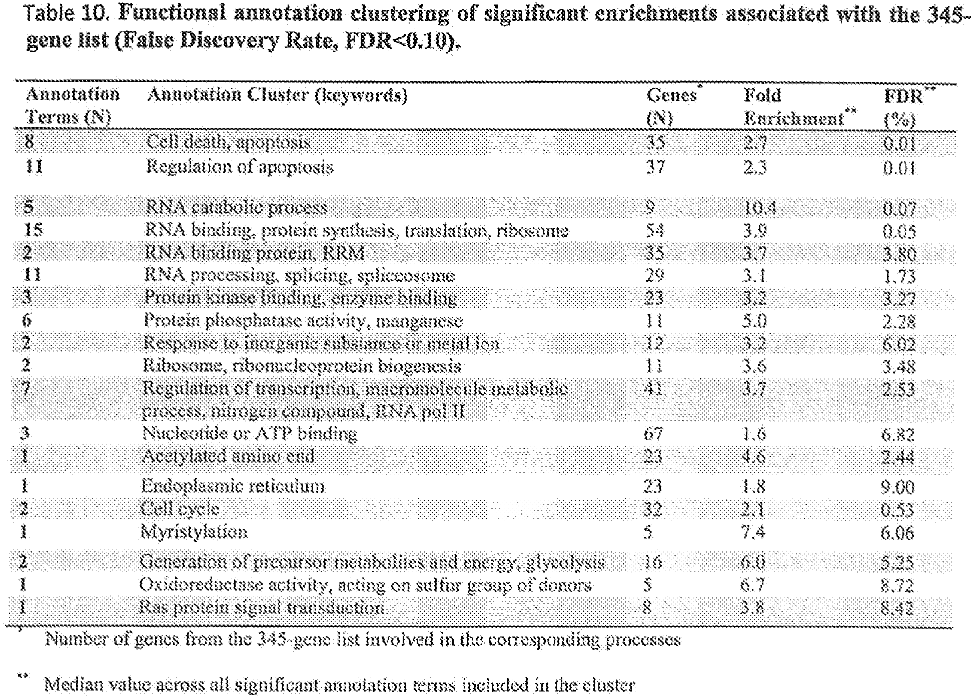

## Claims

1. An *in vitro* method for diagnosing, identifying or monitoring breast cancer in a human, which method comprises the following step:
a) measuring the level of gene expression of all of the genes set forth in Table 1 or of all of the genes set forth in Table 2 in a blood sample from said human;
b) comparing the level of gene expression of said genes in the sample from said human with the level of gene expression of said genes in a standard gene expression pattern extracted from healthy subjects;
c) wherein change of gene expression of each and one said gene in said sample as compared to a standard gene expression pattern being indicative for breast cancer.

2. The method of anyone of claim 1, wherein the change of gene expression is measured to be at least 10%, preferably at least 20%, more preferably at least 30% when measured as an intensity value of scanned images.

3. The method of claim 1 or 2, wherein the level of gene expression from all of the genes set forth in Table 1 or Table 2 is measured using the oligonucleotide probes from Table 1 or 2 or oligonucleotides derived from the sequences set forth in Table 1 or 2, or an oligonucleotide with a complementary sequence.

4. The method of any one of claims 1 to 3, wherein the oligonucleotide probes hybridize under high stringency conditions with all of the genes set forth in Table 1 or Table 2.

5. The method of any one of claims 1 to 4, wherein the oligonucleotide probes are immobilized on one or more solid supports.

6. The method of any one of claims 1 to 5, wherein said solid support is a membrane, plate, or a biochip.

7. The method of any one of claims 1 to 6, wherein said gene expression is measured using oligonucleotide probes of at least about 20, 50, 100 or 200 nucleotides in length.

8. The method of any one of claims 1 to 7, wherein the levels of gene expression in all of the genes set forth in Table 1 or Table 2 are detected in said sample by determining the levels of RNA molecules encoded by said genes.

9. An in vitro method for preparing a standard gene expression pattern reflecting breast cancer in a human, which method comprises the following step:
a) measuring the level of gene expression in a sample from said human,
b) measuring the level of gene expression in a control sample from a healthy human;
c) comparing level of gene expression of the sample from said human with the level of gene expression in a control sample from the healthy human (not suffering from breast cancer) to produce a characteristic standard gene expression pattern reference from genes reflecting breast cancer as set forth in Table 1 or 2.

10. A set of oligonucleotide probes, wherein said set consists of the oligonucleotides of Table 1 or 2 or oligonucleotides derived from the sequences set forth in Table 1 or 2, or an oligonucleotide with a complementary sequence, for use in the method of any one of claims 1-9.

11. The set of oligonucleotide probes according to claim 10, wherein said set hybridize under high stringency conditions with all of the genes set forth in Table 1 or Table 2 .

12. The set of oligonucleotide probes according to claims 10 or 11, wherein said probes are immobilized on one or more solid supports.

13. The set of oligonucleotide probes according to claim 12, wherein said solid support is a membrane, plate or biochip.

14. A kit for *in vitro* diagnosing for use in the method of any one of claims 1-9, identifying or monitoring proliferative disorder consisting of:
a collection of oligonucleotide probes and/or primers for detecting the level of
expression of all of the genes set forth in Table 1 or of all of the genes set forth in Table 2.

15. The kit of claim 14, wherein said probes are capable of specifically hybridizing to RNA transcripts of said genes.

16. A kit comprising a set of oligonucleotide probes as defined in any one of claims 10-13.

17. Use of a method of any one of claims 1 to 9, or a set of oligonucleotide probes of any one of claims 10-13, or a kit of any one of claims 14 to 16 for diagnosing, identifying or monitoring breast cancer in a human.

## Patentansprüche

1. *In vitro*-Verfahren zum Diagnostizieren, Identifizieren oder Überwachung von Brustkrebs in einem Menschen, wobei das Verfahren die folgenden Schritte umfasst:
a) Messen der Ebene von Genexpression von allen der in Tabelle 1 dargelegten Gene oder von allen der in Tabelle 2 dargelegten Gene in einer Blutprobe des Menschen;
b) Vergleichen der Ebene von Genexpression der Gene in der Probe des Menschen mit der Ebene von Genexpression der Gene in einem Standardgenexpressionsmuster, das aus gesunden Probanden extrahiert ist;
c) wobei die Änderung der Genexpression von jedem und demjenigen Gen in der Probe verglichen mit einem Standardgenexpressionsmuster in Hinweis für Brustkrebs ist.

2. Verfahren nach Anspruch 1, wobei die gemessene Änderung von Genexpression mindestens 10%, bevorzugt mindestens 20%, höher bevorzugt mindestens 30% beträgt, wenn sie als ein Intensitätswert von gescannten Bildern gemessen wird.

3. Verfahren nach Anspruch 1 oder 2, wobei die Ebene von Genexpression von allen der in Tabelle 1 oder Tabelle 2 dargelegten Gene unter Verwendung von den Oligonukleotidproben der Tabelle 1 oder 2 oder von Oligonukleotiden, die von den in Tabelle 1 oder 2 dargelegten Sequenzen abgeleitet sind, oder von einem Oligonukleotid mit einer komplementären Sequenz gemessen wird.

4. Verfahren nach einem der Ansprüche 1 bis 3, wobei die Oligonukleotidproben unter hohen Stringenzbedingungen mit allen der in Tabelle 1 oder Tabelle 2 dargelegten Gene hybridisieren.

5. Verfahren nach einem der Ansprüche 1 bis 4, wobei die Oligonukleotidproben auf einem oder mehreren festen Träger(n) immobilisiert werden.

6. Verfahren nach einem der Ansprüche 1 bis 5, wobei der feste Träger eine Membran, eine Platte oder ein Biochip ist.

7. Verfahren nach einem der Ansprüche 1 bis 6, wobei die Genexpression unter Verwendung von Oligonukleotidproben von mindestens etwa 20, 50, 100 oder 200 Nukleotiden in der Länge gemessen wird.

8. Verfahren nach einem der Ansprüche 1 bis 7, wobei die Ebenen von Genexpression in allen der in Tabelle 1 oder Tabelle 2 dargelegten Gene durch Bestimmen der Ebenen von RNA-Molekülen, die von den Genen kodiert sind, erfasst werden.

9. In vitro-Verfahren zur Herstellung eines Standardgenexpressionsmusters, das Brustkrebs in einem Menschen reflektiert, wobei das Verfahren die folgenden Schritte umfasst:
a) Messen der Ebene von Genexpression in einer Probe des Menschen,
b) Messen der Ebene von Genexpression in einer Kontrollprobe eines gesunden Menschen,
c) Vergleichen der Ebene von Genexpression der Probe des Menschen mit der Ebene von Genexpression in einer Kontrollprobe des gesunden Menschen (der nicht an Brustkrebs erkrankt ist), um eine charakteristische Standardgenexpressionsmusterreferenz aus Genen, die Brustkrebs, wie in Tabelle 1 oder 2 dargelegt, reflektieren, zu erstellen.

10. Satz von Oligonukleotidproben, wobei der Satz aus den Oligonukleotiden der Tabelle 1 oder 2 oder Oligonukleotiden, die von den in Tabelle 1 oder 2 dargelegten Sequenzen abgeleitet sind, oder einem Oligonukleotid mit einer komplementären Sequenz besteht, zur Verwendung in dem Verfahren nach einem der Ansprüche 1-9.

11. Satz von Oligonukleotidproben nach Anspruch 10, wobei der Satz unter hohen Stringenzbedingungen mit allen der in Tabelle 1 oder Tabelle 2 dargelegten Gene hybridisiert.

12. Satz von Oligonukleotidproben nach Anspruch 10 oder 11, wobei die Proben auf einem oder mehreren festen Träger(n) immobilisiert werden.

13. Satz von Oligonukleotidproben nach Anspruch 12, wobei der feste Träger eine Membran, eine Platte oder ein Biochip ist.

14. Kit für *In* vitro-Diagnostizieren zur Verwendung in dem Verfahren nach einem der Ansprüche 1-9, Identifizieren oder Überwachung einer proliferativen Erkrankung, bestehend aus:
einer Sammlung von Oligonukleotidproben und/oder Primern zum Erfassen der Ebene von Expression von allen der in Tabelle 1 dargelegten Gene oder von allen der in Tabelle 2 dargelegten Gene.

15. Kit nach Anspruch 14, wobei die Proben dazu im Stande sind, zu RNA-Transkripten der Gene spezifisch zu hybridisieren.

16. Kit umfassend einen Satz von Oligonukleotidproben, wie sie in einem der Ansprüche 10-13 definiert sind.

17. Verwendung eines Verfahrens nach einem der Ansprüche 1 bis 9, oder Satz von Oligonukleotidproben nach einem der Ansprüche 10-13, oder Kit nach einem der Ansprüche 14 bis 16 zum Diagnostizieren, Identifizieren oder Überwachung von Brustkrebs in einem Menschen.

## Revendications

1. Procédé *in vitro* pour diagnostiquer, identifier ou surveiller un cancer du sein chez un être humain, ledit procédé comprenant les étapes suivantes :
a) mesurer le niveau d'expression génique de l'ensemble des gènes présentés dans le Tableau 1 ou de l'ensemble des gènes présentés dans le Tableau 2 dans un échantillon de sang dudit être humain ;
b) comparer le niveau d'expression génique desdits gènes dans l'échantillon provenant dudit être humain avec le niveau d'expression génique desdits gènes dans un modèle d'expression génique standard extrait de sujets sains ;
c) dans lequel le changement d'expression génique de chacun et dudit gène dans ledit échantillon par rapport à un modèle d'expression génique standard est indicatif d'un cancer du sein.

2. Procédé selon la revendication 1, dans lequel le changement d'expression génique est mesuré comme étant d'au moins 10%, de préférence d'au moins 20%, plus préférablement d'au moins 30% si mesuré comme une valeur d'intensité d'images scannées.

3. Procédé selon la revendication 1 ou 2, dans lequel le niveau d'expression génique de l'ensemble des gènes présentés dans le Tableau 1 ou le Tableau 2 est mesuré en utilisant des sondes oligonucléotidiques provenant du Tableau 1 ou 2 ou des oligonucléotides dérivés des séquences indiquées dans le Tableau 1 ou 2 ou un oligonucléotide avec une séquence complémentaire.

4. Procédé selon l'une quelconque des revendications 1 à 3, dans lequel les sondes oligonucléotidiques s'hybrident dans des conditions de forte rigueur avec l'ensemble des gènes présentés dans le Tableau 1 ou le Tableau 2.

5. Procédé selon l'une quelconque des revendications 1 à 4, dans lequel les sondes oligonucléotidiques sont immobilisées sur un ou plusieurs supports solides.

6. Procédé selon l'une quelconque des revendications 1 à 5, dans lequel ledit support solide est une membrane, une plaque ou une biopuce.

7. Procédé selon l'une quelconque des revendications 1 à 6, dans lequel ladite expression génique est mesurée en utilisant des sondes oligonucléotidiques d'une longueur d'au moins environ 20, 50, 100 ou 200 nucléotides.

8. Procédé selon l'une quelconque des revendications 1 à 7, dans lequel les niveaux d'expression génique dans l'ensemble de gènes présentés dans au Tableau 1 ou au Tableau 2 sont détectés dans ledit échantillon en déterminant les niveaux de molécules d'ARN codées par lesdits gènes.

9. Procédé in vitro pour préparer un modèle d'expression génique standard reflétant un cancer du sein chez un être humain, ledit procédé comprenant les étapes suivantes :
a) mesurer le niveau d'expression génique dans un échantillon dudit être humain,
a) mesurer le niveau d'expression génique dans un échantillon témoin d'un être humain sain ;
c) comparer le niveau d'expression génique de l'échantillon dudit être humain avec le niveau d'expression génique dans un échantillon témoin provenant de l'être humain sain (ne souffrant pas d'un cancer du sein) pour produire une référence de modèle standard caractéristique d'expression génique à partir de gènes reflétant un cancer du sein comme indiqué dans le Tableau 1 ou 2.

10. Ensemble de sondes oligonucléotidiques, dans lequel ledit ensemble est constitué d'oligonucléotides du Tableau 1 ou 2 ou d'oligonucléotides dérivés d'une séquence présentée dans le Tableau 1 ou 2 ou d'un oligonucléotide avec une séquence complémentaire, à utiliser dans le procédé selon l'une quelconque des revendications 1 à 9.

11. Ensemble de sondes oligonucléotidiques selon la revendication 10, dans lequel ledit ensemble s'hybride dans des conditions de forte rigueur avec l'ensemble de gènes présentés dans le Tableau 1 ou le Tableau 2.

12. Ensemble de sondes oligonucléotidiques selon les revendications 10 ou 11, dans lequel les sondes oligonucléotidiques sont immobilisées sur un ou plusieurs supports solides.

13. Ensemble de sondes oligonucléotidiques selon la revendication 12, dans lequel ledit support solide est une membrane, une plaque ou une biopuce.

14. Kit pour diagnostiquer *in vitro* à utiliser dans le procédé selon l'une quelconque des revendications 1 à 9, identifier ou surveiller un trouble prolifératif, comprenant :
une collection de sondes oligonucléotidiques et / ou d'amorces destinée à détecter le niveau d'expression de l'ensemble des gènes présentés dans le Tableau 1 ou de l'ensemble des gènes présentés dans le Tableau 2.

15. Kit selon la revendication 14, dans lequel lesdites sondes sont capables de s'hybrider spécifiquement aux transcrits d'ARN desdits gènes.

16. Kit comprenant un ensemble de sondes oligonucléotidiques telles que définies dans l'une quelconque des revendications 10 à 13.

17. Utilisation d'un procédé selon l'une quelconque des revendications 1 à 9, ou d'un ensemble de sondes oligonucléotidiques selon l'une quelconque des revendications 10 à 13, ou d'un kit selon l'une quelconque des revendications 14 à 16 pour diagnostiquer, identifier ou surveiller un cancer du sein chez un être humain.
